(19) Europäisches Patentamt
European Patent Office
Office européen des brevets

(11) Publication number: **0 242 482 B1**

(12) # EUROPEAN PATENT SPECIFICATION

(45) Date of publication of patent specification: **06.09.95**

(51) Int. Cl.[6]: **C07D 473/16**, C07D 473/18, C07D 473/32, A61K 31/52, //C07D233/90,C07D239/50, C07C239/06

(21) Application number: **86309667.3**

(22) Date of filing: **11.12.86**

(54) **Antiviral purine derivatives and process for their preparation.**

(30) Priority: **13.12.85 GB 8530813**
**10.02.86 GB 8603228**
**28.08.86 GB 8620849**
**31.10.86 GB 8626041**

(43) Date of publication of application:
**28.10.87 Bulletin 87/44**

(45) Publication of the grant of the patent:
**06.09.95 Bulletin 95/36**

(84) Designated Contracting States:
**AT BE CH DE ES FR GB GR IT LI LU NL SE**

(56) References cited:
**EP-A- 0 049 072**
**EP-A- 0 108 285**
**EP-A- 0 130 126**

**Heterocycles 17,615(1982)**

**Top.Curr.Chem. 52,63(1974)**

**Topics in Med.Chem. 65,212(1988)**

(73) Proprietor: **BEECHAM GROUP plc**
**Four New Horizons Court**
**Harlequin Avenue**
**Brentford**
**Middlesex TW8 9EP (GB)**

(72) Inventor: **Harnden, Michael Raymond Beecham Pharmaceuticals**
**47 Guildford Road**
**Horsham**
**West Sussex RH12 1ND (GB)**
Inventor: **Wyatt, Paul Graham Beecham Pharmaceuticals**
**28 Tabor Court**
**Dallas Road**
**Cheam Village**
**Surrey (GB)**

(74) Representative: **Tocher, Pauline et al**
**SmithKline Beecham plc**
**Corporate Intellectual Property**
**SB House**
**Great West Road**
**Brentford, Middlesex TW8 9BD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**THE JOURNAL OF ORGANIC CHEMISTRY, vol. 39, no. 19, September 20, 1974, pages 2911-2916; Am. Chem. Soc., US A.A. WATSON:" Purine N-oxides. LVII. 9-hydroxyhypoxanthine, xanthine, and guanine"**

## Description

The present invention relates to compounds having antiviral activity, to processes for their preparation and to their use as pharmaceuticals.

9-[(2-hydroxyethoxy)methyl]guanine (Acyclovir), 9-(1,3-dihydroxy-2-propoxymethyl)guanine (DHPG) and 9-(4-hydroxy-3-hydroxymethylbut-1-yl)guanine, (disclosed in EP-A-141927), are all guanine derivatives having antiviral activity.

EP-A-130126 (Merck and Co. Inc.), EP-A-108285 (The Wellcome Foundation Limited) and EP-A-49072 (Ens Biologicals Inc.), disclose purine derivatives having antiviral activity.

A novel, structurally distinct class of compounds has now been discovered, these compounds also having antiviral activity.

Accordingly, the present invention provides a compound of formula (I), or a pharmaceutically acceptable salt thereof:

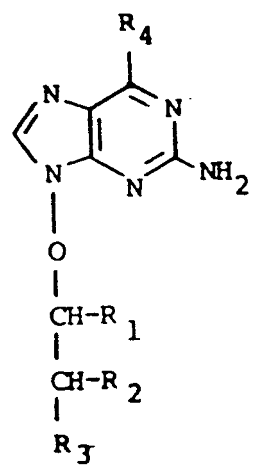

(I)

wherein:

$R_1$ is hydrogen or $CH_2OH$;

$R_2$ is hydrogen or, (when $R_1$ is hydrogen), hydroxy or $CH_2OH$;

$R_3$ is $CH_2OH$ or, (when $R_1$ and $R_2$ are both hydrogen), $CH(OH)CH_2OH$;

$R_4$ is hydrogen, hydroxy, amino or $OR_5$ wherein

$R_5$ is $C_{1-6}$ alkyl, phenyl or phenyl $C_{1-2}$ alkyl either of which phenyl moieties may be substituted by one or two halo, $C_{1-4}$ alkyl or $C_{1-4}$ alkoxy groups;

and in which any OH groups in $R_1$, $R_2$ and/or $R_3$ may be in the form of O-acyl, phosphate, cyclic acetal or cyclic carbonate derivatives thereof.

There are groups of compounds within formula (I) as follows:

(a) $R_1$ and $R_2$ are both hydrogen and $R_3$ is $CH_2OH$, and derivatives thereof as defined;

(b) $R_1$ is hydrogen and $R_2$ and $R_3$ are both $CH_2OH$, and derivatives thereof as defined;

(c) $R_1$ is hydrogen, $R_2$ is hydroxy and $R_3$ is $CH_2OH$, and derivatives thereof as defined;

(d) $R_1$ is $CH_2OH$, $R_2$ is hydrogen and $R_3$ is $CH_2OH$, and derivatives thereof as defined.

(e) $R_1$ and $R_2$ are both hydrogen and $R_3$ is $CH(OH)CH_2OH$, and derivatives thereof as defined.

Examples of $R_5$ include methyl, ethyl, n- and iso-propyl, phenyl and benzyl optionally substituted by one or two or methyl, ethyl, n- and iso-propyl, methoxy, ethoxy, n- and iso- propoxy, fluoro, chloro, bromo or $CF_3$.

O-Acyl derivatives are those wherein one or two of OH groups in $R_1$, $R_2$ and/or $R_3$ form carboxylic ester groups; which are $C_{1-7}$ alkanoyl and benzoyl optionally substituted by one or two $C_{1-4}$ alkyl, $C_{1-4}$ alkoxy, halogen or $CF_3$ groups. Preferably, carboxylic ester groups are $C_{1-7}$ alkanoyl groups, such as acetyl, propionyl, butyryl, heptanoyl and hexanoyl, most preferably acetyl or propionyl.

Phosphate esters of the compounds of formula (I) are those where one of the acyclic -OH groups is replaced by $(HO)_2$-$PO_2$- groups or salts thereof, or where two -OH groups on adjacent carbon atoms are replaced by a bridging -O-P(OH)$O_2$- group.

When $R_1$, $R_2$ and $R_3$ together contain two OH groups, cyclic acetal groups, -O-C($C_{1-3}$alkyl)$_2$-O- or cyclic carbonate, -O-CO-O- may be formed.

It is believed that the groups of compounds within formula (I) which are preferred are those indicated hereinbefore as (a) and (b); and that $R_4$ is preferably hydroxy or hydrogen.

Examples of pharmaceutically acceptable salts of the compound of formula (I) are acid addition salts formed with a pharmaceutically acceptable acid such as hydrochloric acid, orthophosphoric acid and sulphuric acid. Pharmaceutically acceptable salts also include those formed with organic bases, preferably with amines, such as ethanolamines or diamines; and alkali metals, such as sodium and potassium.

When the compound of formula (I) contains a phosphate group suitable salts include metal salts, such as alkali metal salts, for example sodium or potassium, alkaline earth metal salts such as calcium or magnesium and ammonium or substituted ammonium salts, for example those with lower alkylamines such as triethylamine, hydroxy-lower alkylamines such as 2-hydroxyethylamine, bis-(2-hydroxyethyl)-amine or tris-(2-hydroxyethyl)-amine.

It will be appreciated that some of the compounds of formula (I) have at least one asymmetric centre, and therefore are capable of existing in more than one stereoisomeric form. The invention extends to each of these forms individually and to mixtures thereof, including racemates. The isomers may be separated conventionally by chromatographic methods or using a resolving agent. Alternatively, the individual isomers may be prepared by asymmetric synthesis using chiral intermediates.

It will be further appreciated that, when $R_4$ is hydroxy in formula (I), the compound exists in the preferred tautomeric form of formula (IA):

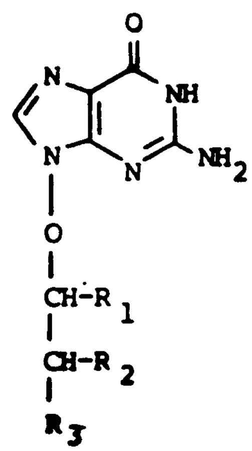

(IA)

The compounds of formula (I) including their alkali metal salts may form solvates such as hydrates and these are included wherever a compound of formula (I) or a salt thereof is herein referred to.

It will also be appreciated that compounds of formula (I) wherein $R_4$ is other than hydroxy are pro-drugs for the compounds of formula (I) wherein $R_4$ is hydroxy.

The invention also provides a process for the preparation of a compound of formula (I), or a pharmaceutically acceptable salt thereof, which process comprises cyclising a compound of formula (II):

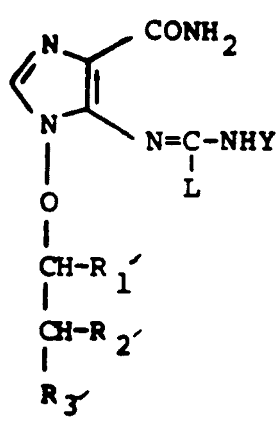

(II)

wherein L is a sulphur leaving group or $NH_2$, Y is a protecting group and $R_1'$, $R_2'$ and $R_3'$ are $R_1$, $R_2$ and $R_3$ respectively or $R_1$, $R_2$ and/or $R_3$ wherein OH groups are protected; and thereafter, when $R_4$ in the desired compound of formula (I) is other than hydroxy, converting an $R_4$ hydroxy group to chloro, followed by either (i) reduction to $R_4$ is hydrogen, (ii) replacement of chloro by $NH_2$ to give $R_4$ is amino; or (iii) reaction with $OR_5^-$ ion to give $R_4$ is $OR_5$; and deprotecting Y and converting $R_1'$ to $R_1$, $R_2'$ to $R_2$ and $R_3'$ to $R_3$ when desired or necessary, and optionally forming a pharmaceutically acceptable salt, O-acyl, phosphate, cyclic acetal or cyclic carbonate derivative thereof.

The cyclisation when L is a sulphur leaving group may take place in the presence of ammonia in an inert solvent, such as dimethylformamide; or, more preferably in basic conditions such as in 7M sodium hydroxide and dimethylsulphoxide; at elevated temperatures 80-150°C, preferably 100-120°C.

Suitable values for L then include alkylthio groups, such as methylthio, ethylthio, n- and iso-propylthio. Preferably L is methylthio.

When L is $NH_2$, the reaction preferably takes place in more weakly basic conditions, such as in 1M sodium hydroxide, at elevated temperatures 80-150°C, preferably 100-120°C.

Suitable values for Y include hydrolysable NH protecting groups, such as benzoyl optionally substituted as hereinbefore described for $R_5$ when phenyl. Y is preferably benzoyl.

Conversion of an $R_4$ hydroxy group to chloro may be achieved by chlorination using a reagent such as phosphorus oxychloride, preferably in the presence of tetraethylammonium chloride and dimethylaniline (as acid acceptor) in $CH_3CN$ at reflux temperatures, according to the method described by M.J. Robins and B. Uzanski, Can.J. Chem. 59, 2601(1981).

Reduction of a resulting chloro compound may preferably be achieved using catalytic methods, such as palladium on charcoal in an inert solvent, such as methanol or ethanol at reflux temperatures. The hydrogen source may be cyclohexene or ammonium formate. The procedure is analogous to that described in Example 1 of EP-A-182024 or to that described by T.A. Krenitsky et.al. Proc.Natl.Acad Sci. USA. 81, 3209 (1984).

Conversion to $R_4$ is amino may be achieved conventionally by treatment with ammonia in methanol in an autoclave at 100°C for a period of about 7 hours, or alternatively, with sodium axide in dimethylformamide to form an azido intermediate (wherein $R_4$ is $N_3$), followed by reduction of this intermediate with ammonium formate/palladium on charcoal, in methanol.

Reaction with $OR_5^-$ with the resulting chloro compound may be achieved using, preferably, $NaOR_5$ in a suitable solvent, such as methanol or ethanol when $R_5$ is methyl or ethyl respectively, at 0-150°C, preferably around 50°C. The procedure is analogous to that described in Example 15 of EP-A-141927.

Deprotection of Y may take place by conventional basic hydrolysis, such as aqueous sodium hydroxide at 100°C.

$R_1'$, $R_2'$ and/or $R_3'$ when protected OH groups, the protecting group(s) are often hydrogenolysable such as a benzyl group optionally substituted as defined above for $R_5$ when phenyl, also including nitro as an optional substituent.

Removal of benzyl protecting groups may be achieved conventionally, by catalytic hydrogenation using palladium on charcoal as catalyst (when $R_4$ is other than hydrogen).

Other suitable protecting groups include substituted benzyl groups such as p-methoxybenzyl, removable by treatment with DDQ, described in Example 8 hereinafter.

Another suitable protecting groups is the $^t$Butyl dimethylsilyl group removable by 80% acetic acid at elevated temperatures, around 90°C, or treatment with tetrabutyl ammonium fluoride in a solvent such as tetrahydrofuran, at ambient temperature.

Another suitable protecting group is wherein two OH groups or carbon atoms $\alpha$- or $\beta$- to one another are reacted with 2,2-dimethoxypropane, forming a 1,3-dioxolan or 1,3-dioxan ring respectively. This group may be removed by acidic hydrolysis.

Alternative values for $R_2'$ and $R_3'$, when protected OH groups, include that wherein two OH groups on adjacent carbon atoms are replaced by a bond; for example, when $R_1$ is hydrogen, $R_2$ is hydroxy and $R_3$ is $CH_2OH$; $R_3'CHR_2'CHR_1'O$- is $CH_2=CH-CH_2-O$-. The diol formation ('deprotection') may be achieved conventionally, for example, using osmium tetroxide, preferably catalytically in the presence of N-methylmorpholine N-oxide.

Pharmaceutically acceptable salts, O-acyl derivatives and phosphate derivatives may be prepared conventionally, for example as described in EP-A-141927 and EP-A-182024.

Acyl derivatives of compounds of formula (I) may be prepared by acylating an optionally protected compound of formula (I) in accordance with conventional acylating processes known in the art, and where necessary, deprotecting the resulting product.

The acylation reaction may be carried out by using an acylating agent containing a suitable carboxylic acid acyl group.

Examples of acylating agents suitable for the above process are carboxylic acids, acid halides, such as chlorides or acid anhydrides, preferably anhydrides or acids.

When the acylating agent is a carboxylic acid, a condensation promoting agent such as dicyclohexylcarbodiimide should be included, but this is not necessary when the acylating agent is an acid anhydride.

The acylation reaction may produce a single acyl derivative of a compound of formula (I), or a mixture of derivatives, depending on a number of factors, such as the relative amounts and chemical natures of reactants, the physical conditions of the reaction, and the solvent system. Any mixture produced in this way may be separated into its pure components using standard chromatographic techniques.

The above described acylation process of the invention can yield mono- or di-acylated derivatives of compounds of formula (I) containing two OH groups, according to the form of protection/deprotection utilised. The following are examples of products obtained by different methods:

(a) Acylated derivatives of the OH groups in $R_1/R_2/R_3$ when both acyl groups are the same, may be obtained by direct acylation of compounds of formula (I).

(b) Mono-acylated derivatives of one OH group when $R_1$, $R_2$ and $R_3$ together contain two OH groups may be obtained by acylation of protected intermediates of compounds of formula (I) in which the other -OH group in $R_1/R_3/R_3$ is preferably protected by, for example, a monomethoxytrityl or trityl group, and subsequent deprotection by acid treatment. Di-acylated derivatives wherein the acyl groups are different may then be prepared as in (a).

Acyl derivatives of the compounds of formula (I) can be converted to a compound of formula (I) by conventional deacylation or partial deacylation processes. For example, reaction with methanolic ammonia can be used to effect complete deacylation to yield a compound of formula (I) the ot both OH groups are deacylated. Reaction with a mild base such as potassium carbonate can result in partial deacylation of a di-acylated derivative to produce a compound of formula (I) wherein one acyl group and one OH group are present.

Phosphate derivatives are formed by reaction with a phosphorylating agent such as phosphorus oxychloride in pyridine. The $NH_2$ and any OH groups in $R_1$, $R_2$ and/or $R_3$ are protected as desired or necessary, preferably using a trityl or methoxytrityl protecting group, removable by acid hydrolysis, using acetic acid.

When more than one OH group in $R_1$, $R_2$ and $R_3$ is phosphorylated, a cyclic phosphate derivative is produced with phosphorus oxychloride, when the OH groups are $\alpha$ or $\beta$ to one another.

Another suitable phosphorylating agent is cyanoethyl phosphoric acid, in which case the product is normally treated with aqueous ammonia, which yields the ammonium salt of the phosphate ester as the final product.

A monophosphate may be converted to a cyclic phosphate using a dehydrating agent, such as dicyclohexylcarbodiimide.

Cyclic acetal derivatives of the compounds of formula (I) may be prepared from the compound of formula (I) wherein two OH groups in the side chain are present, preferably $\beta$- to one another, using an acyclic acetal, such as $R_{10}O\text{-}C(C_{1-3}alkyl)_2\text{-}OR_{10}$ wherein $R_{10}$ is $C_{1-4}$ alkyl, such as methyl or ethyl. The reaction is preferably carried out in an inert solvent such as tetrahydrofuran or dimethylformamide in the presence of an acid such as p-toluenesulphonic acid.

Cyclic carbonate derivatives of the compounds of formula (I) may be prepared from the compound of formula (I), wherein the $-NH_2$ group is preferably protected; with phosgene or 1,1-carbonyldimidazole, and thereafter deprotecting where necessary. Suitable protecting groups include trityl and monomethoxytrityl. The reaction is preferably carried out in dry pyridine at 0-50°C, preferably at ambient temperature.

It will be appreciated that conversions of $R_4$, deprotections and derivative formations may take place in any desired or necessary order.

Compounds of the formula (II) may be prepared according to the following reaction scheme:

Scheme 1

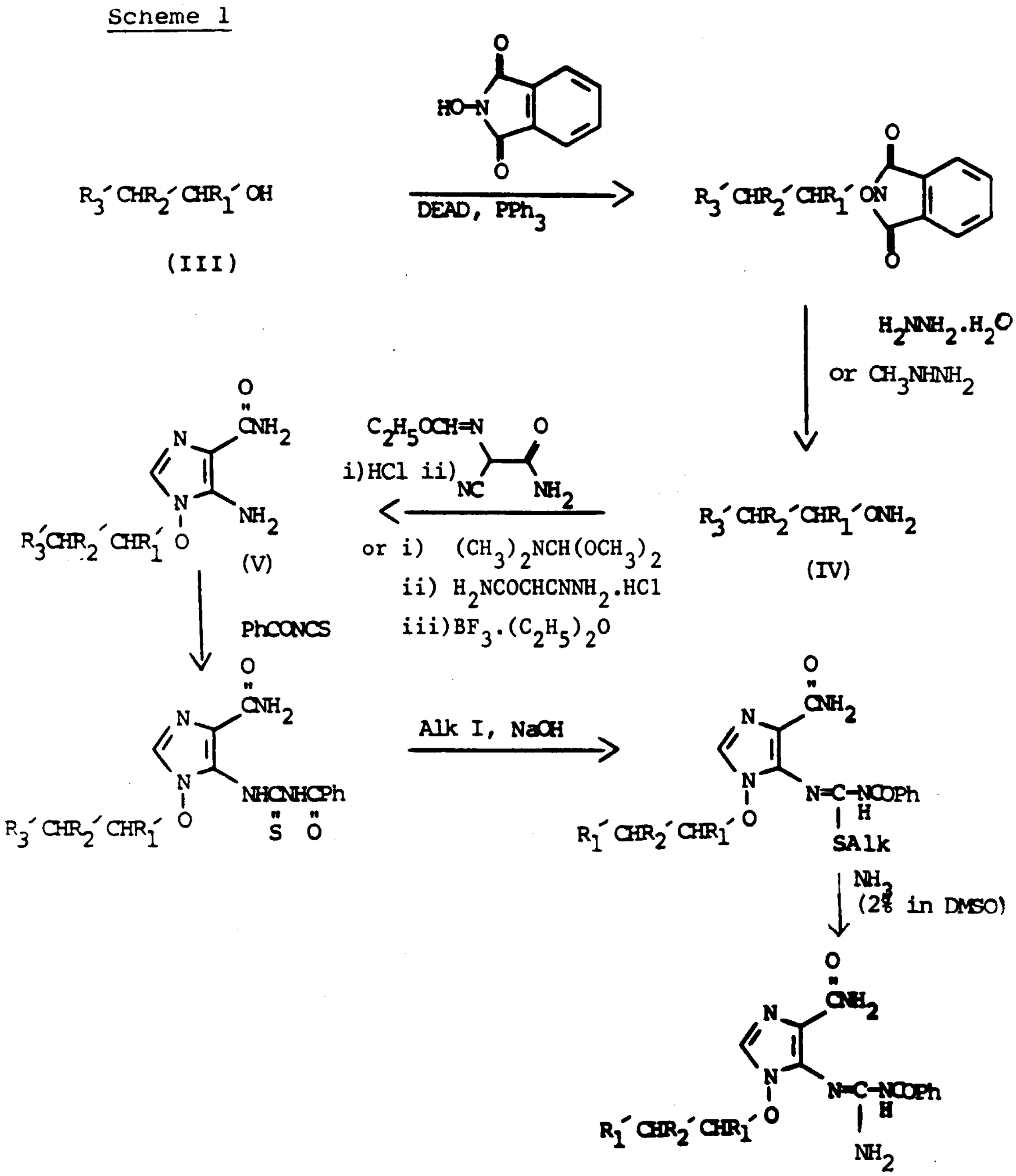


Alk is an alkyl group, such as methyl.

These methods are described in detail in Descriptions 1 and 6 hereinafter.

Compounds of the formula (III) are known or prepared by methods analogous to those used for the preparation of structurally similar known compounds.

Intermediates of the formulae (II) and (V) are novel and form an aspect of the invention.

Intermediates of the formula (IV), other than those wherein two OH groups on adjacent carbon atoms are replaced by a bond, are believed to be novel and also form an aspect of the invention.

The invention provides a further (preferred) process for the preparation of a compound of formula (I), or a pharmaceutically acceptable salt thereof, which process comprises converting the chloro group in a compound of formula (VI):

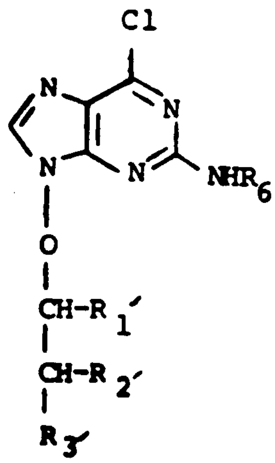

(VI)

wherein $R_6$ is hydrogen or an N-protecting group, such as $C_{1-7}$ alkanoyl, and $R_1'$, $R_2'$ and $R_3'$ are as hereinbefore defined, by either (i) hydrolysis to give $R_4$ as hydroxy, (ii) reduction to give a compound of formula (I) wherein $R_4$ is hydrogen, (iii) replacement of chloro by $NH_2$ to give $R_4$ is amino; or (iv) reaction with $OR_5^-$ to give a compound of formula (I) wherein $R_4$ is $OR_5$, and thereafter converting $R_1'$ to $R_1$, $R_2'$ to $R_2$ and/or $R_3'$ to $R_3$ when necessary, and/or optionally forming a pharmaceutically acceptable salt or derivative thereof as defined.

The hydrolysis (i) may be carried out using aqueous mineral acid, such as hydrochloric acid, or more preferably using an organic acid, such as formic acid at elevated temperature, suitably 70° - 150°C, preferably 100°C.

Reactions (ii), (iii) and (iv) above may be carried out as hereinbefore described for (i), (ii) and (iii) respectively under the process utilising a compound of formula (II).

Suitable values for protected OH groups in $R_1'$, $R_2'$ and $R_3'$ and methods for deprotection of groups in $R_1'$, $R_2'$ and $R_3'$ and methods for deprotection and formation of salts and derivatives are also described under the process utilising a compound of formula (II).

Compounds of the formula (VI) may be prepared by chlorination of the corresponding compound of formula (I) wherein $R_4$ is hydroxy, or, more preferably, from the compound of formula (VII):

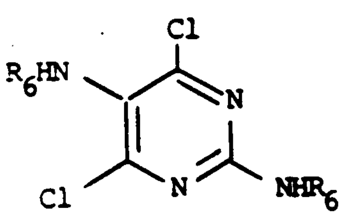

(VII)

wherein $R_6$ is formyl; by reaction with the compound of formula (IV) as hereinbefore defined, followed by cyclisation of the resulting compound of formula (VIII):

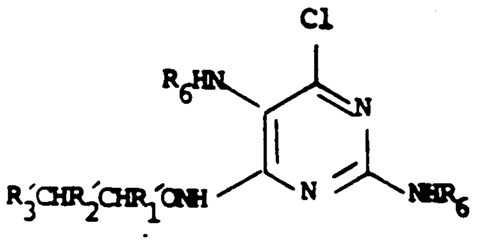

(VIII)

wherein $R_6$ is formyl; either with a suitable cyclisation condensing agent such as diethoxymethylacetate or triethylorthoformate, or by fusion. Suitable conditions for these reactions include those described hereinafter in Descriptions 2 to 5.

The compound of formula (VII) wherein $R_6$ is formyl may be prepared by reaction of the corresponding compound of formula (VII) wherein $R_6$ is hydrogen with formic acid and acetic anhydride.

The compound of formula (VII) wherein $R_6$ is hydrogen, 2,5-diamino-4,6-dichloropyrimidine is a known compound as described in C. Temple, Jr, B. H. Smith and J. A. Montgomery, J. Org. Chem., 40 (21), 3141, 1975.

Compounds of the formula (VI) and (VIII) are novel and form an aspect of the invention.

It will be appreciated that the invention provides a process for the preparation of a compound of formula (I) which process comprises the deprotection of a compound of formula (I) wherein any OH groups in $R_1$, $R_2$ and/or $R_3$ are in protected form.

Preferred methods for deprotection, as hereinbefore described are hydrogenation of benzyl protecting groups (when $R_4$ is other than hydrogen), DDQ removal of p-methoxybenzyl protecting groups, removal of the $^t$Butyldimethylsilyl group and (where appropriate) oxidation of a compound wherein OH groups on adjacent carbon atoms are replaced by a bond.

The compounds of the invention are useful in the treatment or infections caused by viruses, especially herpesviruses such as herpes simplex type 1, herpes simplex type 2; varicella zoster viruses; and also lentiviruses such as visna virus.

Compounds of the invention may be formulated for use in a pharmaceutical composition. Accordingly, in a further aspect of the invention, there is provided a pharmaceutical composition which comprises a compound of formula (I) or pharmaceutically acceptable salt thereof together with a pharmaceutically acceptable carrier or excipient.

A composition which may be administered by the oral route to humans may be compounded in the form of a syrup, tablet or capsule. When the composition is in the form of a tablet, any pharmaceutical carrier suitable for formulating such solid compositions may be used, for example magnesium stearate, starch, lactose, glucose, rice, flour and chalk. The composition may also be in the form of an ingestible capsule, for example of gelatin, to contain the compound, or in the form of a syrup, a solution or a suspension. Suitable liquid pharmaceutical carriers include ethyl alcohol, glycerine, saline and water to which flavouring or colouring agents may be added to form syrups. The compounds may also be presented with a sterile liquid carrier for injection.

The composition may also be formulated for topical application to the skin or eyes.

For topical application to the skin, the composition may be in the form of a cream, lotion or ointment. These formulations may be conventional formulations well known in the art, for example, as described in standard books of pharmaceutics and cosmetics, such as Harry's Cosmeticology published by Leonard Hill Books and the British Pharmacopaeia.

The composition for application to the eyes may be a conventional eye-drop composition well known in the art, or an ointment composition.

Preferably, the composition of this invention is in unit dosage form or in some other form that may be administered in a single dose. A suitable dosage unit might contain from 50 mg to 1 g of active ingredient, for example 100 to 500 mg.

Such doses may be administered 1 to 4 times a day or more usually 2 or 3 times a day. The effective dose of compound will in general be in the range of from 1.0 to 20 mg/kg of body weight per day or more usually 2.0 to 10 mg/kg per day.

No toxicological effects are indicated at the above described dosage levels.

The invention also provides the use of a compound of formula (I) in the manufacture of a medicament for use in the treatment of viral infections.

The invention also provides a compound of formula (I) or a pharmaceutically acceptable salt thereof for use as an active therapeutic substance, in particular for the treatment of viral infections.

The compounds of the invention also exhibit a synergistic antiviral effect in conjunction with interferons; and combination products comprising these two components for sequential or concomitant administration, by the same or different routes, are therefore within the ambit of the present invention.

The following examples illustrate the invention; the following descriptions illustrate the preparation of intermediates. The intermediates of Descriptions 10(f), 11 and 12 are also examples of active compounds of the invention.

Description 1 (Intermediates for Example 1, Method A)

(a) N-(3-Benzyloxyprop-1-oxy)phthalimide

Diethyl azodicarboxylate (15.6ml, 99.4mmol) was added to a solution of 3-benzyloxy-1-propanol (15g, 90.4mmol), N-hydroxyphthalimide (14.7g, 90.1mmol) and triphenylphosphine (23.7g, 90.4mmol) in tetrahydrofuran (450ml). After 16 hours at 20°C the solvent was removed under reduced pressure and the residue chromatographed on silica gel (eluted with hexane:acetone, 3:1) to yield the title compound as a yellow oil (27.8g, 99%). $^1$H NMR: $\delta_H$ ($CDCl_3$) 2.05 (2H, quintet, J = 6Hz, $CH_2CH_2CH_2$), 3.70 (2H, t, J = 6Hz, $CH_2OCH_2Ph$), 4.35 (2H, t, J = 6Hz, $CH_2ON$), 4.50 (2H, s, $CH_2Ph$), 7.35 (5H, s, $CH_2Ph$), 7.85 (4H, s, phthalimide protons).

(b) 3-Benzyloxyprop-1-oxyamine hydrochloride

A solution of N-(3-benzyloxyprop-1-oxy)phthalimide (27g, 86.8mmol) and hydrazine hydrate (4.2ml, 86.8mmol) in ethanol (200ml) was heated at reflux temperature for 1 hour, cooled and then added to 3% sodium carbonate solution (500ml). The aqueous solution was extracted with ether, the combined ether extracts were dried (magnesium sulphate), and evaporated to a syrup. Ethereal hydrogen chloride was added and the white solid obtained was separated by filtration, washed with ether and dried, yielding the title compound (15.2g, 81%).
$^1$H NMR: $\delta_H$ [$(CD_3)_2SO$] 1.80 (2H, quintet, J = 6Hz, $CH_2CH_2CH_2$), 3.50 (2H, t, J = 6Hz, $CH_2OCH_2Ph$), 4.10 (2H, t, J = 6Hz, $CH_2ON$), 4.40 (2H, s, $CH_2Ph$), 7.30 (5H, s, Ar), 11.20 (3H, br.s, $NH_3^+$).

(c) 5-Amino-1-(3-benzyloxyprop-1-oxy)-4-carboxamidoimidazole

A mixture of 3-benzyloxyprop-1-oxyamine hydrochloride (11.1g, 51.0mmol), ethyl N-(carbamoylcyano)-methylformimidate (8.8g, 70.3mmol), ether (300ml) and methanol (200ml) was stirred at 20°C for 24 hours. The ether was removed under reduced pressure and the methanolic solution boiled under reflux for 16 hours. The residue obtained on removal of the methanol was chromatographed on silica gel (eluted with chloroform then chloroform-ethanol, 19:1). Recrystallisation from acetone-petroleum ether (b.p. 60-80°C) gave the title compound (2.2g, 15%), m.p. 139-141°C.
UV: $\lambda_{max}$ (EtOH) 264 ($\epsilon$13,400)nm. IR: $\nu_{max}$ 3380, 3330, 3270, 3210, 3100, 1670, 1635, 1555, 1495, 1465, 1420cm$^{-1}$. $^1$H NMR: $\delta_H$ [$(CD_3)_2SO$] 1.98 (2H, quintet, J = 6.3, 6.6Hz, $CH_2CH_2CH_2$), 3.59 (2H, t, J = 6.3Hz $CH_2OCH_2Ph$), 4.22 (2H, t, J = 6.6Hz, $CH_2ON$), 4.49 (2H, s, $CH_2Ph$), 5.81 (2H, br.s, $CNH_2$), 6.71 (2H, br.s, $CONH_2$), 7.34 (6H, m, Ph, H-2). Found: C, 58.02; H, 6.31; N, 19.33%; $^m/e$, 290.1378. $C_{14}H_{18}N_4O_3$ requires: C, 57.91; H, 6.26; N, 19.30%; $^m/e$, 290.1379.

(d) 5-(N′-Benzoylthiocarbamoyl)amino-1-(3-benzyloxyprop-1-oxy)imidazole-4-carboxamide

Benzoylisothiocyanate (1.0ml, 7.6mmol) in acetone (100ml) was added to a solution of 5-amino-1-(3-benzyloxyprop-1-oxy)imidazole-4-carboxamide (2.0g, 6.9mmol) in hot acetone (200ml). The solution was boiled under reflux for 6 hours, cooled, evaporated under reduced pressure, and the residue chromatographed on silica gel (eluted with chloroform and then chloroform-ethanol, 30:1), yielding the title compound (3.0g, 96%), IR: $\nu_{max}$ (KBr) 3470, 3300, 3130, 1670, 1610, 1535, 1495cm$^{-1}$. $^1$H NMR: $\delta_H$ [$(CD_3)_2SO$] 1.93 (2H, quintet, J = 6.3Hz, $CH_2CH_2CH_2$), 3.54 (2H, t, J = 6.3Hz, $CH_2OCH_2Ph$), 4.39 (4H, m, $CH_2ON$, $CH_2Ph$), 7.12 (1H, CONH), 7.25 (6H, m, $PhCH_2$, CONH), 7.54 (2H, t, 2 protons of PhCO), 7.68 (1H, t, 1 proton of PhCO), 8.10 (3H, m, H-2, 2 protons of PhCO), 11.95 (2H, br.s, 2 x NH).

(e) 5-(N'-Benzoyl-S-methylthiocarbamoyl)amino-1-(3-benzyloxyprop-1-oxy)imidazole-4-carboxamide

A mixture of 5-(N'-benzoylthiocarbamoyl)amino-1-(3-benzyloxyprop-1-oxy)imidazole-4-carboxamide (2.9g, 6.4mmol), 0.1N sodium hydroxide solution (100ml) and methyl iodide (0.64ml; 10.3mmol) was stirred at 20°C for 16 hours and then the solution adjusted to pH 5 with glacial acetic acid. After several extractions with chloroform, the combined extracts were dried (magnesium sulphate) and evaporated to a syrup. Column chromatograhy on silica gel (eluted with chloroform-ethanol, 30:1) afforded the title compound (2.7g, 88.7%).

IR: $\nu_{max}$ (KBr) 3460, 3300, 3200, 3160, 1675, 1650, 1635, 1595, 1540, 1520, 1490, 1415cm$^{-1}$. $^1$H NMR: $\delta_H$ [-$(CD_3)_2SO$] 2.10 (2H, quintet, J = 6.3Hz, $CH_2\underline{CH_2}CH_2$), 2.50 (3H, s, $SCH_3$), 3.70 (2H, t, J = 6.3Hz, $\underline{CH_2}OCH_2Ph$), 4.40 (2H, t, J = 6.3Hz, $CH_2ON$), 4.55 (2H, s, $\underline{CH_2}Ph$), 7.50 (11H, $CH_2\underline{Ph}$, 3 protons of COPh, H-2, $CONH_2$), 8.00 (2H, m, 2 protons of COPh), 11.75 (1H, br.s, HN-C=N).

(f) 9-(3-Benzyloxyprop-1-oxy)guanine

5-(N'-Benzoyl-S-methylthiocarbamoyl)amino-1-(3-benzyloxyprop-1-oxy)imidazole-4-carboxamide (0.76g, 1.63mmol) was treated with 2% ammonia in dimethylformamide (35ml) at 120°C in a steel bomb for 6 hours. The solvent was removed under reduced pressure to yield a syrup, which was then dissolved in 1N sodium hydroxide and heated at 100°C for 3 hours. The cooled reaction mixture was acidified to pH 5 with concentrated hydrochloric acid. The precipitate obtained was collected, washed with several portions of ether and recrystallised from aqueous methanol, affording 9-(3-benzyloxyprop-1-oxy)guanine as white crystals (120mg, 25%). IR: $\nu_{max}$ (KBr) 3340, 3180, 2680, 1695, 1640, 1595, 1475cm$^{-1}$. $^1$H NMR: $\delta_H$ [$(CD_3)_2SO$] 1.93 (2H, quintet, J = 6.3, 6.6Hz, $CH_2\underline{CH_2}CH_2$), 3.58 (2H, t, J = 6.3Hz, $\underline{CH_2}OCH_2Ph$), 4.33 (2H, t, J = 6.6Hz, $\underline{CH_2}ON$), 4.48 (2H, s, $\underline{CH_2}Ph$), 6.61 (2H, br.s, $NH_2$), 7.33 (5H, m, Ar), 7.91 (1H, s, H-8), 10.67 (1H, br.s, H-1). Found: C, 57.08; H, 5.41; N, 22.25%. $C_{15}H_{17}N_5O_3$ requires C, 57.12; H, 5.44; N, 22.21%.

Description 2 (Intermediates for Example 1, Method B and Examples 2 to 4)

(a) 4,6-Dichloro-2,5-diformamidopyrimidine

Acetic anhydride (40ml) was added dropwise over 10 minutes to a mixture of 2,5-diamino-4,6-dichloropyrimidine (8.0g, 44.7mmol) and formic acid (100ml) at 0°C. The mixture was stirred at 20°C for 4 hours, evaporated to dryness and co-evaporated with toluene. Solidification from acetone-hexane afforded the title compound (6.1g, 60%). IR: $\nu_{max}$ (KBr) 3230, 1715, 1680, 1575, 1550, 1485, 1415cm$^{-1}$. Found m/e 233.9695; $C_6H_4N_4O_2Cl_2$ requires 233.9709.

(b) 6-(3-Benzyloxyprop-1-oxyamino)-4-chloro-2.5-diformamidopyrimidine

A mixture of 4,6-dichloro-2,5-diformamidopyrimidine (5.4g, 23.0mmol), 3-benzyloxyprop-1-oxyamine (4.14, 23.0mmol), triethylamine (10ml) and dioxan (50ml) was stirred at reflux temperature for 3 hours, cooled, filtered and evaporated to dryness. Column chromatography on silica gel (eluted with chloroform-ethanol, 25:1) gave the title compound (4.38g, 50%). IR: $\nu_{max}$ (KBr) 3248, 1692, 1589, 1570, 1473, 1420cm$^{-1}$. $^1$H NMR: δH [$(CD_3)_2SO$] 1.88 (2H, quintet, J = 6.3Hz, $CH_2\underline{CH_2}CH_2$) 3.57 (2H, t, J = 6.3Hz, $\underline{CH_2}OCH_2Ph$), 3.95 (2H, t, 6.3Hz, $\underline{CH_2}ONH$), 4.47 (2H, s, $\underline{CH_2}Ph$), 7.32 (5H, m, Ph), 8.14 (1H, s, $\underline{CH}ONH$), 9.26 (1H, s, $\underline{CH}ONH$), 9.42 (1H, br.s, $D_2O$ exchangeable, $\underline{NH}OCH_2$), 10.83 (2H, br.s, 2 x $\underline{NH}CHO$).

(c) 9(3-Benzyloxyprop-1-oxy)-6-chloro-2-formamidopurine

6-(3-Benzyloxyprop-1-oxyamino)-4-chloro-2,5-diformamido pyrimidine (4.3g, 11.3mmol) in diethoxymethyl acetate (40ml) was heated at 120°C for 2.5 hours. The mixture was then cooled and evaporated to a syrup. The residue was dissolved in methanol (40ml) and concentrated aqueous ammonia (5ml). The solution was then stirred for 30 minutes at 20°C, evaporated under reduced pressure and the residue co-evaporated with methanol. Column chromatography on silica gel (eluted with chloroform-methanol, 50:1) gave the title compound (3.93g, 95%). IR: $\nu_{max}$ 3120, 3080, 1700, 1615, 1580, 1500, 1435cm$^{-1}$. $^1$H NMR: δH ($CDCl_3$) 2.15 (2H, quintet, J = 6Hz, $CH_2\underline{CH_2}CH_2$), 3.75 (2H, t, J = 6Hz, $\underline{CH_2}OCH_2Ph$), 4.55 (4H, m, $CH_2ON$, $\underline{CH_2}Ph$), 7.40 (5H, m, Ph), 8.10 (1H, s, H-8), 8.40 (1H, d, J = 10Hz, $D_2O$ exchangeable, $\underline{NH}CHO$), 9.60 (1H, d, J = 10Hz, $NH\underline{CH}O$).

(d) 2-Amino-9-(3-benzyloxyprop-1-oxy)-6-ethoxypurine

A solution of 9-(3-benzyloxyprop-1-oxy)-6-chloro-2-formamidopurine (500mg, 1.38mmol) in 0.4M sodium ethoxide in ethanol (10ml) was heated at 80°C for 2.5 hours. The mixture was then cooled and evaporated under reduced pressure. The residue was dissolved in water (20ml) and the solution was brought to pH 7 with dilute hydrochloric acid. The aqueous solution was extracted with chloroform (2 x 20ml) and the combined extracts were washed with water (10ml), dried (magnesium sulphate) and evaporated under reduced pressure. Column chromatography on silica gel (eluted with chloroform-ethanol, 50:1) yielded the title compound (390mg, 81%). IR: $\nu_{max}$ (KBr) 3353, 3217, 1652, 1611, 1579, 1506, 1461, 1445, 1407cm$^{-1}$. $^1$H NMR: $\delta$H [$(CD_3)_2SO$] 1.35 (3H, t, J = 7.2Hz, $CH_3CH_2O$), 1.95 (2H, quintet, J = 6.3, 6.6Hz, $CH_2CH_2CH_2$), 3.60 (2H, t, 6.3Hz), 4.37 (2H, t, J = 6.6Hz, $CH_2ON$), 4.42 (2H, quartet, J = 7.2Hz, $CH_3CH_2O$), 4.48 (2H, s, $CH_2Ph$), 6.55 (2H, br.s, $D_2O$ exchangeable, $NH_2$), 7.33 (5H, m, Ph), 8.07 (1H, s, H-8).

Description 3 (Intermediates for Example 5)

(a) 6-(3-Benzyloxy-2-benzyloxymethylprop-1-oxyamino) -4-chloro-2,5-diformamidopyrimidine

A mixture of 4,6-dichloro-2,5-diformamidopyrimidine (from Description 1a) (3.3g, 14.0mmol), 3-benzyloxy-2-benzyloxymethylprop-1-oxyamine (4.2g, 14.0mmol), triethylamine (5.8ml, 41.6mmol) and dioxan (100ml) was stirred at 100°C for 2.5 hours. The cooled reaction mixture was filtered and the precipitate collected and washed with dioxan (2 x 25ml). The filtrate and washings were combined and evaporated to a syrup. Column chromatography on silica gel (eluted with chloroform-ethanol, 50:1) afforded the title compound (4.9g, 70%). IR: $\nu_{max}$ (KBr) 3240, 2910, 2860, 1690, 1585, 1565, 1475, 1420cm$^{-1}$. $^1$H NMR: $\delta$H [$(CD_3)_2SO$] 2.29 (1H, quintet, J = 6.0, 5.6Hz, CH), 3.56 (4H, d, J = 5.6Hz, 2 x $CH_2OCH_2Ph$), 3.94 (2H, d, J = 6.0Hz, $CH_2ON$) 4.46 (4H, s, 2 x $CH_2Ph$), 7.30 (10H, m, 2 x Ph), 9.28 (1H, br.s, NCHO), 9.42 (1H, br.s, NCHO), 10.86 (2H, br.s, $D_2O$ exchangeable, 2 x NHCHO).

(b) 9-(3-Benzyloxy-2-benzyloxymethylprop-1-oxy)-6-chloro-2-formamidopurine

6-(3-Benzyloxy-2-benzyloxymethylprop-1-oxyamino)-4-chloro-2,5-diformamidopyrimidine (4.8g, 9.6mmol) and diethoxymethyl acetate (50ml) was stirred at 120°C for 1.5 hours, cooled and evaporated under reduced pressure. The residue in methanol (80ml) and concentrated ammonia solution (2ml) was stirred at 20°C for 30 minutes, the solvent removed under reduced pressure and the residue co-evaporated with methanol. Column chromatography on silica gel (eluted with chloroform and then chloroform-ethanol, 50:1) afforded the title compound (4.11g, 89%). IR: $\nu_{max}$ (KBr) 3110, 2900, 2860, 1700, 1610, 1575, 1500, 1440cm$^{-1}$. $^1$H NMR: $\delta$H [$(CD_3)_2SO$] 2.40 (1H, quintet, J = 6.1, 6.7Hz, CH), 3.64 (4H, J = 6.7Hz, 2 x $CH_2OCH_2Ph$), 4.50 (6H, m, $CH_2ON$, 2 x $CH_2Ph$), 7.30 (10H, m, 2 x $CH_2Ph$), 9.36 (1H, s, OCHNH), 11.31 (1H, s, NHCHO). Found; C, 59.44; H, 5.09; N, 13.62%. $C_{24}H_{24}N_5O_4Cl.0.5EtOH$ requires: C, 59.45; H, 5.40; N, 13.87%.

Description 4 (Intermediates for Examples 6, 7 and 26)

(a) 4-Allyloxyamino-6-chloro-2,5-diformamidopyrimidine

A mixture of 4,6-dichloro-2,5-diformamidopyrimidine (3.75g, 16mmol), O-allylhydroxylamine hydrochloride (1.8g, 16.4mmol) and triethylamine (9.1ml, 66mmol) in dioxan (80ml) was stirred at 100°C for 6 hours. The cooled reaction mixture was filtered and evaporated to dryness under reduced pressure. The residue was chromatographed on silica gel (eluted withe chloroform-methanol, 10:1) and the product recrystallised from chloroform-methanol affording the title compound (2.1g, 48%), m.p. 170-181°C. IR:$\nu_{max}$ (KBr) 3240, 1705, 1650, 1590, 1570, 1495, 1465, 1420cm$^{-1}$. $^1$H NMR: $\delta$H [$(CD_3)_2SO$] 4.37 (2H, d, J = 6Hz, $CH_2ON$), 5.28 (2H, m, CH = $CH_2$), 6.00 (1H, m, CH = $CH_2$), 8.17 (1H, s, CHO), 9.30 (1H, s, CHO), 9.45 (1H, br.s, NH), 10.87 (2H, br.s, 2 x NH). Found: C, 39.64; H, 3.49; N, 25.34%, M$^+$ 271.0469. $C_9H_{10}ClN_5O_3$ requires C, 39.79; H, 3.71; N, 25.78%, M$^+$ 271.0469.

(b) 9-Allyloxy-2-amino-6-chloropurine

A solution of 4-allyloxyamino-6-chloro-2,5-diformamido pyrimidine (1.23g, 4.53mmol) in diethoxymethyl acetate (20ml) was stirred at 120°C for 4 hours. The reaction was cooled and evaporated to dryness under reduced pressure. The residue was dissolved in methanol (5ml) containing 0.88 ammonia (10ml) and stirred at 25°C for 16 hours. The solvents were evaporated under reduced pressure and the residue chromatographed on silica gel (eluted with ethyl acetate-hexane 1:1) yielding the title compound (520mg, 51%), m.p. 137-9°C. UV: $\lambda_{max}$ (MeOH) 224 ($\epsilon$ 25,700), 247 ($\epsilon$ 5,300), 309 ($\epsilon$ 7,400) nm. IR: $\nu_{max}$ (KBr) 3480, 3400, 3310, 3200, 1635, 1615, 1560, 1510, 1465cm$^{-1}$. $^1$H NMR: $\delta$H [$(CD_3)_2SO$] 4.84 (2H, d, J = 6Hz, $CH_2ON$), 5.35 (2H, m, $\underline{CH_2}$ = CH), 6.13 (1H, m, $\underline{CH}$ = $CH_2$), 7.10 (2H, br.s, $NH_2$), 8.35 (1H, s, H-8). Found: C, 42.45; H, 3.63; N, 30.39%; M$^+$ 225.0406. $C_8H_8ClN_5O$ requires C, 42.58; H, 3.57; N, 31.04%; M$^+$ 225.0414.

(c) 2-Amino-6-chloro-9-(2,3-dihydroxyprop-1-oxy)purine

A solution of 9-allyloxy-2-amino-6-chloropurine (374mg, 1.66mmol) and osmium tetroxide (catalytic) in acetone (10ml) and water (10ml) was treated with 4-methylmorpholine N-oxide (290mg, 2.49mmol) and stirred under nitrogen at 25°C for 16 hours. The reaction was evaporated to dryness under reduced pressure and the residue chromatographed on silica gel (eluted with acetone) affording the title compound (325mg, 76%), m.p. 173-5°C. UV: $\lambda_{max}$ (MeOH) 224 ($\epsilon$ 26,400), 310 ($\epsilon$ 7,600) nm. IR: $\nu_{max}$ (KBr) 3500, 3410, 3330, 3200, 3100, 1645, 1630, 1615, 1560, 1520, 1470cm$^{-1}$. $^1$H NMR: $\delta$H [$(CD_3)_2SO$] 3.42 (2H, m, $\underline{CH_2}OH$), 3.78 (1H, m, $\underline{CH}OH$), 4.20 (1H, dd, J = 10.7, 7.3Hz, $CH_2ON$), 4.41 (1H, dd, J = 10.7, 3.2Hz, $CH_2\underline{ON}$), 4.74 (1H, t, J = 5.7Hz, OH), 5.14 (1H, d, J = 5.2Hz), 7.13 (2H, br.s, $NH_2$), 8.36 (1H, s, H-8). Found: C, 36.41; H, 3.91, N, 25.91%, M$^+$ 259.0462. $C_8H_{10}ClN_5O_3.0.3H_2O$ requires C, 36.18; H, 4.04; N, 26.35%, M$^+$ 259.0469.

Description 5 (Intermediates for Examples 8 and 9)

(a) 1,4-Bis(4-methoxybenzyloxy)but-2-ene

To a solution of 2-buten-1,4-diol (4.94g, 60mmol) in dry dimethylformamide (120ml) was added sodium hydride (60% dispersion in oil; 5.28g, 132mmol) and the mixture was stirred at room temperature for 100 minutes. To this solution was added 4-methoxybenzyl chloride (17.9ml, 132mmol) in dimethylformamide (30ml) dropwise over 45 minutes and the mixture was stirred for a further 40 minutes. The mixture was partitioned between ether (150ml) and water (150ml). The organic layer was further washed with water (150ml), dried (magnesium sulphate) and the solvent was removed. The residue was purified by column chromatography on silica gel eluting with hexane-acetone (4:1, 3:1) to afford 1,4-bis(4-methoxybenzyloxy)-but-2-ene as a clear colourless liquid (13.7g, 70%). IR: $\nu_{max}$ (film) 2840, 1615, 1515 and 1250cm$^{-1}$. $^1$H NMR: $\delta$H ($CDCl_3$) 3.80 (6H, s, 2 x $CH_3$), 4.02 (4H, d, J = 4.5Hz, 2 x $CH\underline{CH_2}$), 4.43 (4H, s, 2 x $ArCH_2$), 5.79 (2H, t, J = 4.5Hz, 2 x CH), 6.88 (4H, d, J = 9Hz, ArH) and 7.27 (4H, d, J = 9Hz, ArH).

(b) 1,4-Bis(4-methoxybenzyloxy)butan-2-ol

To a solution of mercury (II) trifluoroacetate (17.1g, 40ml) in aqueous tetrahydrofuran (1:1, 80ml) was added a solution of 1,4-bis(4-methoxybenzyloxy)but-2-ene (12.8g, 39mmol) in tetrahydrofuran (10ml) over 5 minutes. The resulting 2-phase mixture was stirred vigorously at room temperature for 15 minutes. To the mixture was added aqueous sodium hydroxide (40ml, 3M) followed by sodium borohydride (0.5M solution in 3M sodium hydroxide; 40ml) with water-bath cooling. The solution was saturated with sodium chloride and allowed to stand. The organic layer was collected, dried (magnesium sulphate) and filtered through celite. The solvent was removed to afford 1,4-bis(4-methoxybenzyloxy)butan-2-ol (12.27g, 91%), IR: $\nu_{max}$ (KBr) 3440, 2940, 2860, 1610, 1510 and 1250cm$^{-1}$; $^1$H NMR: $\delta$H ($CDCl_3$) 1.76 (2H, q, J = 6.0Hz, 3-H), 2.90 (1H, br, $D_2O$ exchangeable, OH), 3.41 (2H, AB of ABX $J_{AX}$ = 6.9Hz, $J_{BX}$ = 4.2Hz and $J_{AB}$ = 9.5Hz, 1-H), 3.62 (2H, m, 4-H), 3.80 (6H, s, 2 x $CH_3$), 4.00 (1H, m, 2-H), 4.43 (2H, s, $ArCH_2$), 4.48 (2H, s, $ArCH_2$), 6.86 (4H, m, ArH) and 7.24 (4H, m, ArH).

(c) N-[1,4-Bis(4-methoxybenzyloxy)but-2-oxy]phthalimide

To a solution of 1,4-bis(4-methoxybenzyloxybutan-2-ol (12.12g, 35mmol), triphenylphosphine (14.7g, 56mmol) and N-hydroxyphthalimide (9.14g, 56mmol) in dry tetrahydrofuran (140ml) was added diethyl azodicarboxylate (8.82ml, 56mmol). The solution became hot and took on a dark red colour. After stirring for

22 hours, N-hydroxyphthalimide (1.63g, 10mmol), triphenylphosphine, (2.62g, 10mmol) and diethyl azodicarboxylate (1.57, 10mmol) were added. After a further 2 hours the solvent was removed. The residue was purified by column chromatography on silica gel eluting with hexane-ethyl acetate (2:1, 7:5) to afford N-[1,4-bis(4-methoxybenzyloxy)but-2-oxy]phthalimide as a clear colourless oil (10.9g, 63%). UV: $\lambda_{max}$ (EtOH) 222 ($\epsilon$ 45,700) and 274 ($\epsilon$ 3,590)nm. IR: $\nu_{max}$ (film) 2940, 2860, 1735, 1615, 1520 and 1250cm$^{-1}$. $^1$H NMR: $\delta$H ($CDCl_3$) 2.04 (2H, m, 3-H), 3.7-3.8 (10H, m, 2 x $CH_3$, 1-H and 4-H), 4.25-4.55 (4H, m, 2 x $ArCH_2$), 4.60 (1H, m, 2-H), 6.7-7.3 (8H, m, 2 x $CH_3OC_6\underline{H_4}CH_2$) and 7.73 (4H, m, phthalyl-H).

(d) 1,4-Bis(4-methoxybenzyloxy)but-2-oxyamine

To a solution of N-[1,4-bis(4-methoxybenzyloxy)but-2-oxy]phthalimide (10.3g, 21mmol) in dichloromethane (80ml) was added methylhydrazine (1.49ml, 28mmol) and the solution was stirred at room temperature. After 20 minutes, further methylhydrazine (0.22ml) was added. After a further 20 minutes the solution was filtered and the filtrate was extracted with aqueous sodium carbonate (3%). The organic layer was dried (magnesium sulphate) and the solvent removed. The residue was purified by column chromatography on silica gel eluting with hexane-ethyl acetate (1:1) to afford 1,4-bis(4-methoxybenzyloxy)but-2-oxyamine as a clear colourless oil (5.16g, 68%). IR: $\nu_{max}$ (film) 2860, 1615, 1515 and 1250cm$^{-1}$; $^1$H NMR: $\delta$H ($CDCl_3$) 1.80 (2H, m, 3-H), 3.54 (4H, m, 1-H and 4-H), 3.80 (6H, s, 2 x $CH_3$), 3.86 (1H, m, 2-H), 4.42 (2H, s, $ArCH_2$), 4.48 (2H, s, $ArCH_2$), 5.37 (2H, s, $D_2O$ exchangeable, $NH_2$), 6.87 (4H, m, Ar-H) and 7.25 (4H, m, Ar-H).

(e) 6-[1,4-Bis(4-methoxybenzyloxy)but-2-oxyamino]-4-chloro-2,5-diformamidopyrimidine

A solution of 4,6-dichloro-2,5-diformamidopyrimidine (3.27g, 13.9mmol), 1,4-bis(4-methoxybenzyloxy)-but-2-oxyamine (5.06g, 14mmol) and triethylamine (5.88ml, 42mmol) in dioxan (100ml) was heated at 100°C for 90 minutes. The solution was allowed to cool, filtered and the solvent removed. The residue was purified by column chromatography on silica gel eluting with chloroform-methanol (30:1) to afford 6-[1,4-bis (4-methoxybenzyloxy)but-2-oxyamino]-4-chloro-2,5-diformamidopyrimidine as a pale yellow foam. $^1$H NMR: $\delta$H ($CDCl_3$) 1.88 (2H, m, 3′-H), 3.54 (4H, m, 1′-H and 4′-H), 3.73 (3H, s, $CH_3$), 3.74 (3H, s, $CH_3$), 4.07 (1H, m, 2′-H), 4.37 (4H, m, 2 x $ArCH_2$), 6.88 (4H, m, ArH), 7.21 (4H, m, ArH), 9.17, 9.27, 9.30, 9.43 (total 2H, 4 x s, $D_2O$ exchange leaves s at 9.27, 2 x HCO), 10.67 (1H, br.s, $D_2O$ exchangeable, NH) and 10.85 (1H, br.s, $D_2O$ exchangeable, NH).

(f) 9-[1,4-Bis(4-methoxybenzyloxy)but-2-oxy]-6-chloro-2-formamidopurine and 2-Amino-9-[1,4-Bis(4-methoxybenzyloxy)but-2-oxy]-6-chloropurine

A solution of 6-[1,4-bis(4-methoxybenzyloxy)but-2-oxyamino)2,5-diformamido-4-chloropyrimidine (4.71g, 8.4mmol) in diethoxymethyl acetate (45ml) was heated at 120°C for 1 hour. The solution was allowed to cool and the solvent was removed. The residue was taken up in methanol (60ml) and concentrated aqueous ammonia (20ml) and the solution was stirred at 50°C for 1 hour and left at room temperature for 4 hours. The solvent was removed and the residue was purified by column chromatography on silica gel eluting with chloroform. Fractions 8-12 yielded 9-[1,4-bis(4-methoxybenzyloxy)but-2-oxy]-6-chloro-2-formamidopurine as a clear glass (0.66g, 14.5%). $^1$H NMR: $\delta$H [$(CD_3)_2SO$] 2.00 (2H, m, 3′-H), 3.57-3.70 (4H, m, 1′-H and 4′-H), 3.73 (6H, s, 2 x $CH_3$), 4.31 (2H, AB, J = 11.4 Hz, $ArCH_2$), 4.39 (2H, s, $ArCH_2$), 4.71 (1H, m, 2′-H), 6.85 (4H, m, ArH), 7.06 (2H, d, J = 8.8Hz, ArH), 7.21 (2H, d, J = 8.8Hz, ArH), 8.61 (1H, s, 8-H), 9.33 (1H, s, HCO) and 11.27 (1H, s, $D_2O$ exchangeable, 2-NH). Fractions 13-15 yielded a mixture of 2-formamido and 2-aminopurines (0.61g, 14%). Fractions 16 and 17 yielded 2-amino-9-[1,4-bis-(4-methoxybenzyloxy)but-2-oxy)-6- chloropurine as a clear glass. $^1$H NMR: $\delta$H [$(CD_3)_2SO$] 1.96 (2H, m, 3′-H), 3.46-3.65 (4H, m, 1′-H and 4′-H), 3.73 (6H, s, 2 x $CH_3$), 4.33 (2H, AB, J = 11.3Hz, $ArCH_2$), 4.36 (2H, s, $ArCH_2$), 4.62 (1H, m, 2′-H), 6.86 (4H, m, ArH), 7.03 (2H, s, $D_2O$ exchangeable, 2-$NH_2$), 7.11 (2H, d, J = 8.5Hz, ArH), 7.19 (2H, d, J = 8.5Hz, ArH) and 8.21 (1H, s, 8-H).

Description 6 (Intermediates for Example 1, Method A, alternative method to Description 1)

(a) N′-3-Benzyloxyprop-1-oxy-N,N-dimethylmethanimidamide

A solution of 3-benzyloxyprop-1-oxyamine (1.83g, 10mmol) in N,N-dimethylformamide dimethyl acetal (15ml) was stirred at room temperature for 30 mins. The solvent was removed under reduced pressure and

the residue dissolved in dichloromethane (30ml) and washed with water (2 x 30ml). The organic phase was dried ($MgSO_4$) and the solvent removed to give N-3-benzyloxyprop-1-oxy-N,N-dimethylmethanimidamide (2.1g, 93%); IR: $\nu_{max}$ (film) 3090, 3060, 3030, 2930, 2860, 1630, 1495, 1480, 1450, 1440, 1410, 1390, 1380, 1365, 1320, 1250, 1205, 1105, 1075, 1065, 1030, 990, 950, 930, 910, 845, 740, 700cm$^{-1}$; $^1$H NMR: $\delta$H ($CDCl_3$) 1.95 (2H, m, $CH_2$), 2.76 (6H, s, 2 x $CH_3$), 3.57 (2H, t, J = 6.5Hz, $CH_2$), 3.95 (2H, t, J = 6.5Hz, $CH_2$), 4.51 (2H, s, $CH_2$), 7.3 (5H, m, $C_6H_5$), 7.61 (1H, s, CH); m/z 236 ($M^+$, 5%), 163 (5), 145 (25), 130 (20), 107 (35), 101 (5), 92 (10), 91 (100), 89 (15), 72 (15), 71 (50), 69 (15), 65 (15), 57 (15); $M^+$ observed 236.1527; $C_{13}H_{20}N_2O_2$ requires $M^+$ 236.1524.

(b) 2-[N-(3-benzyloxyprop-1-oxy)iminomethyl]amino-2-cyano-acetamide

A solution of 2-amino-2-cyanoacetamide hydrochloride (5.38g, 40mmol) and N′-3-benzyloxyprop-1-oxy-N,N-dimethylmethanimidamide (8.7g, 39mmol) in methanol (35ml) was stirred at room temperature for 22 hours. The solvent was removed under reduced pressure and the residue treated with ethyl acetate (200ml) and brine (1ml). The ethyl acetate solution was decanted, the oily residue was washed with ethyl acetate (50ml) and the combined organic phases were dried ($MgSO_4$). The solvent was removed under vacuum and the residual oil washed with hexane (3 x 50ml), the hexane layer decanted and the residue dissolved in warm chloroform (40ml) and treated with hexane (150ml). The mixture was refrigerated at -18°C for 1 hour, the organic phase decanted and the residual oil dried under reduced pressure to give 2-[N-(3-benzyloxyprop-1-oxy)iminomethyl]amino-2-cyanoacetamide (8g, 70%); IR: $\nu_{max}$ (film) 3340, 3190, 3090, 3060, 3030, 2930, 2870, 2800, 1700, 1660, 1600, 1495, 1455, 1380, 1370, 1215, 1100, 1075, 1030, 980, 910, 860, 750, 700cm$^{-1}$; $^1$H NMR: $\delta$H [$(CD_3)_2SO$] 1.86 (2H, m, $CH_2$), 3.53 (2H, m, $CH_2$), 3.84 (0.28H, t, J = 6.5Hz, $CH_2$), 3.94 (1.72H, t, J = 6.5Hz, $CH_2$), 4.46 (2H, s, $CH_2$), 5.06 (0.14H, d, J = 8Hz, CH), 5.23 (0.86H, d, J = 8Hz, CH), 6.76 (1H, d, J = 10.5Hz, CH), 6.95 (1H, dd, J = 10.5 and 8Hz, $D_2O$ exchangeable NH), 7.2-7.4 (5H, m, $C_6H_5$), 7.95 (2H, br.s., $D_2O$ exchangeable $NH_2$); m/z 290 ($M^+$, 10%), 215 (3), 199 (5), 198 (10), 182 (5), 169 (5), 163 (10), 125 (10), 123 (10), 107 (35), 106 (10), 105 (10), 92 (15), 91 (100), 79 (10), 71 (10), 65 (10), 44 (15). Found: C, 54.06; H, 5.97; N, 17.64% $M^+$ 290.1369.
$C_{14}H_{17}N_4O_3.1.15H_2O$ requires: C, 54.23; H, 6.27; N, 18.07%. $M^+$ 290.1376.

(c) 5-Amino-1-(3-benzyloxyprop-1-oxy)-4-carboxamidoimidazole

To a solution of 2-[N-(3-benzyloxyprop-1-oxy)iminomethylamino-2-cyanoacetamide (2g, 6.9mmol) in dry dimethoxyethane (150ml) under an atmosphere of dry nitrogen was added boron trifluoride etherate (0.85ml, 6.9mmol). The solution was heated at 60°C for 1 hour, cooled to room temperature and the solvent removed under vacuum. The residue was partitioned between chloroform (100ml) and sodium bicarbonate solution (100ml). The aqueous phase was extracted with chloroform (30ml), the combined organic phases dried ($MgSO_4$) and the solvent removed. The residue was chromatographed on silica, eluting with chloroform-methanol 20:1 to give 5-amino-1-(3-benzyloxyprop-1-oxy)-4-carboxamidoimidazole(1.25g, 63%).

Description 7 Chiral Intermediates for Examples 14 and 15

(a) (S)-2-Methoxymethoxybutanedioic acid, bis dimethoxymethyl ester

To a solution of (S)-2-hydroxybutanedioic acid (10g, 75mmol) in dry N,N-dimethylformamide (100ml) was added diisopropylethylamine (29ml, 165mmol). The mixture was cooled to 0°C and treated dropwise with a solution of chloromethyl methyl ether (13ml, 165mmol) in dry N,N-dimethylformamide. After stirring at room temperature for 18 hours, the solvent was removed and the residue treated with ethyl acetate (100ml). The mixture was filtered and the precipitate washed with ethyl acetate (2 x 50ml). The combined organic solutions were washed with brine (2 x 50ml) and dried ($MgSO_4$). The solvent was removed under reduced pressure and the residual oil dissolved in dry dimethoxyethane (50ml) and diisopropylethylamine (19.5ml, 112mmol) and treated dropwise with a solution of chloromethyl methyl ether (8.8ml, 112mmol) in dimethoxyethane (10ml). The solution was heated at 80°C for 2 hours, the solvent removed under reduced pressure and the residue dissolved in ethyl acetate (100ml) and filtered. The filtrate was washed with brine (3 x 30ml), dried ($MgSO_4$) and the solvent removed to leave a liquid which was distilled to give (S)-2-methoxymethoxybutanedioic acid, bis dimethoxymethyl ester (15g, 75%), b.p. 116-122°C $[\alpha]_D^{25}$ -42.7° (c 1.3 in ethanol); IR: $\nu_{max}$ (film) 3000, 2880, 2900, 2830, 1745, 1470, 1450, 1440, 1410, 1370, 1275, 1210, 1150, 1115, 1095, 1030, 930cm$^{-1}$; $^1$H NMR: $\delta$H ($CDCl_3$) 2.88 (2H, d, J = 6.5Hz, $CH_2$), 3.40 (1H, s, $OCH_3$), 3.48 (3H, s, $OCH_3$), 4.56 (1H, t, J = 6.5Hz, CH), 4.75 (2H, s, $CH_2$), 5.27 (2H, s, $CH_2$), 5.33 (2H, s, $CH_2$).

Found: C, 45.23; H, 6.92%. $C_{10}H_{18}O_7$ requires C, 45.11; H, 6.82%.

(R)-2-Methoxymethoxybutanedioic acid, bis methoxymethyl ester

The (R)-isomer was prepared in an identical fashion from (R)-2-hydroxybutanedioic acid, and was obtained in 82% yield after chromatography on silica, eluting with ethyl acetate-hexane 1:2; $[\alpha]_D^{25}$ + 47.6° (c 1.1 in ethanol).

(b) (S)-1,4-Dibenzyloxy-2-(methoxymethoxy)butane

A solution of (S)-2-methoxymethoxybutanedioic acid, bis methoxymethyl ester (10g, 37.5mmol) in dry tetrahydrofuran (10ml) under dry nitrogen was treated with borane dimethylsulphide (8.3ml, 83mmol). The solution was heated between 60°C and 80°C over a period of 5.5 hours and then cooled in ice and treated dropwise with methanol (50ml). After effervescence had ceased the solution was stirred at room temperature for 18 hours and the solvent removed under vacuum. The residue was evaporated to dryness with methanol (2 x 50ml) and the residue chromatographed on silica, eluting with chloroform-methanol 5:1 to give (S)-1,4-dihydroxy-2-(methoxy)methoxybutane (3.9g, 69%); IR: $\nu_{max}$ (film) 3400, 2940, 2890, 2820, 1470, 1440, 1410, 1380, 1300, 1210, 1150, 1100, 1030, 920cm$^{-1}$; $^1$H NMR: δH ($CDCl_3$) 1.78 (2H, m, $CH_2$), 2.96 (2H, s, $D_2O$ exchangeable OH's), 3.44 (3H, s, $OCH_3$), 3.5-3.85 (5H, m, CH plus 2 x $CH_2$'s), 4.72 (1H, d, J = 7Hz, CH of $CH_2$), 4.77 (1H, d, J = 7Hz, CH of $CH_2$).

A 60% suspension of sodium hydride in oil (1.34g, 33-mmol) was washed with hexane (2 x 20ml) under an atmosphere of dry nitrogen. After decanting the hexane the solid was suspended in dry N,N-dimethylformamide (20ml) and treated with a solution of (S)-1,4-dihydroxy-2-(methoxymethoxy)butane (2g, 13mmol) in N,N-dimethylformamide (5ml). After stirring at room temperature for 6 hours the mixture was treated with a solution of benzyl bromide (3.9ml, 33mmol) in N,N-dimethylformamide (5ml) and the mixture was stirred at room temperature overnight. The solvent was removed under reduced pressure and the residue partitioned between ethyl acetate (100ml) and water (50ml). The organic phase was washed with water (2 x 50ml), dried with $MgSO_4$ and the solvent removed to give an oil which was chromatographed on silica eluting with ethyl acetate-hexane 1:2 to give (S)-1,4-dibenzyloxy-2-(methoxymethoxy)butane (3.6g, 82%),

$$[\alpha]_D^{25} - 16.0^o$$

(c 1.6 in ethanol); IR: $\nu_{max}$ (film) 3080, 3060, 3030, 2920, 2890, 2860, 1495, 1450, 1360, 1210, 1155, 1100, 1040, 920, 740, 700cm$^{-1}$; $^1$H NMR: δH ($CDCl_3$) 1.90 (2H, m, $CH_2$), 3.35 (3H, s, $OCH_3$), 3.60 (4H, m, 2 x $CH_2$), 3.90 (1H, m, CH), 4.50 (4H, m, 2 x $CH_2$), 4.68 (1H, d, J = 7Hz, CH of $CH_2$), 4.74 (1H, d, J = 7Hz, CH of $CH_2$), 7.30 (10H, m, 2 x $C_6H_5$); m/z ($NH_3$ CI), $MH^+$151, $MNH_4{}^+$168.

(R)-1,4-Dibenzyloxy-2-(methoxymethoxy)butane

The (R)-isomer was prepared in an identical fashion from (R)-2-(methoxymethoxy)butanedioic acid, bis dimethoxymethyl ester and had $[\alpha]_D^{25}$ + 16.8° (c 1.1 in ethanol).

(c) (S)-1,4-Dibenzyloxy-2-hydroxybutane

To a solution of (S)-1,4-dibenzyloxy-2-(methoxymethoxy)butane (2g, 6mmol) in methanol (14ml) was added a 2% solution of methanolic hydrogen chloride (6ml, 2.5mmol). The solution was stirred at room temperature for 7 hours, the solvent removed and the residue chromatographed on silica eluting with ethyl acetate-hexane 1:1 to give (S)-1,4-dibenzyloxy-2-hydroxybutane (1.46g, 84%),

$$[\alpha]_D^{25} - 7.3^o$$

(c 1.1 in ethanol); IR: $\nu_{max}$ (film) 3450, 3080, 3060, 3015, 2920, 2860, 2800, 1950, 1870, 1810, 1605, 1585, 1495, 1450, 1365, 1310, 1260, 1205, 1100, 1030, 1000, 950, 915, 820, 805, 760, 700, 610cm$^{-1}$; $^1$H NMR: δH

($CDCl_3$) 1.79 (2H, d, t J = 6Hz and 6Hz, $CH_2$), 2.86 (1H, d, J = 3Hz, $D_2O$ exchangeable, OH), 3.45 (2H, m, $CH_2$), 3.65 (2H, m, $CH_2$), 4.05 (1H, m, CH), 4.51 (2H, s, $CH_2$), 4.55 (2H, s, $CH_2$), 7.3 (10H, m, 2 x $C_6H_5$). Found: C, 75.64; H, 7.87%. $C_{18}H_{22}O_3$ requires: C, 75.50; H, 7.74%.

(R)-1,4-dibenzyloxy-2-hydroxybutane

The (R)-isomer was prepared in an identical fashion from (R)-1,4-dibenzyloxy-2-(methoxymethoxy)-butane and had

$$[\alpha]^{25}_{D} + 7.7^{o}$$

(c 0.5 in ethanol).

Description 8 (Intermediates for Example 14)

(a) (R)-N-(1,4-Dibenzyloxybut-2-oxy)phthalimide

To a solution of (S)-1,4-dibenzyloxy-2-hydroxybutane (5g, 17.5mmol) in dry tetrahydrofuran (100ml) was added triphenylphosphine (5.1g, 19.2mmol) and N-hydroxyphthalimide (3.1g, 19.2mmol). The solution was treated dropwise with diethyl azadicarboxylate (3ml, 19.2mmol). A red colouration appeared, gradually fading and the solution became warm. The solution was stirred at room temperature for 48 hours and the solvent removed under vacuum. The residue was dissolved in ethylacetate-hexane 1:1 (100ml) and refrigerated. A white solid crystallised out and was filtered off. The solution was evaporated to dryness and the residue chromatographed on silica, eluting with ethylacetate-hexane 1:2 to give (R)-N-(1,4-dibenzyloxybut-2-oxy)phthalimide (4.6g, 61%), $[\alpha]^{25}_{D}$ + 16.4° (c 1.3 in ethanol); IR:
$\nu_{max}$ (film) 3090, 3060, 3030, 2930, 2860, 1810, 1790, 1730, 1610, 1495, 1465, 1450, 1370, 1240, 1190, 1120, 1100, 1080, 1030, 1015, 980, 880, 785, 740, 700$cm^{-1}$;
$^1H$ NMR: $\delta H$ ($CDCl_3$) 2.1 (2H, m, $CH_2$), 3.75 (4H, m, 2 x $CH_2$), 4.5 (5H, m, 2 x $CH_2$ plus CH), 7.1-7.9 (14H, m, 2 x $C_6H_5$ plus $C_6H_4$); m/z (FAB) $MH^+$ 432.

(b) (R)-1,4-Dibenzyloxybut-2-oxyamine

A solution of (R)-N-(1,4-dibenzyloxybut-2-oxy)phthalimide (0.6g, 1.3mmol) in dichloromethane (5ml) was treated with methylhydrazine (86µl, 1.6mmol). A deep red colouration developed, disappearing gradually as a white solid precipitated out of solution. After stirring at room temperature for 30 minutes additional methylhydrazine (8µl, 0.15mmol) was added. The mixture was stirred at room temperature for an additional 30 minutes, filtered and the filtrate washed with 3% sodium carbonate solution (5ml). The organic phase was dried ($MgSO_4$), the solvent removed under reduced pressure and the residue chromatographed on silica eluting with ethyl acetate-hexane to give (R)-1,4-dibenzyloxybut-2-oxyamine (0.32g, 77%), $[\alpha]^{25}_{D}$ + 10.1° (c 0.14 in ethanol); IR: $\nu_{max}$ (film) 3310, 3250, 3090, 3060, 3030, 2920, 2860, 1585, 1495, 1450, 1365, 1310, 1200, 1100, 1030, 950, 915, 740, 700$cm^{-1}$;
$^1H$ NMR: $\delta H$ ($CDCl_3$) 1.85 (2H, m, $CH_2$), 3.55 (4H, m, 2 x $CH_2$), 3.90 (1H, m, CH), 4.49 (2H, s, $CH_2$), 4.45 (2H, s, $CH_2$), 5.36 (2H, s, $D_2O$ exchangeable $NH_2$) 7.35 (10H, m, 2 x $C_6H_5$); m/z 302 ($MH^+$), 301 ($M^+$, 3%), 181 (5), 163 (10), 106 (15), 105 (5), 92 (15), 91 (100), 71 (15), 65 (10). $M^+$ observed 301.1670; $C_{18}N_{23}NO_3$ requires $M^+$ 301.1678.

(c) (R)-N'-1,4-Dibenzyloxybut-2-oxy-N,N-dimethylmethanimidamide

A solution of (R)-1,4-dibenzyloxybut-2-oxyamine (1.6g, 5.3mmol) in N,N-dimethylformamide dimethyl acetal (10ml) was stirred at room temperature for 30 minutes. The solvent was removed and the residue dissolved in dichloromethane (50ml) and washed with brine (2 x 20ml). The organic phase was dried ($MgSO_4$), the solvent removed under reduced pressure and the residue chromatographed on silica, eluting with ethyl acetate-hexane 1:2 to give, as an oil, (R)-N'-1,4-dibenzyloxybut-2-oxy-N,N-dimethylmethanimidamide (1.51g, 77%), IR: $\nu_{max}$ (film) 3090, 3060, 3030, 3000, 2920, 2900, 2860, 1630, 1495, 1480, 1455, 1410, 1390, 1365, 1320, 1250, 1205, 1105, 1030, 990, 945, 925, 910, 740, 700$cm^{-1}$; $^1H$ NMR: $\delta H$ ($CDCl_3$) 1.97 (2H, m, $CH_2$), 2.73 (6H, s, 2 x $CH_3$), 3.60 (4H, m, 2 x $CH_2$), 4.13 (1H, m, CH), 4.53

(4H, m, 2 x $CH_2$), 7.3 (10H, m, 2 x $C_6H_5$), 7.62 (1H, s, CH); m/z 356 ($M^+$ <1%), 265, (5), 129 (5), 107 (30), 92 (15), 91 (100), 88 (15), 71 (20), 65 (15), 57 (5), 44 (20). Found: C, 71.81; H, 8.03; N, 7.95%; $M^+$ 356.2107. $C_{21}H_{28}N_2O_3$ requires: C, 71.71; H, 7.66; N, 7.60%; $M^+$ 356.2100.

(d) (R)-2-[N-(1,4-Dibenzyloxybut-2-oxy)iminomethyl]amino-2-cyanoacetamide

A solution of 2-amino-2-cyanoacetamide hydrochloride (0.19g, 1.4mmol) and (R)-N′-(1,4-dibenzyloxybut-2-oxy)-N,N-dimethylmethanimidamide (0.5g, 1.4mmol) in methanol (1.5ml) was stirred at room temperature for 20 hours. The solvent was removed under reduced pressure and the residue treated with ethyl acetate (20ml) and brine (0.05ml). The organic solution was decanted and dried ($MgSO_4$). The solvent was removed under reduced pressure and the residual oil extracted with hexane (3 x 10ml). The hexane was decanted and the residual oil was dissolved in warm chloroform (3ml) and treated with hexane (20ml). After refrigeration (-18 °C) for 1 hour, the organic solution was decanted and the insoluble oil dried under vacuum to give (R)-2-[N-(1,4-dibenzyloxybut-2-oxy)iminomethyl]amino-2-cyanoacetamide (0.37g, 74%); IR: $\nu_{max}$ -(film) 3340, 3190, 3090, 3060, 3030, 2950, 2920, 2860, 2800, 1700, 1660, 1600, 1495, 1455, 1375, 1310, 1240, 1215, 1145, 1125, 1025, 985, 945, 905, 740, 700cm$^{-1}$; $^1$H NMR: $\delta$H [$(CD_3)_2SO$] 1.87 (2H, m, $CH_2$), 3.54 (4H, m, 2 x $CH_2$), 4.05 (0.12H, m, CH), 4.13 (0.88H, m, CH), 4.45 (4H, m, 2 x $CH_2$), 5.07 (0.13H, d, J = 8Hz, CH), 5.26 (0.87H, d, J = 8Hz, CH), 6.80 (1H, d, J = 10.5Hz, CH), 6.92 (1H, dd, J = 10.5 and 8Hz, NH), 7.2-7.8 (12H, m, 2 x $C_6H_5$ plus $D_2O$ exchangeable $NH_2$); m/z ($NH_3$ CI) $M^+$ 411. Found: C, 61.83; H, 6.50; N, 13.32%. $C_{22}H_{26}N_4O_4.H_2O$ requires: C, 61.67; H, 6.59; N, 13.08%.

(e) (R)-5-Amino-4-carboxamido-1-(1,4-dibenzyloxybut-2-oxy)-imidazole

To a solution of 2-[N-(1,4-dibenzyloxybut-2-oxy)iminomethyl]amino-2-cyanoacetamide (0.32g, 0.8mmol) in dry dimethoxyethane (25ml) under an atmosphere of dry nitrogen was added boron trifluoride etherate (0.1ml, 0.8mmol). The solution was heated at 60 °C for 1 hour, cooled to room temperature and the solvent removed under vacuum. The residue was partitioned between chloroform (50ml) and saturated sodium bicarbonate (50ml). The aqueous phase was extracted with chloroform and the combined organic phases were washed with brine (30ml) and dried ($MgSO_4$). The solvent was removed to leave an oil which was chromatographed on silica, eluting with chloroform-methanol 40:1 to give, as a gum, (R)-5-amino-4-carboxamido-1-(1,4-dibenzyloxybut-2-oxy)imidazole (0.19g, 60%); IR: $\nu_{max}$(film) 3450, 3320, 3180, 3100, 3070, 3030, 2930, 2870, 2880, 1650, 1635, 1570, 1500, 1465, 1455, 1420, 1370, 1315, 1255, 1210, 1175, 1100, 1030, 1010, 750, 700cm$^{-1}$; $^1$H NMR: $\delta$H [$(CD_3)_2SO$] 2.04 (2H, m, $CH_2$), 3.63 (4H, m, 2 x $CH_2$), 4.45 (5H, m, 2 x $CH_2$ plus CH), 5.67 (2H, s, $D_2O$ exchangeable $NH_2$), 6.75 (2H, br., $D_2O$ exchangeable $NH_2$), 7.3 (11H, m, 2 x $C_6H_5$ plus CH); m/z 410 ($M^+$, 2%), 163 (5), 142 (10), 125 (5), 107 (10), 92 (10), 91 (100), 71 (10), 65 (10), 44 (5). Found: C, 64.70; H, 6.49; N, 13.15%; $M^+$ 410.1962. $C_{22}H_{26}N_4O_4$ requires: C, 64.38; H, 6.39; N, 13.65%; $M^+$ 410.1954.

(f) (R)-5-(N′-Benzoylthiocarbamoyl)amino-4-carboxamido-1-(1,4-dibenzyloxybut-2-oxy)imidazole

To a solution of (R)-5-amino-4-carboxamido-1-(1,4-dibenzyloxybut-2-oxy)imidazole (0.8g, 2mmol) in dry acetone (60ml) was added benzoylisothiocyanate (0.32ml, 2.4mmol). The solution was refluxed for 6 hours, cooled to room temperature and the solvent removed under reduced pressure. The residue was dissolved in chloroform (60ml) and washed with water (30ml) and the organic phase dried ($MgSO_4$). The solvent was removed under reduced pressure and the residue chromatographed on silica, eluting with chloroform-methanol 30:1 to give (R)-5-(N′-benzoylthiocarbamoyl)amino-4-carboxamido-1-(1,4-dibenzyloxybut-2-oxy)-imidazole (1.04g, 93%) as a glass; IR: $\nu_{max}$ (KBr) 3450, 3200, 3060, 3015, 2920, 2860, 1670, 1610, 1580, 1510, 1490, 1450, 1415, 1360, 1330, 1310, 1260, 1180, 1060, 1100, 1075, 1030, 1000, 740, 715, 700cm$^{-1}$; $^1$H NMR: $\delta$H ($CDCl_3$) 1.95 (2H, m, $CH_2$), 3.60 (4H, m, 2 x $CH_2$), 4.33 (2H, s, $CH_2$) 4.48 (2H, s, $CH_2$), 4.60 (1H, m, CH), 7.1-8.1 (18H, m, 3 x $C_6H_5$, CH plus $D_2O$ exchangeable $NH_2$), 12.00 (2H, s, $D_2O$ exchangeable $NH_2$).

(g) (R)-5-(N′-Benzoyl-S-methylthiocarbamoyl)amino-4-carboxamido-1-(1,4-dibenzyloxybut-2-oxy)imidazole

A solution of (R)-5-(N′-benzoylthiocarbamoyl)amino-4-carboxamido-1-(1,4-dibenzyloxybut-2-oxy)-imidazole (0.4g, 0.7mmol) in 0.2N sodium hydroxide solution (10ml) was cooled to 0 °C and treated with methyl iodide (0.22ml, 3.5mmol). The mixture was stirred at 0 °C for 1 hour and at room temperature for 2 hours, then neutralised by addition of acetic acid. The product was extracted into chloroform (50ml), the

organic phase dried ($MgSO_4$) and evaporated to dryness. The residue was chromatographed on silica, eluting with chloroform-methanol 30:1 to give as a glass, (R)-5-(N′-benzoyl-S-methylthiocarbamoyl)amino-4-carboxamido-1-(1,4-dibenzyl oxybut-2-oxy)imidazole (0.3g, 73%); IR: $\nu_{max}$(film) 3466, 3330, 3164, 3118, 3088, 3063, 3030, 3006, 2928, 2863, 1674, 1610, 1581, 1540, 1496, 1479, 1454, 1411, 1350, 1321, 1308, 1296, 1270, 1206, 1179, 1148, 1097, 1061, 1028, 1010, 1001, 886, 875, 860, 788, 741, 722, 699cm$^{-1}$; $^1$H NMR: $\delta$H [$(CD_3)_2SO$] 2.00 (2H, m, $CH_2$), 2.40 (3H, s, $CH_3$), 3.5-3.7 (4H, m, 2 x $CH_2$ plus CH), 4.45 (4H, m, 2 x $CH_2$), 4.62 (1H, m, CH), 7.2-7.8 (18H, m, 3 x $C_6H_5$ CH plus $D_2O$ exchangeable $NH_2$), 11.76 (1H, s, $D_2O$ exchangeable NH).

(h) (R)-9-(1,4-Dibenzyloxybut-2-oxy)guanine

A mixture of (R)-5-(N′-benzoyl-S-methylthiocarbamoyl)amino-4-carboxamido-1-(1,4-dibenzyloxybut-2-oxy)imidazole (160mg, 0.3mmol) and 7M sodium hydroxide solution (9ml) was heated to 100°C and treated with dimethylsulphoxide (3ml). The mixture was heated at 100°C for 45 minutes, cooled and neutralised with 5M hydrochloric acid. The product was extracted into chloroform (50ml) and the aqueous phase washed with chloroform (2 x 30ml). The organic phase was dried ($MgSO_4$), the solvent removed under reduced pressure and the residue treated with water (100ml) and extracted with chloroform (2 x 30ml). The organic phase was dried ($MgSO_4$), the solvent removed and the residue chromatographed on silica, eluting chloroform-methanol 20:1 to give (R)-9-(1,4-dibenzyloxybut-2-oxy)guanine (50mg, 42%) recrystallised from methanol, m.p. 214-215°C (dec); IR: $\nu_{max}$ (KBr), 3450, 3320, 3160, 3030, 2920, 2360, 2740, 1695, 1650, 1630, 1600, 1585, 1540, 1500, 1475, 1465, 1390, 1365, 1330, 1250, 1205, 1160, 1100, 1070, 1030, 1010, 910, 870, 820, 780, 740, 700, 625cm$^{-1}$; $^1$H NMR: $\delta$H [$(CD_3)_2SO$] 2.0 (2H, m, $CH_2$), 3.5-3.75 (4H, m, 2 x $CH_2$), 4.4-4.65 (5H, m, 2 x $CH_2$ plus CH), 6.50 (2H, s, $D_2O$ exchangeable $NH_2$), 7.30 (10H, m, 2 x $C_6H_5$), 7.77 (1H, s, CH), 10.65 (1H, s, $D_2O$ exchangeable NH); m/z 435 ($M^+$ <1%), 167 (10), 151 (10), 107 (30), 105 (10), 92 (15), 91 (100), 99 (10), 77 (10), 71 (10), 65 (15), 43 (10). Found: C, 63.12; H, 5.80; N, 15.71%. $C_{23}H_{25}N_5O_4$ requires C, 63.44; H, 5.79; N, 16.08%.

Description 9 (Intermediates for Example 15)

(a) (S)-N-(1,4-Dibenzyloxybut-2-oxy)phthalimide

To a solution of (R)-1,4-dibenzyloxy-2-hydroxybutane (8g, 28mmol) in dry tetrahydrofuran (150ml) was added triphenylphosphine (11g, 42mmol) and N-hydroxyphthalimide (6.8g, 42mmol). The solutoin was treated dropwise with diethyl azodicarboxylate (6.6ml, 42mmol). A dark red colouration appeared, gradually fading and the solution became warm. The solution was stirred at room temperature for 16 hours, then treated with additional triphenyl phosphine (2.25g, 8.5mmol), N-hydroxyphthalimide (1.35g, 8.5mmol) and diethyl azodicarboxylate (1.35ml, 8.5mmol). After stirring for an additonal 24 hours at room temperature, the solvent was removed under reduced pressure and the residue dissolved in ethyl acetate-hexane 1:1 (100ml) and refrigerated. A white solid crystallised out and was filtered off. The solution was evaporated to dryness and the residue dissolved in ethyl acetate-hexane 1:1 (50ml) and refrigerated. After filtration, the filtrate was evaporated to dryness and the residue chromatographed on silica, eluting with ethyl acetate-hexane 1:2 to give (S)-N-(1,4-dibenzyloxybut-2-oxy)phthalimide (11g, 91%),

$$[\alpha]^{25}_{D} - 17.4^{o}$$

(c 0.8 in ethanol); IR: $\nu_{max}$ (film) 3087, 3065, 3031, 2930, 2863, 2804, 1809 1790, 1734, 1608, 1496, 1468, 1454, 1411, 1373, 1239, 1189, 1120, 1102, 1083, 1028, 1016, 981, 878, 786, 739, 699cm$^{-1}$; $^1$H NMR: $\delta$H ($CDCl_3$) 2.05 (2H, m, $CH_2$), 3.75 (4H, m, 2 x $CH_2$), 4.4-4.7 (5H, m, 2 x $CH_2$ plus CH), 7.1-7.9 (14H, m, 2 x $C_6H_5$ plus $C_6H_4$), m/z (FAB) $MH^+$ 432.

(b) (S)-1,4-Dibenzyloxybut-2-oxyamine

A solution of (S)-N-(1,4-dibenzyloxybut-2-oxy)phthalimide (5g, 11.6mmol) in dichloromethane (50ml) was treated with methylhydrazine (0.8ml, 15mmol). A deep red colouration developed, disappearing gradually as a white solid precipitated out of solution. After stirring at room temperature for 1 hour, the solid was filtered off and the filtrate was washed with a 3% sodium carbonate solution. After drying ($MgSO_4$) the solution was

evaporated to dryness and the residual oil was chromatographed on silica eluting with ethyl acetate-hexane 5:1 to give (S)-1,4-dibenzyloxybut-2-oxyamine (2.66g, 76%), $[\alpha]_D^{25}$ -13.2° (c 0.3 in ethanol); IR: $\nu_{max}$ (film) 3315, 3248, 3087, 3062, 3030, 2922, 2861, 1588, 1496, 1454, 1365, 1205, 1101, 1028, 738, 698cm$^{-1}$; $^1$H NMR: δH ($CDCl_3$) 1.85 (2H, m, $CH_2$), 3.55 (4H, m, 2 x $CH_2$), 3.90 (1H, m, CH), 4.49 (2H, s, $CH_2$), 4.55 (2H, s, $CH_2$), 5.36 (2H, s, $D_2O$ exchangeable $NH_2$), 7.30 (10H, m, 2 x $C_6H_5$); m/z 302 ($MH^+$), 301 ($M^+$, 3%), 181 (5), 163 (10), 106 (15), 105 (5), 92 (15), 91 (100), 71 (15), 65 (10). Found: C, 71.40; H, 7.66, N, 4.61%, $M^+$ 301.1670. $C_{18}H_{23}NO_3$ requires: C, 71.73; H, 7.69, N, 4.65% $M^+$ 301.1678.

(c) (S)-4-Chloro-6-(1,4-dibenzyloxybut-2-oxyamino)-2,5-diformamidopyrimidine

A solution of (S)-N-1,4-dibenzyloxybut-2-oxyamine (2.17g, 7.2mmol), 4,6-dichloro-2,5-diformamidopyrimidine (1.7g, 7.2mmol) and triethylamine (3ml, 2.4mmol) in dioxan (50ml) was heated at 100°C for 1.5 hours. The mixture was cooled to room temperature, filtered and the solvent removed under reduced pressure. The residue was chromatographed on silica eluting with chloroform-methanol 30:1 to give as an oil, (S)-4-chloro-6-(1,4-dibenzyloxybut-2-oxyamino)-2,5-diformamidopyrimidine (2.59g, 71%); IR: $\nu_{max}$ (film) 3241, 3062, 3031, 2924, 2863, 1695, 1635, 1587, 1567, 1496, 1477, 1454, 1417, 1388, 1365, 1248, 1207, 1094, 1028, 905, 803, 775, 739, 698cm$^{-1}$; $^1$H NMR: δH [$(CD_3)_2SO$] 1.94 (2H, m, $CH_2$), 3.60 (4H, m, 2 x $CH_2$), 4.13 (1H, m, CH), 4.47 (2H, m, 2 x $CH_2$), 7.28 (10H, m, 2 x $C_6H_5$), 7.85 and 8.14 (1H, m, CH), 9.16-9.41 (2H, m, CH plus $D_2O$ exchangeable NH). Found: C, 57.67; H, 5.34; N, 13.40%. $C_{24}H_{25}ClN_6O_5$ requires C, 57.77; H, 5.05; N, 14.04%.

(d) (S)-6-Chloro-9-(1,4-dibenzyloxybut-2-oxy)-2-formamido-purine

A solution of (S)-4-chloro-6-(1,4-dibenzyloxybut-2-oxyamino)-2,5-diformamidopyrimidine (0.7g, 1.4mmol) in diethoxymethyl acetate (15ml) was heated at 100°C for 1.5 hours. The solvent was removed under reduced pressure and the residue dissolved in methanol (20ml) and treated with 0.88 ammonia solution (0.5ml). After stirring at room temperature for 30 minutes, the solvent was removed and the residue co-evaporated with methanol (3 x 20ml). The residue was chromatographed on silica eluting with chloroform-methanol 60:1 to give, as an oil, (S)-6-chloro-9-(1,4-dibenzyloxybut-2-oxy)-2-formamidopurine (0.57g, 84%); IR: $\nu_{max}$ (film) 3226, 3168, 3120, 3089, 3063, 3030, 3007, 2920, 2864, 1704, 1612, 1576, 1504, 1476, 1454, 1439, 1388, 1326, 1208, 1138, 1099, 1028, 1018, 995, 922, 862, 784, 739, 699, 655, 621, 608cm$^{-1}$; $^1$H NMR: δH [$(CD_3)_2SO$] 2.04 (2H, m, $CH_2$), 3.55-3.8 (4H, m, 2 x $CH_2$), 4.30-4.55 (4H, m, 2 x $CH_2$), 4.76 (1H, m, CH), 7.05-7.4 (10H, m, 2 x $C_6H_5$), 8.64 (1H, s, CH), 9.33 (1H, br.s, CH), 11.27 (1H, s, $D_2O$ exchangeable NH).

Description 10 (Intermediates for Examples 16-19)

(a) 2-Hydroxymethyl-1,2-propanediol

2N Borane: dimethylsulphide complex in tetrahydrofuran (170.5ml) was added to triethylmethanetricarboxylate (24.9g 0.107 mol) under nitrogen. The reaction was heated under reflux and dimethylsulphide removed. After 8 hours the reaction was cooled, methanol (100ml) added and the solution stirred for 15 hours. The solvent was removed under reduced pressure and the residue co-evaporated with methanol (3x50ml). Column chromatography on silica gel (eluted with chloroform:methanol (3.1)) gave 2-hydroxymethyl-1,3-propanediol (9.43g; 83%), m.p. 65-68°C. IR:$\nu_{max}$ (KBr) 3267, 2944, 28o1, 1489, 1479, 1113, 1058, 1006 cm$^{-1}$. $^1$H NMR: $\delta_H$[$(CD_3)_2SO$] 1.60 (1H, septet, J=6Hz,CH), 3.40(6H, t, J=6Hz, 3 x $CH_2$), 4.25 (3H, t, J=6Hz, $D_2O$ exchangeable, 3 x OH). Found: C,45.29; H,9.77%. $C_4H_{10}O_3$ requires: C,45.26; H,9.52%.

(b) 2,2-Dimethyl-5-hydroxymethyl-1,3-dioxan

A mixture of 2-Hydroxymethyl-1,3-propanediol (9.0g, 61.6 mmol), 2,2-dimethoxypropane (11.7ml, 95.2 mmol) 4-toluenesulphonic acid monohydrate (0.49g, 2.58 mmol) and tetrahydrofuran (450ml) was stirred at 20°C for 1 hour. Triethylamine (5ml) was then added and the solvent removed under reduced presure. Chromatography on silica gel (eluted with chloroform ethanol, 10.1) afforded the title compound (9.62g, 78%) as a colourless oil. IR: $\nu_{max}$ (film) 3431, 2993, 2943, 2874, 1482, 1456, 1373 cm$^{-1}$. $^1$H NMR: $\delta_H$[$(CD_3)_2SO$] 1.30(6H, s, 2 x $CH_3$), 1.69(1H,m,CH), 3.38(2H,dd, J=5.2, 6.6Hz, $\underline{CH_2}$OH), 3.61(2H,dd, J=11.8, 7.1Hz, 2 x H(ax)), 3.82 (2H, dd, J=11.8, 4.4Hz, 2 x H(eq)), 4.53(1H, t, J=5.2Hz, $D_2O$ exchangable, OH). Found: C,56.73; H,9.80%. $C_7H_{14}O_3.0.1H_2O$ requires: C,56.80; H,9.69%.

(c) N-(2,2-Dimethyl-1,3-dioxan-5-ylmethoxy)phthalimide

A mixture of 2,2-Dimethyl-5-hydroxymethyl-1,3-dioxan (9.60g, 65.8 mmol), triphenylphosphine (20.74g, 79.2 mmol), N-hydroxyphthalimide (12.90g, 79.2 mmol), diethyl azodicarboxylate (12.45ml, 79.2 mmol) and tetrahydrofuran (300 ml) was stirred at 20°C for 16 hours. The solvent was then removed under reduced pressure, the residue triturated with ether, filtered and the filtrate evaporated. The process was repeated and then the residue was chromatographed on silica (eluted with hexane: acetone, 3:1; then hexane: acetone 5:2), affording the title compound (16.39g, 86%). IR: $\nu_{max}$ (KBr) 3500, 2988, 2880, 1791, 1726, 1702, 1466, $cm^{-1}$. $^1$H NMR: $\delta_H$[$(CD_3)_2SO$] 1.32(3H,s,$CH_3$), 1.35(3H, s,$CH_3$, 2.04(1H,m,CH), 3.77(2H,dd, J=11.9, 6.0Hz, 2 x H(ax)), 4.00 (2H,dd, J=11.9, 4.1 Hz, 2 x H(eq)) 4.22(2H,d, J=7.0 Hz, $CH_2ON$), 7.86(H,s, aromatic). Found: C, 61.77; H,5.79; N,4.88%. $C_{15}H_{17}NO_5$ requires: C,61.84; H,5.89; N, 4.81%.

(d) (2,2-Dimethyl-1,3-dioxan-5-ylmethoxy)amine

Methylhydrazine (0.55ml, 10.3 mmol) was added to a stirred solution of N-(2,2-dimethyl-1,3-dioxan-5-ylmethyloxy)phthalimide (2g, 6.87 mmol) in dichloromethane (15 ml) at 0°C. The solution was then allowed to warm to 20°C and stirred for 1 hour. The suspension was filtered, the filtrate evaporated to dryness and the residue triturated with ether (20ml). The suspension was filtered and the residue obtained on evaporation of the filtrate was chromatographed on silica (eluted with chloroform-ethanol, 100:1), yielding the title compound (0.87g, 79%). IR: $\nu_{max}$(film) 3320, 3000, 2950, 2875, 1600, 1480, 1435$cm^{-1}$. $^1$H NMR: $\delta_H$-[$(CD_3)_2SO$]1.29(3H,s,$CH_3$), 1.30(3H,s,$CH_3$), 1.95(1H,m,CH), 3.51(2H,d, J=6.9Hz, $CH_2ON$), 3.58(2H,dd, J=11.8, 6.9Hz, 2 x H(ax)), 3.84(2H,dd, J=11.8, 4.4Hz, 2 x H(eq)), 5.97(2H, br.s, $D_2O$ exchangeable, $NH_2$).

(e) 4-Chloro-2,5-diformamido-6-(2,2-dimethyl-1,3-dioxan-5-ylmethoxyamino)pyrimidine

A mixture of 4,6-dichloro-2,5-diformamidopyrimidine (3.25g, 13.8 mmol), 2,2-dimethyl-1,3-dioxan-5-ylmethoxyamine (2.26g, 14.0 mmol), trithylamine (5ml) and dioxan (50ml) was heated at reflux temperature for 2 hours. The suspension was cooled, filtered and the filtrate evaporated under reduced pressure. The residue was chromatographed on silica gel (eluted with chloroform-ethanol, 50:1 then 30:1), yielding the title compound (2.10g, 42%). IR: $\nu_{max}$(KBr) 3240, 1690, 1585, 1570, 1480, 1420 $cm^{-1}$. $^1$H NMR: $\delta_H$[$(CD_3)_2SO$] 1.30(3H,s,$CH_3$), 1.34(3H,s,$CH_3$), 1.99(1H,m,CH), 3.70(2H,m, 2 x H(ax)), 3.93(4H,m,$CH_2ON$, 2 x H (eq)), 8.15, 8.31(1H, 2 x s, NHCHO), 9.17, 9.42(1H, 2 x br.s, $D_2O$ exchangeable, NHCHO), 9.26(1H, br.s, NHCHO), 10.83(2H, br.s, $D_2O$ exchangeable, NHCHO, NHO). Found: C,42.93; H,5.09; N,19.75%. $C_{13}H_{18}ClN_5O_5$ requires: C,43.39; H,5.05; N, 19.47%.

(f) 6-Chloro-9-(2,2-dimethyl-1,3-dioxan-5-ylmethoxy)-2-formamidopurine.

4-Chloro-2,5-diformamido-6-(2,2-dimethyl-1,3-dioxan-5-ylmethoxyamino)pyrimidine (1.9g, 5.28 mmol) in diethoxymethylacetate (25ml) was heated at 120°C for 2 hours. The mixture was then cooled and evaporated to a syrup. The residue was dissolved in methanol (70ml) and concentrated aqueous ammonia (2.5ml). The solution was then stirred at 20°C for 1 hour, evaporated under reduced pressure and the residue co-evaporated with methanol. Column chromatography on silica gel (eluted with chloroform-methanol, 50:1) gave the title compound (1.47g, 81%). IR: $\nu_{max}$(KBr) 3419, 1720, 1616, 1579, 1513, 1507, 1439$cm^{-1}$. $^1$H NMR:
$\delta_H$[$(CD_3)_2SO$]1.32(3H,s,$CH_3$), 1.37(3H,s,$CH_3$), 2.04(1H,m,CH), 3.80(2H,dd, J=11.8, 5.5Hz, 2 x H(ax)), 4.03-(2H,dd, J=12.1, 3.9Hz, 2 x H(eq)), 4.51(2H,d, J=7.3Hz,$CH_2ON$), 8.84(1H,s,H-8),9.38(1H,s,CHO), 11.31(1H, br.s, $D_2O$ exchangeable, $NH_2$). $M^+$ observed 341.0891. $C_{13}H_{16}ClN_5O_4$ requires: 341.0891.

(g) 2-Amino-9-(2,2-dimethyl-1,3-dioxan-5-ylmethoxy)purine.

A mixture of 4-Chloro-9-(2,2-dimethyl-1,3-dioxan-5-ylmethoxy)-2-formamidopurine (1.47g, 4.30 mmol), 10% palladium on charcoal (75mg), ammonium formate (3.0g, 47.6 mmol) and methanol (50ml) was stirred at reflux temperature for 4 hours. Additional ammonium formate (0.75g) was added after 1.5, 2 and 3 hours. After cooling the mixture was evaporated to dryness and the residue partioned between ethyl acetate (50ml) and water (50ml). The phases were separated and the aqueous layer extracted with ethyl acetate (25ml). The combined ethyl acetate extracts were washed with water (25ml) dried (magnesium sulphate) and evaporated under reduced pressure. The residue was dissolved in methanol (25ml) and hydrazine hydrate (2ml). The solution was heated at reflux temperature for 45 minutes, cooled and evaporated under reduced

pressure. Column chromatography on silica gel (eluted with chloroform-methanol, 15:1) gave the title compound. (530mg, 43%). IR: $\nu_{max}$ (KBr) 3327, 3193, 1655, 1622, 1580, 1515, 1434 $cm^{-1}$. $^1H$ NMR: $\delta_H$[-$(CD_3)_2SO$] 1.33(3H,s,$CH_3$), 1.35(3H,s,$CH_3$), 2.02(1H,m,CH), 3.79(2H,dd, J=12.1, 5.8Hz, 2 x H(ax)), 4.05-(2H,dd, J=12.1, 4.1Hz, 2 x H(eq), 4.39(2H,d, J=7.1Hz, $CH_2ON$), 6.70(2H,br,s.,$NH_2$), 8.34(1H,s,H-8),8.59 (1H,s,H-6). Found: C,51.33: H,6.20; N,25.18%; $M^+$ 279. 1339. $C_{12}H_{17}N_5O_3$ requires: C,51.59; H,6.15; N,25.08%; $M^+$ 279.1331.

Description 11 (Intermediate for Example 20)

2-Amino-9-(2,2-dimethyl-1,3-dioxan-5-ylmethoxy)-6-methoxypurine.

6-Chloro-9-(2,2-dimethyl-1,3-dioxan-5-ylmethoxy)-2-formamidopurine (0.5g, 1.46 mmol) in 1.2M sodium methoxide in methanol (3.38ml) and methanol (5ml) was heated at reflux temperature for 1.5 hours and then cooled. Acetic acid (0.16ml) was added and the solution evaporated to dryness. The residue was suspended in water and extracted with ethyl acetate (2 x 50ml). The combined ethyl acetate extracts were washed with water, dried (magnesium sulphate) and evaporated under reduced pressure. Chromatography on silica gel (eluted with chloroform-ethanol, 100:1) gave the title compound (310mg, 69%). IR:$\nu_{max}$ (KBr) 3397, 3208, 1640, 1616, 1581, 1480, 1390 $cm^{-1}$. $^1H$ NMR: $\delta_H$ [$(CD_3)_2SO$] 1.32(3H,s,$CH_3$), 1.35(3H,s,$CH_3$), 2.00(1H,m,CH), 3.77(2H,dd, J=11.8, 6.1Hz, 2 x X(ax), 3.9 (3H,s,$OCH_3$), 3.99(2H,dd,J=11.8, 4.1Hz, 2 x H-(eq)), 4.36(2H,d, J=6.8Hz, $CH_2ON$), 6.60(2H,br.s, $D_2O$ exchangeable, $NH_2$), 8.14(1H,s,H-8). Found: C,49.30; H, 6.12; N,22.03%; $M^+$ 309.1455. $C_{13}H_{19}N_5O_4$ $0.5H_2O$ requires: C,49.04; H,6.34; N,22.00%; $M^+$ 309.1437.

Description 12 (Intermediate for Example 21)

2,6-Diamino-9-(2,2-dimethyl-1,3-dioxan-5-ylmethoxy)purine

A mixture of 6-Chloro-9-(2,2-dimethyl-1,3-dioxan -5-ylmethoxy)-2-formamidopurine (630mg, 1.84 mmol), ammonia (10ml) and methanol (15ml) was heated at 110 °C for 7.5 hours in an autoclave and then allowed to cool over 16 hours. The mixture was evaporated to dryness and the residue chromatographed on silica (eluted with chloroform-ethanol, 20:1), affording the title compound (340mg, 63%). IR: $\nu_{max}$ (KBr) 3409, 3321, 3158, 1669, 1640, 1589, 1488, 1457, 1409 $cm^{-1}$. H NMR: $\delta_H$ [$(CD_3)_2SO$] 1.32(3H,s,$CH_3$), 1.35-(3H,s,$CH_3$), 2.00(1H,m,$CH_3$), 3.77(2H,dd, J=11.8, 6.1Hz, 2 x H(ax)), 3.98(2H,dd, J=11.8, 4.1Hz, 2 x H(eq)), 4.32(2H,d, J=7.1Hz, $CH_2ON$), 5.91(2H,br.s, $D_2O$ exchangeable, 6-$NH_2$), 6.78(2H.br,s,$D_2O$ exchangeable, 2-$NH_2$), 7.96(1H,s,H-8). Found: C,48.34; H,6.18; N,28.21%; $M^+$ 294.1437. $C_{12}H_{18}N_6O_3.0.2H_2O$ requires: C,48.37; H,6.24; N,28.21%; $M^+$ 294.1440.

Description 13 (Intermediates for Examples 10,22-24)

(a) 3-Bromo-1-t-butyldimethylsilyloxypropane.

A mixture of 3-Bromo-1-propanol (10g, 6.51ml, 71.9 mmol), t-butyldimethylsilylchloride (13.0g, 86.2 mmol), imidazole (12.24g, 180 mmol) and N,N-dimethylformamide (60ml) was stirred at 20 °C for 24 hours. The reaction was then poured into water (300ml) and extracted with ether (2 x 200ml). The combined ether extracts were washed with dilute hychrochloric acid (50ml), brine (50ml), dried (magnesium sulphate) and evaporated to dryness. IR: $\nu_{max}$ (film) 2956, 2930, 2858, 1473, 1257, 1106, $cm^{-1}$. $^1H$ NMR: $\delta_H$ [($CDCl_3$) 0.00-(6H,s, 2 x $CH_3Si$), 0.80(9H,s, 3 x $CH_3C$), 1.90(2H,m, $CH_2\underline{CH_2}CH_2$), 3.70(4H,m,$CH_2O$,$CH_2Br$).

(b) N-(3-t-Butyldimethylsilyloxy-1-propoxy)-phthalimide.

N-Hydroxyphthalimide (9.81g, 60.2 mmol) was added portionwise to a stirred suspension of sodium hydride (60%, 2.41g, 60.2 mmol) in N,N-dimethylformamide (100 ml) at 20 °C. After 20 minutes 3-bromo-1-t-butyldimethylsilyloxypropane (15.22g, 60.2 mmol) was added and the mixture heated at 50 °C for 24 hours.

The mixture was cooled, poured into water (300 ml) and extracted with ether (2 x 200 ml). The combined ether extracts were washed with brine (100 ml), dried and evaporated under reduced pressure. Chromatography on silica gel (eluted with hexane: acetone, 5:1) gave the title compound (10.80g, 53%). IR: $\nu_{max}$ (film) 2955, 2930, 2857, 1791, 1737, 1468 $cm^{-1}$. $^1H$ NMR: $\delta_H CDCl_3$) 0.05(6H,s, 2 x $CH_3Si$), 0.90(9H,s, 3 x $CH_3C$), 1.90(2H, quintet, J=6Hz, $CH_2\underline{CH_2}CH_2$), 3.80(2H,t, J=6Hz, $CH_2OSi$), 4.25(2H,t, J=6Hz, $CH_2ON$),

7.75(4H,s,aromatic). Found: $^{m}/z$ 320.1324. $C_{16}H_{22}NO_4Si$-$CH_3$ requires $^{m}/z$ 320.1318.

(c) 3-t-Butyldimethylsilyloxy-1-propoxyamine.

Methylhydrazine (2.5ml, 40.0 mmol) was added to N-(3-t-butyldimethylsilyoxyprop-1-oxy)phthalimide (10.5g, 31.3 mmol) in dichloromethane (70 ml) at 0 °C. The suspension was then allowed to warm to 20 °C and stirred for 1 hour. The suspension was filtered, the filtrate evaporated to dryness and the residue triturated with ether (20ml). The suspension was filtered and the residue obtained on evaporation of the filtrate was chromatographed on silica (eluted with chloroform-hexane, 10:1), affording the title compound (5.13g, 80%). IR:$\nu_{max}$ (film) 2956, 2930, 2858, 1588, 1473, 1464 $cm^{-1}$. $^1H$ NMR: $\delta_H$ ($CDCl_3$) 0.0(6H,s, 2 x $CH_3Si$), 0.85(9H,s, 3 x $CH_3C$), 1.70(2H, quintet, J=6Hz, $CH_2CH_2CH_2$), 3.60(2H, t, J=6Hz, $CH_2O$), 3.70 (2H, t, J=6Hz, $CH_2O$), 5.20(2H,br.s, $D_2O$ exchangeable, $NH_2$).

(d) 6-(3-t-Butyldimethylsilyloxy-1-propoxyamino)-4-chloro-2,5-diformamidopyrimidine.

A mixture of 4,6-dichloro-2,5-diformamidopyrimidine (3g, 12.8 mmol), 3-t-butyldimethylsilyloxyprop-1-oxyamine (2.62g, 12.8 mmol), triethylamine (5ml) and dioxan (50ml) was heated under reflux for 3 hours. The suspension was cooled, filtered and the filtrate evaporated under reduced pressure. The residue was chromatographed on silica (eluted with chloroform-ethanol, 50:1), yielding the title compound (2.15g, 41%). $^1H$ NMR: $\delta_H$ ($CDCl_3$) 0.0(6H,s, 2 x $CH_3Si$), 0.89(9H,s, 3 x $CH_3C$), 1.90(2H, quintet, J=6.3, 6.1Hz, $CH_2CH_2CH_2$), 3.77(2H, t, J=6Hz, $CH_2OSi$), 4.07 (2H, t, J=6Hz, $CH_2ON$), 7.85(1H,br.d, J=10Hz, NHCHO), 8.34(1H,s,NNCHO), 8.75, 8.76(1H, 2 x s, NH), 9.40(1H,d, J=10Hz, NHCHO).

(e) 9-(3-t-Butyldimethylsilyloxyprop-1-oxy)-6-chloro-2-formamidopurine

6-(3-t-Butyldimethylsilyloxyprop-1-oxyamino)-4-chloro -2,5-diformamidopyrimidine (2.15g, 5.33 mmol) in diethoxymethyl acetate (20ml) was heated at 120 °C for 1.5 hours. The mixture was then cooled and evaporated to a syrup. The residue was dissolved in methanol (20ml) and concentrated aqueous ammonia (0.5ml). The solution was then stirred for 30 minutes at 20 °C, evaporated under reduced pressure and the residue co-evaporated with methanol. Column chromatography on silica gel (eluted with chloroform-ethanol, 50:1) afforded the title compound (1.66g, 81%). IR: $\nu_{max}$ (KBr) 3125, 2956, 2930, 1718, 1700, 1613, 1583, 1508, 1439 $cm^{-1}$. $^1H$ NMR: $\delta_H$ [$(CD_3)_2SO$] 0.04(6H,s, 2 x $CH_3Si$), 0.85(9H,s, 3 x $CH_3C$), 1.90(2H, quintet, J=6.2Hz, $CH_2CH_2CH_2$), 3.79(2H, t, J=6.2Hz, $CH_2OSi$), 4.50 (2H, t, J=6.2Hz, $CH_2ON$), 8.81(1H,s,H-8), 9.36-(1H,s,CHO), 11.31 (1H,br.s, $D_2O$ exchangeable, NHCHO). Found C,46.94; H,6.26; N,17.96%. $C_{15}H_{24}ClN_5O_3Si$ requires: C,46.67; H,6.28; N,18.15%.

(f) 9-(3-t-Butyldimethylsilyloxyprop-1-oxy)-2-formamidopurine

A mixture of 9-(3-t-Butyldimethylsilyloxyprop-1-oxy)-6-chloro-2-formamidopurine (1.60g, 4.15 mmol), 10% palladium on charcoal (80 mg), ammonium formate (1.8g, 24.9 mmol) and methanol (50 ml) was heated under reflux for 3 hours. Additional ammonium formate (0.8 g) was added after 1 and 2 hours. The mixture was then cooled, evaporated to dryness and the residue partioned between ethyl acetate (50 ml) and water (50 ml). The phases were separated and the aqueous phase extracted with ethyl acetate (25 ml). The combined organic phases were washed with water (25 ml), dried (magnesium sulphate) and evaporated under reduced pressure. Column chromatography on silica (eluted with chloroform-ethanol, 30:1) afforded the title compound (0.81g, 56%).
IR: $\nu$max (KBr)3120, 2950, 2925, 1695, 1615, 1410$cm^{-1}$.
$^1H$ NMR: $\delta_H$[$(CD_3)_2SO$] 0.04(6H,s,2x$CH_3Si$), 0.85(9H,s,3x$CH_3C$), 1.90(2H, quintet, J=6.3Hz,$CH_2CH_2CH_2$), 3.79(2H,t,J=6.3Hz, $CH_2OSi$), 4.49(2H,t, J=6.3Hz, $CH_2ON$), 8.72(1H,s,H-8), 8.98(1H,s,H-6), 9.43-(1H,d,J=9.1Hz, NHCHO), 11.10 (1H,d, J=9.3Hz, $D_2O$ exchangeable, NHCHO).

Description 14 (Intermediates for Example 27)

(a) (R)-N-(2,2-Dimethyl-1,3-dioxolan-4-yl methoxy)phthalimide

To a solution of (S)-2,2-dimethyl-1,3-dioxolane-4-methanol (27g, 0.2 mol), triphenylphosphine (53.5g, 0.2 mol) and N-hydroxyphthalimide (33.3g, 0.2 mol) in dry tetrahydrofuran (500ml) cooled to 0 °C was added diethyl azodicarboxylate (38.5g, 0.22 mol). After stirring for 18 hours at 20 °C the originally dark red solution

turned pale yellow and was then evaporated to dryness under reduced pressure. The residue was loaded onto a silica column in chloroform and eluted with hexane-acetone (3:1), yielding (R)-N-(2,2-dimethyl-1,3-dioxolan-4-ylmethoxy)phthalimide as white plates (31.2g, 55%), m.p. 100-2°C. $[\alpha]_D^{20}$ = +15.6°(C 0.98 in methanol); IR: $\nu_{max}$ (nujol) 1790, 1770, 1720, 1600 $cm^{-1}$; $^1H$ NMR: $\delta_H$ ($CDCl_3$), 1.35 (3H, s, $CH_3$), 1.41 (3H, s, $CH_3$), 3.97 (1H, q, J=5.5, 8.8Hz, $CH_2ON/CH_2OC$), 4.17 (2H, m, 1H of $CH_2ON$+1H of $CH_2OC$), 4.32 (1H, q, J=5.5, 10.0Hz, $CH_2ON/CH_2OC$), 4.50 (1H, m, CH), 7.74-7.87 (4H, m, aromatic); $^m/z$ 262 (M+-$CH_3$, 50%).

| | | | |
|---|---|---|---|
| Found: | C, 60.75; | H, 5.45; | N, 5.05%. |
| $C_{14}H_{15}NO_5$ requires | C, 60.64; | H, 5.45; | N, 5.05%. |

(b) <u>(R)-(2,2-Dimethyl-1,3-dioxolan-4-yl-methoxy)amine</u>

A solution of (R)-N-(2,2-dimethyl-1,3-dioxolan-4-ylmethoxy) phthalimide (10g, 36 mmol) in dichloromethane (150ml) was cooled to 0°C and treated with N-methylhydrazine (2.66g, 58 mmol). The reaction was stirred for 1 hour at 25°C, filtered and evaporated. Ether was added, the suspension filtered and the filtrate evaporated to dryness under reduced pressure. The residue was chromatographed on silica eluting with ethyl acetate, affording (R)-2,2-dimethyl-1,3-dioxolan-4-ylmethoxy)amine (4.7g, 89%) as a pale yellow liquid. IR: $\nu_{max}$ (film) 3550, 3300, 1600 $cm^{-1}$; $^1H$ NMR $\delta_H$ ($CDCl_3$) 1.38 (3H, s, $CH_3$), 1.44 (3H, s, $CH_3$), 3.73 (3H, m, $CH_2ON$+1H of $CH_2OC$), 4.07 (1H, q, J=6.4, 8.2Hz, 1H of $CH_2OC$), 5.56 (2H, br. s, $D_2O$ exchangeable, $NH_2$); $^m/z$ 132 (M+-$CH_3$, 29%).

| | | | |
|---|---|---|---|
| Found: | C, 48.58; | H, 8.90; | N, 9.02%. |
| $C_6H_{13}NO_3$ requires | C, 48.97; | H, 8.90; | N, 9.52%. |

(c) <u>(R)-4-Chloro-2,5-diformamido-6-(2,2-dimethyl-1,3-dioxolan-4-ylmethoxyamino)pyrimidine.</u>

A mixture of 4, 6-dichloro-2,5-diformamidopyrimidine (3.5g, 15 mmol), (R)-(2,2-dimethyl-1,3-dioxolan-4-yl-methoxy)amine (2.65g, 18 mmol) and triethylamine (20 ml, 150 mmol) in dioxan (80ml) was stirred at 100°C for 4 hours. The reaction was cooled, filtered and evaporated under reduced pressure. The residue was chromatographed on silica, eluting with chloroform-methanol (10:1), affording slightly impure (R)-4-chloro-2,5-diformamido-6-(2,2-dimethyl-1,3-dioxolan-4-ylmethoxyamino)pyrimidine (1.05g, 20%) as pale orange solid. IR: $\nu_{max}$ (nujol) 3400, 3300, 3180, 1690, 1650 $cm^{-1}$;
$^1H$ NMR: $\delta_H$ ($CDCl_3$) 1.39 (3H, s, $CH_3$), 1.47(3H,s,$CH_3$), 3.81 (1H, q, J=6.3, 8.2Hz, $CH_2OC$), 4.03 (2H, d, J=5.5 Hz, $CH_2ON$), 4.11 (1H, q, J=6.6, 8.2Hz, $CH_2OC$), 4.41 (1H, m, <u>CH</u>OC), 7.29 (1H, s, $D_2O$ exchangeable, NH), 7.97 (1H, br. d, J=10.5Hz, $D_2O$ exchangeable, NH), 8.34 (<u>1H</u>, s, CHO), 8.94 (1H, br. s, $D_2O$ exchangeable, NH), 9.43 (1H, d, J=10.5Hz, CHO). $^m/z$ FAB (+ve ion thioglycerol) 346 ($MH^+$, 100%).

(d) <u>(R)-6-Chloro-9-(2,2 dimethyl-1,3-dioxolan-4-yl methoxy)-2-formamidopurine</u>

A solution of (R)-4-chloro-2,5-diformamido-6-(2,2-dimethyl-1,3-dioxolan-4-ylmethoxyamino)pyrimidine (900mg, 2.6 mmol) in diethoxymethyl acetate (20ml) was stirred at 120°C for 3 hours. The solvent was removed under reduced pressure, the residue dissolved in methanol (20ml) containing 0.88 ammonia solution (0.5ml) and the mixture stirred for 1 hour at 25°C. The solvents were evaporated under reduced pressure and the residue chromatographed on silica, eluting with ethyl acetate-hexane (2:1), affording (R)-6-chloro-9-(2,2-dimethyl-1,3-dioxolan-4-ylmethoxy)-2-formamidopurine (530mg, 62%), m.p. 145-8°C. UV: λmax (MeOH) 232 (ε 26,800), 291 (ε 9,600)nm; IR: $\nu_{max}$ (nujol) 1700, 1600, 1580 cm-1; $^1H$ NMR: $\delta_H$ ($CDCl_3$) 1.37 (3H,s, $CH_3$), 1.42 (3H, s, $CH_3$), 3.87 (1H, m, $CH_2OC$), 4.12 (1H, M, $CH_2OC$), 4.46 (3H, m, CH+$CH_2ON$), 8.20 (1H, s, H-8), 8.44 (1H, br. d, J=11.5 Hz, $D_2O$ exchangeable, NH), 9.55 (1H, d, J=11.5Hz, <u>H</u>CONH).

| | | | |
|---|---|---|---|
| Found: | C, 43.94; | H, 4.34; | N, 21.59%. |
| $C_{12}H_{14}ClN_5O_4$ requires | C, 43.98; | H, 4.31; | N, 21.37%. |

Description 15 (Intermediates for Examples 28, 29).

(a) 0-(4-Butenyl)benzohydroxamate

To a suspension of 60% sodium hydride (8g = 4.8g NaH, 0.2 mol) in anhydrous N,N-dimethylformamide (200 ml) was added benzohydroxamic acid (27.5g, 0.2 mol) over 20 minutes and the reaction stirred for 1 hour at 25°C. This solution was treated with 4-bromo-1-butane (27g, 0.2 mol) and the reaction stirred at 100°C for 6 hours. After cooling, water (400 ml) was added and the solution was extracted with hexane (3 x 200ml). The aqueous layer was evaporated under reduced pressure, the residue suspended in ethyl acetate (600ml), washed with water (2 x 200ml) and dried ($MgSO_4$). Evaporation of the solvent gave a residual oil which was distilled under high vacuum affording 0-(4-butenyl) benzohydroxamate (19.4g, 51%). b.p.$_{0.2}$ 140-5°C. IR: $\nu_{max}$ (film) 3200, 1640, 1600, 1580, 1510 $cm^{-1}$; $^1$HNMR: $\delta_H$ ($CDCl_3$) 2.44 (2H, q, J=7Hz, $\underline{CH_2}CH_2O$), 4.07 (2h, t, J=7.8 Hz, $CH_2ON$), 5.12 (2H, m, $\underline{CH_2}$=CH), 5.80 (1H, m, $\underline{CH}=CH_2$), 7.26-7.90(5H, m, aromatic), 9.44 (1H, br.s, $D_2O$ exchangeable, NH).

(b) 0-(4-Butenyl)hydroxylamine hydrochloride

0-(4-Butenyl)benzohydroxamate (10g, 52.4 mmol) was dissolved in ethanol (30ml), treated with concentrated hydrochloric acid (15ml) and boiled under reflux for 3 hours. The reaction was cooled, diluted with water (60ml) and extracted with chloroform (3 x 100ml). The aqueous layer was evaporated to dryness and the residue recrystallised from ethanol-ether, affording 0-(4-butenyl)hydroxylamine hydrochloride (4.6g, 71%) as white plates, m.p. 136-40°C; IR: $\nu_{max}$ (KBr) 3400, 3100, 2900, 1650, 1590, 1550, 1515, $cm^{-1}$; $^1$H NMR: $\delta_H$ $[(CD_3)_2SO]$ 2.32 (2H, m, $\underline{CH_2}CH_2ON$), 4.06 (2H, t, J=7Hz, $CH_2ON$), 5.07 (2H, m, $\underline{CH_2}$=CH), 5.72 (1H, m, $\underline{CH}=CH_2$), 11.12 (3H, br.s, $D_2O$ exchangeable, $N^+H_3$). Found: C, 38.23; H, 8.13; N, 11.05%. $C_4H_{10}ClNO$ requires C, 38.87; H, 8.16; N, 11.33%.

(c) 6-(4-Butenyloxyamino)-4-chloro-2,5-diformamidopyrimidine

A solution of 0-(4-butenyl)hydroxylamine hydrochloride (1.94g, 15.7 mmol) and triethylamine (3.96g, 5.5ml, 39.3 mmol) in dioxan (80ml) was stirred for 1 hour at 50°C. The syspension was cooled and filtered and then 4,6-dichloro-2,5-diformamidopyrimidine (3.35g, 14.3 mmol) added to the filtrate. The solution was stirred at 100°C for 4 hours, cooled, filtered and evaporated to dryness. The residue was chromatographed on silica eluting with ethyl acetate-hexane (5:1), affording 6-(4-butenyloxyamino)-4-chloro-2,5-diformamidopyrimidine (1.31g, 32%), m.p. 151-2°C. IR: $\nu_{max}$ (nujol) 3250, 3180, 1710, 1650, 1590, 1560, 1500 $cm^1$; $^1$H NMR: $\delta_H$ $[(CD_3)_2SO]$ 2.37 (2H, ABq, $J_{AB}$=6.6Hz, $\underline{CH_2}CH_2ON$), 3.92 (2H, t, J=6.6Hz, $CH_2ON$), 5.11-(2H, m, $\underline{CH_2}$=CH), 5.87 (1H, m, $CH=\underline{CH_2}$), 8.15 (1H, s, CHO), 9.26 (1H, s, CHO), 9.41 (1H, br.s, $D_2O$ exchangeable, NH), 10.85 (2H, br.s, $D_2O$ exchangeable, 2 x NH). Found: C, 41.93; H, 4.19; N, 25.01%. $C_{10}H_{12}ClN_5O_3$ requires C, 42.19; H, 3.90; N, 24.60%.

(d) 9-(4-Butenyloxy)-6-chloro-2-formamidopurine

A solution of 6-(4-butenyloxyamino)-4-chloro-2,5diformamidopyrimidine (1.2g, 4.2 mmol) in diethoxymethyl acetate (20ml) was stirred at 120°C for 4 hours. The solution was cooled, evaporated to dryness and the residue dissolved in methanol (20ml) containing 0.88 ammonia (0.5ml) and stirred for 1 hour at 25°C. The solvents were removed under reduced pressure and the residue chromatographed on silica, eluting with chloroform, yielding the title compound (600mg, 55%) as a pale yellow solid, m.p. 149-51°C. UV: $\lambda_{max}$ (MeOH) 233 ($\epsilon$ 26,400)nm; IR: $\nu_{max}$ (KBr) 3420, 1720, 1610, 1580, 1540, 1510 $cm^{-1}$; $^1$H NMR $\delta_H$ -($CDCl_3$) 2.53 (2H, m, $\underline{CH_2}CH_2ON$), 4.47 (2H, t, J=7Hz, $CH_2ON$), 5.20 (2H, m, $\underline{CH_2}$=CH), 5.84 (1H, m, $\underline{CH}=CH_2$), 8.14 (1H, s, H-8), 8.33 (1H, br. d, J=11Hz, $D_2O$ exchangeable, NH), 9.56 (1H, d, J=11Hz, CHO). Found: C, 43.94; H, 3.77; N, 25.46% $C_{10}H_{10}ClN_5O_2.0.3H_2O$ requires C, 43.98; H, 3.91; N, 25.65%.

(e) 2-Amino-9-(4-butenyloxy)-6-chloropurine

A solution of 9-(4-butenyloxy)-6-chloro-2-formamidopurine (600mg, 2.24 mmol) in methanol (5ml) was treated with 0.88 ammonia (10ml) and stirred at 80°C for 1 hour. The reaction was cooled and evaporated to dryness. The residue was absorbed on silica and chromatographed, elution with chloroform affording 2-amino-9-(4-butenyloxy)-6-chloropurine (420mg, 78%), m.p. 114-9°C. UV: $\lambda_{max}$ (MeOH) 224 ($\epsilon$ 26,300)nm; IR: $\nu_{max}$ (KBr) 3470, 3310, 1630, 1620, 1570, 1500 $cm^1$; $^1$H NMR: $\delta_H$ ($CDCl_3$) 2.52 (2H, m, $\underline{CH_2}CH_2ON$), 4.42

(2H, t, J=7Hz, $CH_2ON$), 5.22 (2H, m, $\underline{CH_2}=CH$), 5.40 (2H, br.s, $D_2O$ exchangeable, $NH_2$), 5.82 (1H, m, $\underline{CH}=CH_2$), 7.90 (1H, s, H-8). Found: C, 44.96; H, 4.24; N, 28.88%. $C_9H_{10}ClN_5O$ requires C, 45.10; H, 4.21; N, 29.22%.

(f) <u>2-Amino-6-chloro-9-(3,4-dihydroxybut-1-oxy) purine</u>

2-Amino-9-(4-butenyloxy)-6-chloropurine (395mg, 1.65 mmol) was dissolved in acetone (10ml) and water (10ml) containing a catalytic amount of osmium tetroxide. The reaction was treated with 4-methylmorpholine-N-oxide (293mg, 2.51 mmol) and stirred for 16 hours under nitrogen at 25°C. The solvents were evaporated under reduced pressure, the residue absorbed on silica and chromatographed, elution with acetone-hexane (3:1) furnishing the title compound (320mg, 70%), m.p. 159-164°C. UV: $\lambda_{max}$ - (MeOH) 224 ($\epsilon$ 27,400), 247 ($\epsilon$ 5,400), 310 ($\epsilon$ 7,700), nm; IR: $\nu_{max}$ (KBr) 3430, 3320, 3210, 1650, 1620, 1570, 1520 $cm^{-1}$; $^1H$ NMR: $\delta_H$ [$(CD_3)_2SO$] 1.74 (2H, m, $\underline{CH_2}CH_2ON$), 3.27 (2H, m,$\underline{CH_2}OH$), 3.62 (1H, m, $C\underline{H}OH$), 4.42 (2H, t, J=7Hz, $CH_2ON$), 4.60 (2H, m, $D_2O$ exchangeable, 2 x OH), 7.10 (2H, br. s, $D_2O$ exchangeable, $NH_2$), 8.41 (1H, s, H-8).

<u>Description 16</u> (Intermediates for Example 30).

(a) <u>(R)-2-Amino-6-chloro-9-(2,3-dihydroxyprop-1-oxy)purine.</u>

A solution of (R)-2-amino-6-chloro-9-(2,2-dimethyl-1,3-dioxolan-4-ylmethoxy)purine (120mg, 0.4 mmol) in 80% acetic acid was stirred at 25°C for 2 hours and then at 70°C for 1 hour. The reaction was evaporated to dryness, the residue absorbed on silica and chromatographed, eluting with acetone-hexane (3:1), affording the title compound (65mg, 63%) as a white solid, m.p. 155-8°C.IR: $\nu_{max}$ (KBr) 3340, 3220, 1660, 1630, 1570, 1520 $cm^{-1}$; $^m/z$ 259 ($M^+$, 20%).

| | |
|---|---|
| Found: | $M^+$259.0478. |
| $C_8H_{10}ClN_5O_3$ requires | $M^+$ 259.0472. |

(b) <u>(R)-2-Amino-6-chloro-9-(2,2-dimethyl-1,3-dioxolan -4-ylmethoxy)purine.</u>

A solution of (R)-6-chloro-9-(2,2-dimethyl-1,3-dioxolan dioxolan-4-ylmethoxy)-2-formamidopurine (170mg, 0.52 mmol) in methanol (5ml) and 0.88 ammonia (5ml) was stirred at 25°C for 4 hours. The solvents were evaporated under reduced pressure, the residue absorbed on silica and chromatographed, eluting with ethyl acetate-hexane (3:1), affording the title compound (120mg, 77%) as a white solid, m.p. 118-9°C. IR: $\nu_{max}$ (KBr) 3800, 3450, 3320, 1650, 1630, 1620, 1560, 1510, $cm^{-1}$; $^1H$ NMR: $\delta_H$ [$(CD_3)_2SO$)] 1.28 (3H, s, $CH_3$), 1.31 (3H, s, $CH_3$), 3.77 (1H, q, J=5.7, 8.5Hz, $CH_2OC$), 4.08 (1H, q, J=6.2, 8.5Hz, $CH_2OC$), 4.41 (3H, m, CHOC + $CH_2ON$), 7.11 (2H, br. s, $D_2O$ exchangeable, $NH_2$), 8.38 (1H, s, H-8); $^m/z$ 299 ($M^+$, 10%).

| | |
|---|---|
| Found: | $M^+$ 299.0786. |
| $C_{11}H_{14}ClN_5O_3$ requires | $M^+$ 299.0785. |

Example 1

9-(3-Hydroxyprop-1-oxy)guanine

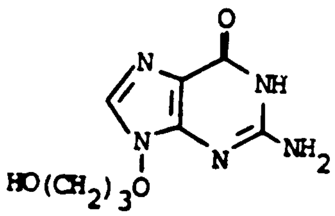

Method A

A mixture of 9-(3-benzyloxyprop-1-oxy)guanine (140mg; 0.44mmol), 10% palladium on charcoal 200mg), water (20ml), 5N hydrochloric acid (10ml) and ethanol (10ml) was stirred at 20°C under an atmosphere of hydrogen for 45 minutes. The catalyst was filtered off, the solution adjusted to pH 7 and evaporated to dryness under reduced pressure. The residue was crystallised from water to yield 42mg of a white solid, which was recrystallised once more from water to give the title compound (23mg, 23%).
$^1$H NMR: $\delta_H$ [$(CD_3)_2SO$] 1.80 (2H, quintet, J = 6.3, 6.6Hz, $CH_2\underline{CH_2}CH_2$), 3.55 (2H, quartet, J = 5.50, 5.77Hz, $\underline{CH_2}OH$), 4.32 (2H, t, J = 6.6Hz, $CH_2ON$), 4.57 (1H, t, J = 5.0, 5.5Hz, $D_2O$ exchangeable, OH), 6.57 (2H, br.s, $D_2O$ exchangeable, $NH_2$), 7.91 (1H, s, H-8), 10.63 (1H, br.s, $D_2O$ exchangeable, H-1). Found: C, 41.33; H, 5.20; N, 30.24%. $C_8H_{11}N_5O_3$.0.4 $H_2O$ requires C, 41.33; H, 5.13; N, 30.14%.

Method B

9-(3-Benzyloxyprop-1-oxy)-6-chloro-2-formamidopurine (3.40g, 9.41mmol) in 80% formic acid (100ml) was heated at 100°C for 1 hour. The reaction mixture was then cooled and stirred with 10% palladium on charcoal (2.0g) under an atmosphere of hydrogen at 20°C for 45 minutes. After removal of the catalyst, the solution was evaporated and the residue was treated with water (50ml) and concentrated aqueous ammonia (4ml) at 100°C for 15 minutes. The solution was then cooled and evaporated under reduced pressure. Recrystallisation of the residue from water afforded 9-(3-hydroxyprop-1-oxy)guanine (800mg, 33%). $^1$H NMR: $\delta$H [$(CD_3)_2SO$] 1.80 (2H, quintet, J = 6.3, 6.6Hz, $CH_2\underline{CH_2}CH_2$), 3.55 (2H, quartet, J = 5.50, 5.8 Hz, $\underline{CH_2}OH$), 4.32 (2H, t, J = 6.6Hz, $CH_2ON$), 4.57 (1H, t, J = 5.5Hz, $D_2O$ exchangeable, OH), 6.57 (2H, br.s, $D_2O$ exchangeable, $NH_2$), 7.91 (1H, s, H-8), 10.63 (1H, br.s, $D_2O$ exchangeable, H-1).

Example 2

9-(3-Acetoxyprop-1-oxy)guanine

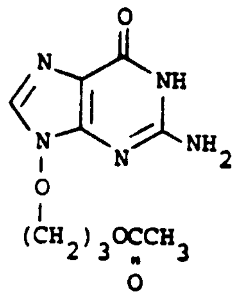

A mixture or 9-(3-hydroxyprop-1-oxy)guanine (150mg, 0.67mmol), 4-dimethylaminopyridine (15.6mg, 0.13mmol), acetic anhydride (0.25ml, 2.65mmol) and N,N-dimethylformamide (5ml) was stirred at 20 °C for 3 hours and then ethanol was added. After a further 15 minutes the solvent was evaporated under reduced pressure and the residue was chromatographed on silica gel (eluted with chloroform-ethanol, 4:1), yielding the title compound (120mg, 67%). Recrystallisation from methanol-water gave 9-(3-acetoxy-prop-1-oxy)-guanine (82mg, 46%). IR: $\nu_{max}$ (KBr) 3330, 3168, 1736, 1696, 1648, 1602, 1589, 1391cm$^{-1}$. $^1$H NMR: $\delta$H [-$(CD_3)_2SO$] 1.98 (2H, quintet, J = 6.3, 6.6Hz, $CH_2\underline{CH_2}CH_2$), 2.02 (3H, s,

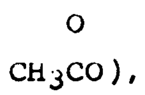

4.17 (2H, t, J = 6.6Hz, $\underline{CH_2}ON$), 4.32 (2H, t, J = 6.3Hz,

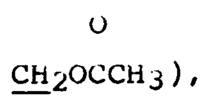

6.60 (2H, br.s, $D_2O$ exchangeable $NH_2$), 7.94 (1H, s, H-8), 10.69 (1H, br.s, $D_2O$ exchangeable, H-1). Found: C, 44.99; H, 4.93; N, 26.20% M$^+$ 267.0964 $C_{10}H_{13}N_5O_4$ requires C, 44.93; H, 4.91; N, 26.21% M$^+$ 267.0968.

Example 3

9-(3-Benzoyloxyprop-1-oxy)guanine

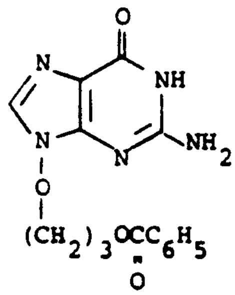

A mixture of 9-(3-hydroxyprop-1-oxy)guanine (150mg, 0.67mmol), 4-dimethylaminopyridine (15.6mg, 0.13mmol) benzoic anhydride (150mg, 2.65mmol) and N,N-dimethylformamide (5ml) was stirred at 20°C for 24 hours. The mixture was then evaporated to dryness and the residue was chromatographed on silica gel (eluted with chloroform-ethanol, 10:1). Recrystallisation from water-methanol afforded 9-(3-benzoyloxyprop-1-oxy)guanine (75mg, 36%). IR: $\nu_{max}$ (KBr) 3393, 3200, 1714, 1700, 1639, 1595, 1582, 1391cm$^{-1}$. $^1$H NMR: $\delta$H [$(CD_3)_2SO$] 2.13 (2H, quintet, J = 6.3Hz, $CH_2\underline{CH_2}CH_2$), 4.43 (2H, t, J = 6.6Hz, $CH_2$), 4.46 (2H, t, J = 6.3Hz, $CH_2$), 6.60 (2H, br.s, $D_2O$ exchangeable, $\overline{NH_2}$), 7.53 (2H, m, 2 protons of $PhCO_2$), 7.67 (1H, m, 1 proton of $PhCO_2$), 7.97 (1H, s, H-8), 7.97 (2H, m, 2 protons of $PhCO_2$), 10.72 (1H, br.s, $D_2O$ exchangeable, H-1). Found; C, 54.52; H, 4.72; N, 20.98%, M$^+$ 329.1126 $C_{15}$ $H_{15}N_5O4$ requires C, 54.70; H, 4.60; N, 21.27% M$^+$ 329.1124.

Example 4

2-Amino-6-ethoxy-9-(3-hydroxyprop-1-oxy)purine

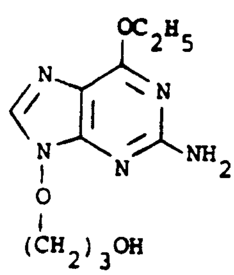

A mixture of 2-amino-9-(3-benzyloxyprop-1-oxy)-6-ethoxypurine (350mg, 1.02mmol), 10% palladium on charcoal (350mg) and 80% formic acid (10ml) was stirred at 20°C under an atmosphere of hydrogen for 45 minutes. The catalyst was removed and the solution was evaporated under reduced pressure. The residue was dissolved in water (10ml) and heated to boiling. Concentrated aqueous ammonia (1ml) was then added and the solution heated for 15 minutes and then cooled and evaporated under reduced pressure. Recrystallisation of the residue from water afforded the title compound (145mg, 56%). IR: $\nu_{max}$ (KBr) 3376, 3326, 3205, 1649, 1611, 1581, 1509, 1454, 1402cm$^{-1}$. $^1$H NMR: $\delta$H [$(CD_3)_2SO$] 1.35, (3H, dd, J = 6.9, 7.1Hz, $\underline{CH_3}CH_2O$), 2.50 (2H, quintet, J = 6.3, 6.6Hz, $CH_2$), 3.56 (2H, dt. J = 5.2, 6.3Hz, $\underline{CH_2}OH$), 4.35 (2H, t, J = $\overline{6}$.6Hz. $CH_2ON$), 4.45 (2H, quartet, J = 6.9, 7.1Hz, $O\underline{CH_2}CH_3$), 4.61 (1H, t, $\overline{J}$ = 5.2Hz, $D_2O$ exchangeable, OH), 6.56 (2H, br.s, $D_2O$ exchangeable, $NH_2$), 8.09 (1H, s, H-8). Found: C, 47.16; H, 5.92; N, 27.60%. $C_{10}H_{15}N_5O_3$ requires: C, 47.41; H, 5.98; N, 27.65%.

Example 5

9-(3-Hydroxy-2-hydroxymethylprop-1-oxy)guanine

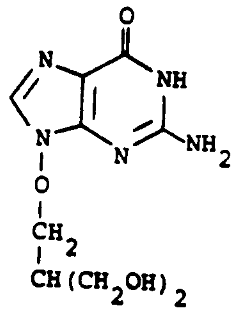

A mixture of 9-(3-benzyloxy-2-benzyloxymethylprop-1-oxy)-2-formamido-6-chloropurine (2.5g, 5.2mmol) and 80% formic acid (100ml) was stirred at 100 °C for 1 hour, cooled and 10% palladium on charcoal (2.0g) added. The mixture was then stirred under an atmosphere of hydrogen for 45 minutes. The catalyst was removed and the solution evaporated under reduced pressure. The residue obtained was heated in water (100ml) until boiling commenced. Concentrated aqueous ammonia (4ml) was added and the solution was then heated for a further 15 minutes, cooled and evaporated under reduced pressure. Recrystallisation from water (charcoal) afforded 9-(3-hydroxy-2-hydroxymethylprop-1-oxy)guanine (430mg, 32%). IR: $\nu_{max}$ (KBr) 3380, 3183, 1679, 1637, 1605, 1541, 1479, 1395cm$^{-1}$. $^1$H NMR: $\delta$H [$(CD_3)_2SO$] 1.94 (1H, m, CH), 3.53 (4H, m, 2 x $\underline{CH_2}OH$), 4.26 (2H, d, J = 6.3Hz), 4.57 (2H, t, J = 5.2Hz, $D_2O$ exchangeable, 2 x OH), 6.58 (2H, br.s, $NH_2$), 7.92 (1H, s, H-8), 10.64 (1H, br.s, $D_2O$ exchangeable, H-1), Found: C, 42.08 H, 5.15; N, 27.41%. $C_9H_{13}N_5O_4$ requires: C, 42.34; H, 5.14; N, 27.44%.

Example 6

9-(2,3-Dihydroxyprop-1-oxy)guanine

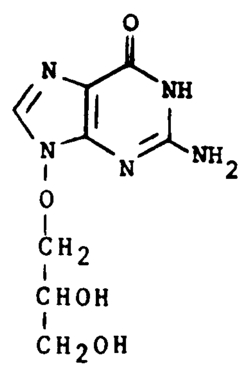

2-Amino-6-chloro-9-(2,3-dihydroxyprop-1-oxy)purine (100mg, 0.39mmol) was dissolved in 80% formic acid solution and stirred at 100 °C for 2.5 hours. The reaction was evaporated to dryness under reduced pressure and the residue treated with methanol (5ml) and 0.88 ammonia solution (3ml). After stirring the reaction at 60 °C for 2 hours, the solvents were evaporated under reduced pressure and the residue recrystallised from water yielding the title compound (35mg, 38%), m.p. 252-3 °C. UV: $\lambda_{max}$ ($H_2O$) 252 ($\epsilon$ 12,600) nm; IR: $\nu_{max}$ (KBr) 3330, 1690, 1640, 1605, 1540, 1475cm$^{-1}$; $^1$H NMR: $\delta$H [$(CD_3)_2SO$] 3.39 (2H, m, $\underline{CH_2}OH$), 3.72 (1H, m, $\underline{CH}OH$), 4.10 (1H, dd, J = 10.7, 7.6Hz, 7.6Hz, $CH_2ON$), 4.33 (1H, dd, J = 10.5, 3.3Hz, $CH_2ON$), 4.70 (1H, t, J = 5.7Hz, OH), 5.15 (1H, d, J = 5.2Hz, OH), 6.62 (2H, br.s, $NH_2$), 7.90 (1H, s, H-8), 10.58 (1H, br.s, NH); m/z 241 (M+, 3%), 210 (2), 167 (27), 151 (100), 109 (20), 61 (47). Found: C,

38.85; H, 4.66; N, 29.14%, $M^+$ 241.0813. $C_8H_{11}N_5O_4$ requires C, 39.84; H, 4.60; N, 29.03%, $M^+$ 241.0811.

Example 7

2-Amino-9-(2,3-dihydroxyprop-1-oxy)purine

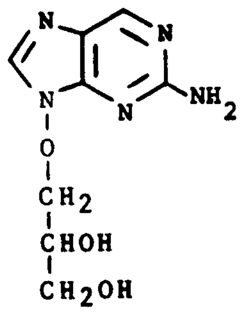

A mixture of 2-amino-6-chloro-9-(2,3-dihydroxyprop-1-oxy)purine (150mg, 0.58mmol), ammonium formate (146mg, 2.32mmol) and 10% palladium-on-charcoal (15mg) in methanol (5ml) was stirred under reflux for 4 hours. The reaction was evaporated under reduced pressure and the residue was dissolved in water and passed through a SEP-PAK $C_{18}$ cartridge for decolourisation. After elution with water, the water was evaporated under reduced pressure and the residue recrystallised from ethanol affording the title compound (58mg, 45%), m.p. 183-5 °C. UV: $\lambda_{max}$ ($H_2O$) 221 ($\epsilon$ 25,600), 305 ($\epsilon$ 7,000) nm; IR: $\nu_{max}$ (KBr) 3670, 3420, 3310, 3200, 1650, 1620, 1570, 1520, 1480, 1425cm$^{-1}$; $^1H$ NMR: $\delta H$ [$(CD_3)_2SO$] 3.41 (2H, m, $\underline{CH_2}OH$), 3.76 (1H, m, $\underline{CH}OH$), 4.18 (1H, dd, J = 7.6, 10.7Hz, $CH_2ON$), 4.39 (1H, dd, J = 3.2, 10.7Hz, $CH_2ON$), 4.72 (1H, t, J = 5.6Hz, OH), 5.15 (1H, d, J = 5.2Hz, OH), 6.72 (2H, br.s, $NH_2$), 8.27 (1H, s, H-8), 8.59 (1H, s, H-6). Found: C, 42.17; H, 4.91; N, 31.07%, $M^+$ 225.0865. $C_8H_{11}N_5O_3$ requires C, 42.67; H, 4.92; N, 31.10%, $M^+$ 225.0862.

Example 8

9-(1,4-Dihydroxybut-2-oxy)guanine

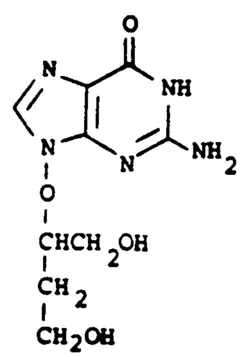

To a solution of 9-[1,4-bis(4-methoxybenzloxy)but-2-oxy]-6-chloro-2-formamidopurine (0.27g, 0.5mmol) in dichloromethane (2.7ml) and water (0.15ml) was added 2,3-dichloro-5,6-dicyanobenzoquinone (0.25g, 1.1mmol) and the solution was stirred at room temperature for 1 hour. The solution was diluted with dichloromethane (3ml) and extracted with water (2 x 5ml). The aqueous layers were combined, filtered and the solvent removed. The residue was purified by column chromatography on silica gel eluting with chloroform-methanol (10:1). The product was dissolved in 50% formic acid and the solution was heated at 100 °C for 1 hour. The solvent was removed and the residue coevaporated with water. The residue was

dissolved in concentrated aqueous ammonia and the solution stirred at 80 °C for 20 minutes. The solvent was removed and the residue was purified by reverse-phase column chromatography on Spherisorb C18 300 silica eluting with water followed by 5% and 10% methanol to afford 9-(1,4-dihydroxybut-2-oxy)guanine (28mg, 23%), m.p. decomposition >215 °C; UV: $\lambda_{max}$ ($H_2O$) 252 ($\epsilon$ 12,400) and 265 (inflexion, $\epsilon$ 9,740)nm; IR: $\nu_{max}$ (KBr) 3360, 3200, 1735, 1690, 1640, 1600, 1540, 1475 and 1400cm$^{-1}$; $^1H$ NMR: $\delta$H [$(CD_3)_2SO$] 1.82 (2H, dq, $J_q$ = 6.5Hz and $J_d$ = 1.8Hz, 3′-H), 3.56 (4H, m, 1′-H and 4′-H), 4.34 (1H, m, 2′-H), 4.63 (1H, t, J = 5.4Hz, $D_2O$ exchangeable, OH), 4.97 (1H, t, J = 6.1Hz, $D_2O$ exchangeable, OH), 6.59 (2H, s, $D_2O$ exchangeable, 2-$NH_2$), 7.87 (1H, s, 8-H) and 10.67 (1H, s, $D_2O$ exchangeable, 1-H). Found: C, 40.33; H, 5.32; N, 26.03%.$C_9H_{13}N_5O_4$.0.7$H_2O$ requires C, 40.36; H, 5.42; N, 26.15%.

Example 9

2-Amino-9-(1,4-dihydroxybut-2-oxy)purine

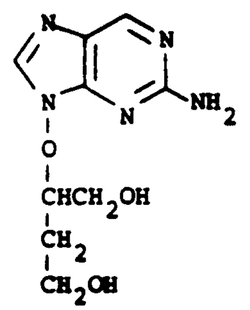

To a solution of 2-amino-9-[1,4-bis(4-methoxybenzyloxy)but-2-oxy]-6-chloropurine (0.94g, 1.8mmol) in dichloromethane (7.2ml) and methanol (0.8ml) was added 2,3-dichloro-5,6-dicyanobenzoquinone (0.91g, 4.0mmol) and the solution was stirred at room temperature for 80 minutes. The solution was diluted with dichloromethane (8ml) and extracted with water ( 3 x 8ml). The aqueous layers were combined, filtered and the solvent removed. The residue was purified by reverse-phase column chromatography on Spherisorb $C_{18}$ 300 silica eluting with 10% methanol in water followed by column chromatography on silica gel eluting with chloroform-methanol (12:1). The product was suspended in a solution of ammonium formate (208mg, 3.3mmol) in methanol (8ml), 10% palladium-on-charcoal was added (25mg) and the mixture was heated under reflux. After 30 minutes further 10% palladium-on-charcoal (10mg) was added and the mixture was heated under reflux for a further 1 hour. The mixture was allowed to cool and water (2ml) was added. The solution was filtered and the solvent was removed. The residue was purified by reverse-phase column chromatography on Spherisorb $C_{18}$ 300 silica eluting with water and 10% methanol to afford 2-amino-9-(1,4-dihydroxybut-2-oxy)purine (155mg, 36%), m.p. 178-179 °C; UV: $\lambda_{max}$ ($H_2O$) 222 ($\epsilon$ 24,100) and 304 ($\epsilon$ 7,020)nm; IR: $\nu_{max}$ (KBr) 3330, 3210, 3070, 1655, 1640, 1580, 1510, 1480 and 1435cm$^{-1}$: $^1H$ NMR: $\delta$H [-$(CD_3)_2SO$] 1.84 (2H, q, J = 6.4Hz, 3′-H), 3.58 (4H, m, 1′-H and 4′-H), 4.42 (1H, m, 2′-H), 4.63 (1H, t, J = 5.2Hz, $D_2O$ exchangeable, OH), 4.98 (1H, t, J = 5.9Hz, $D_2O$ exchangeable, OH), 6.70 (2H, s, $D_2O$ exchangeable, 2-$NH_2$), 8.23 (1H, s, 8-H) and 8.59 (1H, s, 6-H).

| | | | |
|---|---|---|---|
| Found: | C, 45.13; | H, 5.47; | N, 29.30%. |
| $C_9H_{13}N_5O_3$ requires | C, 45.18; | H, 5.48; | N, 29.27%. |

Example 10

2-Amino-9-(3-hydroxyprop-1-oxy)purine

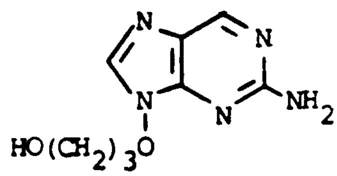

9-(3-t-Butyldimethylsilyloxyprop-1-oxy)-2-formamidopurine (800 mg, 2.28 mmol) in 80% acetic acid (20 ml) was heated at 90 °C for 20 minutes. The mixture was then cooled, evaporated under reduced pressure and the residue co-evaporated with water. The residue was dissolved in ethanol (20 ml) and hydrazine hydrate (1 ml). The solution was heated under reflux for 1 hour, cooled and evaporated to dryness. Column chromatography on silica gel (eluted with chloroform-ethanol, 8:1) gave the title compound (262 mg, 55%).
IR:$\nu_{max}$ (KBr) 3340, 3210, 1655, 1615, 1570, 1510, 1430 $cm^{-1}$.
$^1H$ NMR:$\delta_H$[$(CD_3)_2SO$] 1.84(2H, quintet, J = 6.5Hz, $CH_2\underline{CH_2}CH_2$), 3.58(2H,q, J = 6.5, 5.2Hz, $\underline{CH_2}OH$), 4.39-(2H,t, J = 6.5Hz,$CH_2ON$), 4.62(1H, t, J = 5.2Hz, OH), 6.71(2H, br.s, $D_2O$ exchangeable, $NH_2$) 8.31(1H,s,H-8), 8.59(1H,s,H-6).

| | | | |
|---|---|---|---|
| Found: | C,45.89; | H,5.38; | N,33.85%. |
| $C_8H_{11}N_5O_2$ requires: | C,45.92; | H,5.31; | N,33.49%. |

Example 11

9-(3-Hexanoyloxyprop-1-oxy)guanine

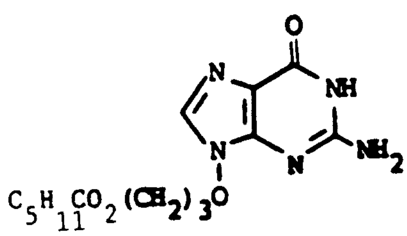

A mixture of 9-(3-hydroxyprop-1-oxy)guanine (150mg, 0.67mmol), 4-dimethylaminopyridine (15.6mg, 0.13mol), hexanoic anhydride (0.31ml, 1.34mmol) and N,N-dimethylformamide was stirred at 20 °C for 2 hours. The mixture was then evaporated to dryness and the residue was chromatographed on silica gel (eluted with chloroform-ethanol, 4:1). Recrystallisation from water-methanol afforded 9-(3-hexanoyloxyprop-1-oxy)guanine (80mg; 39%). IR: $\nu_{max}$ (KBr) 3337, 3172, 2957, 2933, 1696, 1646, 1599, 1587, 1390$cm^{-1}$; $^1H$ NMR: $\delta$H [$(CD_3)_2SO$] 0.84 (3H, t, J = 6.9Hz, $CH_3$), 1.24 (4H, m, $\underline{CH_2}\underline{CH_2}CH_3$), 1.51 (2H, quintet,

$$\overset{O}{C}CH_2\underline{CH_2}CH_2CH_2CH_3),$$

1.98 (2H, quintet, J = 6.6, 6.3Hz, $OCH_2\underline{CH_2}CH_2O$) 2.29 (2H, t, J = 7.4Hz,

$$CH_2\overset{O}{C}O),$$

4.19 (2H, d-d, J = 6.6, 6.3Hz, $CH_2ON$), 4.32 (2H, d-d, J = 6.6, 6.3Hz,

$$CH_2O\overset{O}{C}),$$

6.58 (2H, br.s, $D_2O$ exchangeable, $NH_2$) 7.93 (1H, s, H-8), 10.66 (1H, br.s, $D_2O$ exchangeable, H-1).

| | | | |
|---|---|---|---|
| Found: | C, 51.74; | H, 6.61; | N, 21.49%. |
| $C_{14}H_{21}N_5O_4$ requires | C, 51.99; | H, 6.56; | N, 21.66%. |

Example 12

2-Amino-9-(1,4-diacetoxybut-2-oxy)purine

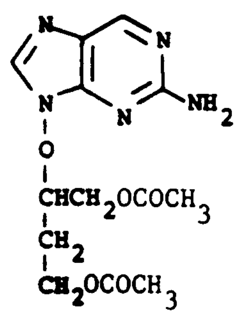

A solution of 2-amino-9-(1,4-dihydroxybut-2-oxy)purine (100mg, 0.42mmol), 4-dimethylaminopyridine (4mg) and acetic anhydride (0.14ml, 1.5mmol) in N,N-dimethylformamide (3ml) was stirred at room temperature for 15 minutes and methanol (0.5ml) was then added. The solvent was removed and the residue was partitioned between chloroform (5ml) and aqueous sodium bicarbonate (3ml). The aqueous layer was extracted with chloroform again (5ml) and the combined organic extracts were dried (magnesium sulphate) and the solvent removed. The residue was purified by column chromatography on silica gel eluting with chloroform-methanol (30:1). Trituration with hexane-ethyl acetate afforded 2-amino-9-(1,4-diacetoxybut-2-oxy)purine as white crystals (112mg, 83%), m.p. 123-125 °C; IR: $\nu_{max}$ (KBr) 3400, 3310, 3190, 1735, 1640, 1615, 1585, 1505, 1470 and 1430cm$^{-1}$; $^1$H NMR: $\delta$H [$(CD_3)_2SO$] 2.0-2.5 (8H, m, 3′-H and 2 x $CH_3$), 4.21 (1H, dd, J = 12.7Hz and 5.8Hz, 1′-H), 4.32 (1H, dt, $J_d$ = 10.9Hz and $J_t$ = 5.4Hz, 4′-H), 4.54 (1H, dd, J = 12.9Hz and 2.8Hz, 1′-H), 4.63 (1H, m, 2′-H), 4.81 (1H, ddd, J = 11.3Hz, 9.4Hz and 4.4Hz, 4′-H, 5.16 (2h, s, $D_2O$ exchangeable, 2-$NH_2$), 7.96 (1H, s, 8-H and 8.67 (1H, s, 6-H).

| | | | |
|---|---|---|---|
| Found: | C, 48.15; | H, 5.23; | N, 21.51%. |
| $C_{13}H_{17}N_5O_5$ requires | C, 48.29; | H, 5.30; | N, 21.66%. |

Example 13

2-Amino-9-(1,4-dibutyryloxybut-2-oxy)purine

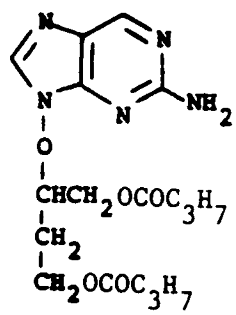

A solution of 2-amino-9-(1,4-dihydroxybut-2-oxy)purine (75mg, 0.31mmol), 4-dimethylaminopyridine (3mg) and butyric anhydride (0.17ml, 1.0mmol) in N,N-dimethylformamide (3ml) was stirred at room temperature for 20 minutes and methanol (0.5ml) was then added. The solvent was removed and the residue was partitioned between chloroform (5ml) and aqueous sodium bicarbonate (3ml). The organic layer was dried (magnesium sulphate) and the solvent was removed. The residue was purified by column chromatography on silica gel eluting with chloroform-methanol (40:1). Crystallisation from ether-hexane afforded 2-amino-9-(1,4-dibutyryloxybut-2-oxy)purine as a white solid (79mg, 67%), m.p. 99-101 °C; IR: $\nu_{max}$ (KBr) 3400, 3310, 3190, 2970, 1735, 1640, 1615, 1580, 1505, 1470 and 1430cm$^{-1}$; $^1$H NMR: $\delta$H [$(CD_3)_2SO$] 0.94 (6H, t, J = 7.4Hz, 2 x $CH_3$), 1.64 (4H, m, 2 x $\underline{CH_2}CH_3$), 2.09 (1H, m, 3′-H), 2.17 (1H, m, 3′-H), 2.30 (4H, q, J = 7.1Hz, 2 x $CH_2CO$), 4.21 (1H, dd, J = 12.6Hz and 5.5Hz, 1′-H), 4.33 (1H, dt, $J_d$ = 11.0Hz and $J_t$ = 5.4Hz, 4′-H), 4.55 (1H, dd, J = 12.8Hz and 2.9Hz, 1′-H), 4.62 (1H, m, 2′-H), 4.79 (1H, ddd, J = 11.3Hz, 9.1Hz and 4.7Hz, 4′-H), 5.14 (2H, s, $D_2O$ exchangeable, 2-$NH_2$), 7.95 (1H, s, 8-H) and 8.66 (1H, s, 6-H).

| | | | |
|---|---|---|---|
| Found: | C, 53.45; | H, 6.66; | N, 18.34%. |
| $C_{17}H_{25}N_5O_5$ requires | C, 53.82; | H, 6.64; | N, 18.46%. |

Example 14

(R)-9-(1,4-Dihydroxybut-2-oxy)guanine

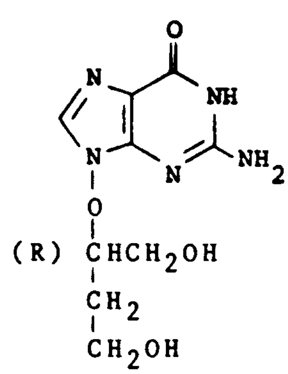

A solution of (R)-9-(1,4-dibenzyloxybut-2-oxy)guanine (80mg, 0.18mmol) in tetrahydrofuran (4ml) and water (1ml) was treated with 5M hydrochloric acid (3 drops) and 10% palladium-on-charcoal (40mg). The mixture was hydrogenated under atmospheric pressure for 2 hours, then filtered and fresh catalyst (100mg)

added. Hydrogenation was continued for 16 hours and the mixture filtered, washing the catalyst with boiling water (3 x 5ml). The solution was neutralised with IR 45 (OH) ion exchange resin, filtered and evaporated to dryness. The residue was chromatographed on silica, eluting with chloroform-methanol 5:2 to give (R)-9-(1,4-dihydroxybut-2-oxy)guanine (22mg, 48%); UV: $\lambda_{max}$ (EtOH) 252, 270nm (s); $^1$H NMR: $\delta$H [$(CD_3)_2SO$] 1.80 (2H, m, $CH_2$), 3.55 (4H, m, 2 x $CH_2$), 4.35 (1H, m, CH), 4.64 (1H, t, J = 5Hz, $D_2O$ exchangeable OH), 5.00 (1H, t, J = 6Hz, $D_2O$ exchangeable OH), 6.67 (2H, s, $D_2O$ exchangeable $NH_2$), 7.87 (1H, s, CH), 10.76 (1H, br.s., $D_2O$ exchangeable NH); m/z 255 ($M^+$, 2%), 221 (10), 178 (15), 167 (20), 165 (10), 152 (10), 151 (35), 150 (5), 135 (5), 108 (10), 91 (10), 75 (55), 58 (60), 57 (100), 45 (50), 43 (60). $M^+$ observed 255.0968. $C_9H_{13}N_5O_4$ requires $M^+$ 255.0967.

Example 15

(S)-9-(1,4-Dihydroxybut-2-oxy)guanine

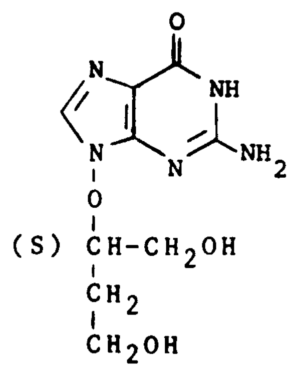

A solution of (S)-6-chloro-9-(1,4-dibenzyloxybut-2-oxy)-2-formamidopurine (0.4g, 0.8mmol) in 80% formic acid (20ml) was heated at 100 °C for 1 hour and cooled to room temperature. To the solution was added 10% palladium-on-charcoal catalyst (200mg) and the mixture was hydrogenated under atmospheric pressure for 1 hour, then filtered. The filtrate was evaporated to dryness under reduced pressure and the residue dissolved in methanol (2ml) and 0.88 ammonia (2ml) and stirred at room temperature for 30 minutes. The solvent was removed under vacuum and the residue chromatographed on reverse phase silica, eluting with water, 5% methanol-water and 10% methanol-water. The product eluted in the 5% and 10% methanol fractions and crystallised from these fractions giving (S)-9-(1,4-dihydroxybut-2-oxy)guanine (0.12g, 56%) m.p. 248 °C (dec),

$$[\alpha]^{25}_{D} \ -32.3^{\circ}$$

(c 0.16 in water); UV: $\lambda_{max}$ 252 ($\epsilon$ 13040), 270 (s); IR: $\nu_{max}$ (KBr) 3364, 3298, 3243, 3199, 3133, 2959, 1710, 1690, 1644, 1610, 1600, 1578, 1529, 1474, 1410, 1385, 1368, 1323, 1257, 1226, 1206, 1167, 1124, 1068, 1055, 1025, 1015, 968, 948, 867, 849, 781, 696$cm^{-1}$; $^1$H NMR: $\delta$H [$(CD_3)_2SO$] 1.80 (2H, m, $CH_2$), 3.55 (4H, m, 2 x $CH_2$), 4.30 (1H, m, CH), 4.63 (1H, t, J = 5Hz, $D_2O$ exchangeable, OH), 4.98 (1H, t, J =6Hz, $D_2O$ exchangeable, OH), 6.60 (2H, s, $D_2O$ exchangeable $NH_2$), 7.86 (1H, s, CH), 10.70 (1H, s, $D_2O$ exchangeable NH).

| | | | |
|---|---|---|---|
| Found: | C, 40.90, | H, 5.16; | N, 26.72%. |
| $C_9H_{13}N_5O_4$.0.5 $H_2O$ requires | C, 40.90; | H, 5.34; | N, 26.50%). |

Example 16

2-Amino-9-(3-hydroxy-2-hydroxymethylprop-1-oxy)purine.

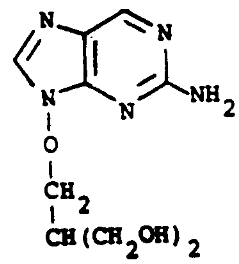

2-Amino-9-(2,2-dimethyl-1,3-dioxan-5-ylmethoxy)purine (500mg, 1.79 mmol) in 80% acetic acid was stirred at 20 °C for 3 hours. The mixture was then evaporated to a syrup and co-evaporated with toluene. Column chromatography on silica gel (eluted with chloroform-ethanol, 5:1 and then 5:2) afforded the title compound (371mg, 87%). IR: $\nu_{max}$ (KBr) 3336, 3203, 1647, 1618, 1578, 1430cm$^{-1}$. $^1$H NMR: $\delta_H$[$(CD_3)_2SO$] 1.97 (1H,m,CH), 3.55(4H,m, 2 x $\underline{CH_2}OH$), 4.33(2H,d, J=6.3Hz, $CH_2ON$), 4.59(2H, t, J=5.3Hz, $D_2O$ exchangeable, 2 x OH), 6.70(2H, br.s, $D_2O$ exchangeable, $NH_2$), 8.30(1H,s,H-8), 8.59(1H,s,H-6).

| | | | | |
|---|---|---|---|---|
| Found: | C,45.05; | H,5.63; | N,29.44%; | M$^+$ 239.1022. |
| $C_9H_{13}N_5O_3$ requires: | C, 45.17; | H, 5.49: | N, 29.28%, | M$^+$ 239.1018. |

Example 17

9-(3-Acetoxy-2-acetoxymethylprop-1-oxy)-2-amino-purine.

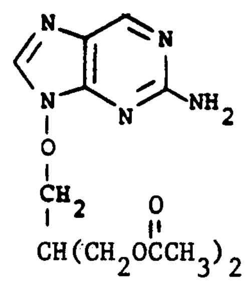

A mixture of 2-Amino-9-(3-hydroxy-2-hydroxymethylprop--1-oxy)purine (90mg, 0.376 mmol), acetic anhydride (0.1ml, 1.06 mmol), 4-dimethylaminopyridine (10mg, 0.0836 mmol) and N,N-dimethylformamide (2ml) was stirred at 20 °C for 1.5 hours. Ethanol (0.2ml) was added, the mixture stirred for a further 15 minutes and then evaporated under reduced pressure. Column chromatography on silica gel (eluted with chloroform-ethanol 19:1) yielded a syrup that solidified on trituration with ether. (110.3mg, 91%). IR: $\nu_{max}$ - (KBr) 3328, 3191, 1740, 1652, 1618, 1581, 1513, 1431 cm$^{-1}$. $^1$H NMR: $\delta_H$[$(CD_3)_2SO$] 2.03 (6H,s, 2 x $CH_3$), 2.50(m,CH,$(CH_3)_2SO$), 4.20(4H,m, 2 x $\underline{CH_2}OC=O$), 4.39(2H,d, J=6.3Hz, $CH_2ON$), 6.68(2H,br.s, $D_2O$ exchangeable, $NH_2$), 8.32(1H,s,H-8), 8.60(1H,s,H-6).

| | | | | |
|---|---|---|---|---|
| Found: | C,48.34; | H,5.30; | N,21.57%; | M$^+$ 323.1225. |
| $C_{13}H_{17}N_5O_5$ requires: | C,48.29, | H,5.31; | N,21.66%; | M$^+$ 323.1230. |

Example 18

2-Amino-9-(3-propionyloxy-2-propionyloxymethylprop1-oxy)purine.

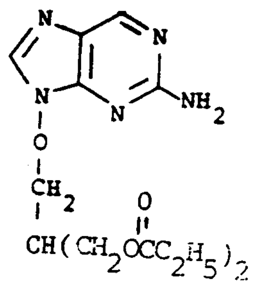

A mixture of 2-Amino-9-(3-hydroxy-2-hydroxymethylprop -1-oxy)purine (90mg, 0.376 mmol), propionic anhydride (0.12ml, 0.920 mmol), 4-dimethylaminopyridine (9mg, 0.0752 mmol) and N,N-dimethylformamide (2ml) was stirred at 20°C for 1.5 hours. Ethanol (0.2ml) was added, the mixture stirred for a further 15 minutes and then evaporated under reduced pressure. Column chromatography on silica gel (eluted with chloroform-ethanol, 19:1) gave a syrup that solidified on trituration with ether (122mg, 83%). IR: $\nu_{max}$ (KBr) 3382, 3313, 1740, 1641, 1619, 1575, 1429 $cm^{-1}$. H NMR: $\delta_H$ [$(CD_3)_sSO$] 1.02(6H, t, J=7.4Hz, 2 x $CH_3$), 2.33(4H, quartet, J=7.4 Hz, 2 x $CH_2$ C=O), 4.22(4H,m,2 x $CH_2OC$=O) 4.39(2H,d, J=6.3 Hz, $CH_2ON$), 6.68(2H, br.s, $D_2O$ exchangeable, $NH_2$), 8.32(1H,s,H-8), 8.60(1H,s,H-6).

| | | | | |
|---|---|---|---|---|
| Found: | C,51.20; | H,6.04; | N,19.94%; | $M^+$ 351.1525. |
| $C_{15}H_{21}N_5O_5$ requires: | C,51.26; | H,6.04; | N,19.93%; | $M^+$ 351.1543. |

Example 19

2-Amino-9-(3-benzoyloxy-2-benzoyloxymethylprop-1-oxy)purine

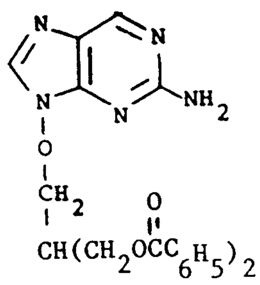

A mixture of 2-amino-9-(3-hydroxy-2-hydroxymethylprop-1-oxy)purine (80mg, 0.335 mmol), benzoic anhydride (189mg, 0.835 mmol), 4-N,N-dimethylaminopyridine (8mg, 0.668 mmol) and N,N-dimethylformamide (2ml) was stirred at 20°C for 2 hours. Ethanol (0.2ml) was added, the mixture stirred for a further 15 minutes and then evaporated under reduced pressure. Column chromatography on silica gel (eluted with chloroform-ethanol, 19:1) gave a syrup that was recrystallised from ether, affording the title compound (92.2mg, 65%). IR: $\nu_{max}$ (KBr) 3327, 1721, 1617, 1576, 1426 $cm^{-1}$. H NMR: $\delta_H$ [$(CD_3)_2SO$] 2.82(1H,m,CH,), 4.60(6H,m 3 x $CH_2$), 6.64(2H,br.s, $D_2O$ exchangeable, $NH_2$), 7.51(4H, t, aromatic), 7.65(2H, t, aromatic), 7.97(4H,d, aromatic), 8.39(1H,s,H-8),8.60 (1H,s,H-6).

| | | | |
|---|---|---|---|
| Found: | C,61.56; | H,4.79; | N,15.56%. |
| $C_{23}H_{21}N_5O_5$ requires: | C,61.73; | H,4.74; | N,15.65%. |

Example 20

2-Amino-9-(3-hydroxy-2-hydroxymethylprop-1-oxy)-6-methoxypurine

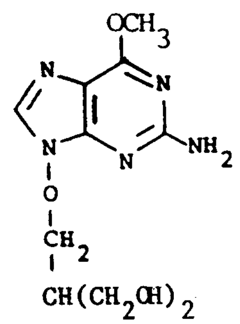

2-Amino-9-(2,2-dimethyl-1,3-dioxan-5-ylmethoxy)-6-methoxypurine (290mg, 0.938 mmol) in 80% acetic acid (10ml) was stirred at 20 °C for 3 hours and then evaporated under reduced pressure. The residue was co-evaporated with toluene and then chromatographed on silica gel (eluted with chloroform-ethanol, 10:1) affording the title compound (224mg, 89%). IR: $\nu_{max}$ (KBr) 3332, 3213, 1617, 1584, 1509, 1491, $cm^{-1}$. $^1H$ NMR: $\delta_H$ [$(CD_3)_2SO$] 1.95(1H,m,CH), 3.53(4H,m, 2 x $CH_2OH$),3.96(3H,s,$OCH_3$), 4.29(2H,d,J=6.3Hz, $CH_2ON$), 4.59(2H, t, J=5.2Hz, $D_2O$ exchangeable, 2 x OH), 6.60(2H,br.s, $D_2O$ exchangeable, $NH_2$), 8.09-(1H,s,H-8).

| | | | | |
|---|---|---|---|---|
| Found: | C,44.51; | H,5.68; | N,26.14%; | $M^+$ 269.1127. |
| $C_{10}H_{15}N_5O_4$. requires: | C,44.60; | H,5.63; | N,26.01%; | $M^+$ 269.1124. |

Example 21

2,6-Diamino-9-(3-hydroxy-2-hydroxymethylprop-1-oxy) -purine.

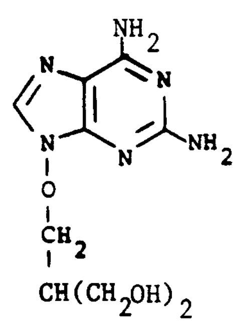

2,6-Diamino-9-(2,2-dimethyl-1,3-dioxan-5-ylmethoxy) purine (310mg, 1.05 mmol) in 80% acetic acid was stirred at 20 °C for 4 hours and then evaporated under reduced pressure. The residue was co-evaporated with toluene and then chromatographed on reverse phase silica (Spherisorb V.L.S. C18 300 pore), eluting with water, 5% methanol and 10% methanol. Recrystallisation from water afforded the title compound (208mg, 78%). IR: $\nu_{max}$ (KBr) 3356, 3208, 1663, 1628, 1600, 1482, 1445, 1409, $cm^{-1}$. $^1H$ NMR: $\delta_H$ [$(CD_3)$-

$_2SO$] 1.95(1H,m,CH), 3.54(4H,m, 2 x $CH_2OH$),4.27(2H,d, J=6.3Hz, $CH_2ON$), 4.62(2H, t, J=5.3Hz, $D_2O$ exchangeable, 2 x OH), 5.92(2H,br.s, $D_2O$ exchangeable, 6-$NH_2$), 6.80(2H,br.s, $D_2O$ exchangeable, 2-$NH_2$), 7.92(1H,s,H-8).

| | | | | |
|---|---|---|---|---|
| Found: | C,39.76; | H,5.90; | N,31.32%; | $M^+$ 254.1124. |
| $C_9H_{14}N_6O_3$. $0.9H_2O$ requires: | C,39.96; | H,5.90; | N,31.07%; | $M^+$ 254.1127. |

Example 22

9-(3-Acetoxyprop-1-oxy)-2-aminopurine

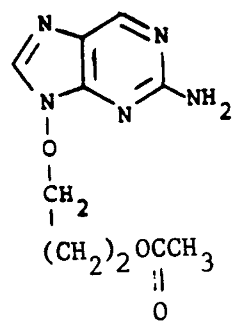

A mixture of 2-amino-9-(3-hydroxyprop-1-oxy)purine (60 mg, 0.287 mmol), acetic anhydride (0.033ml, 0.35 mmol), 4-N,N-dimethylaminopyridine (6mg, 0.05 mmol) and N,N-dimethylformamide (1ml) was stirred at 20°C for 3 hours. Ethanol (0.5ml) was added and the mixture evaporated to dryness. Column chromatography on silica gel (eluted with chloroform-ethanol, 10:1) afforded the title compound (68.8mg, 95%). IR: $\nu_{max}$ (KBr) 3311, 3154, 1721, 1665, 1614, 1572, 1430 $cm^{-1}$. $^1H$ NMR:$\delta_H$[$(CD_3)_2SO$] 2.02-(4H,m,$CH_3$,CH), 4.20(2H, t, J=6.5Hz, $CH_2OC=O$), 4.39(2H, t, J=6.3Hz, $CH_2ON$), 6.70(2H,br.s, $D_2O$ exchangeable, $NH_2$), 8.32(1H,s,H-8), 8.59(1H,s,H-6).

| | |
|---|---|
| Found: | $M^+$ 251.1013. |
| $C_{10}H_{13}N_5O_3$. requires: | $M^+$ 251.1018. |

Example 23

2-Amino-9-(3-hexanoyloxyprop-1-oxy)purine

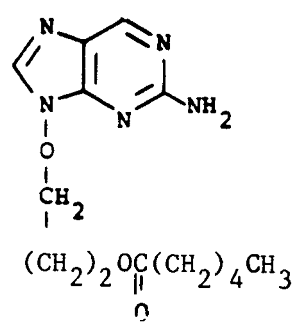

A mixture of 2-amino-9-(3-hydroxyprop-1-oxy)purine (60 mg, 0.287 mmol), hexanoic anhydride (0.1ml, 0.432 mmol), 4-dimethylaminopyridine (6mg, 0.05 mmol) and N,N-dimethylformamide (1ml) was stirred at

20 °C for 3 hours. Ethanol (0.5ml) was added and the solvent removed under reduced pressure. Column chromatography on silica gel (eluted with chloroform-ethanol, 10:1) yielded the title compound (65mg, 74%). IR: $\nu_{max}$ (KBr) 3337, 3187, 1724, 1656, 1616, 1578, 1511, 1429 $cm^{-1}$. $^1H$ NMR:$\delta_H[(CD_3)_2SO]$ 0.84(3H, t, J = 6.8Hz, $CH_3$), 1.24(4H,m,$\underline{CH_2CH_2}CH_3$), 1.52(2H,m,$\underline{CH_2}CH_2C=O$), 2.01(2H,quintet, J = 6.6, 6.3Hz $OCH_2\underline{CH_2}CH_2O$), 2.30(2H, t, J = 7.3Hz, $CH_2C=O$), 4.21(2H, t, J = 6.6Hz,$CH_2OC=O$), 4.39(2H, t, J = 6.3Hz, $CH_2ON$), 6.70(2H,br.s,$D_2O$ exchangeable, $NH_2$), 8.32(1H,s,H-8), 8.59(1H,s,H-6).

| | | | | |
|---|---|---|---|---|
| Found: | C,54.78; | H,6.96; | N,22.99%; | $M^+$ 307.1656. |
| $C_{14}H_{21}N_5O_3$ requires: | C,54.70; | H,6.90; | N,22.79%; | $M^+$ 307.1644. |

Example 24

2-Amino-9-(3-benzoyloxyprop-1-oxy)purine

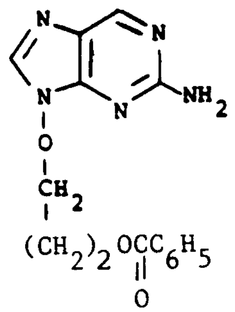

A mixture of 2-amino-9-(3-hydroxyprop-1-oxy)purine (60 mg, 0.287 mmol), benzoic anhydride (97.4mg, 0.431 mmol), 4-dimethylaminopyridine (6mg, 0.05 mmol) and N,N-dimethylformamide (1ml) was stirred at 20 °C for 3 hours. Ethanol (0.5ml) was added and the solvent removed under reduced pressure. Column chromatography on silica gel (eluted with chloroform-ethanol, 10:1) yielded a white solid. Extraction of the solid with boiling ether gave the title compound (65.0mg, 69%). IR: $\nu_{max}$ (KBr) 3351, 3324, 3195, 1713, 1646, 1620, 1573, 1511, 1430 $cm^{-1}$. $^1H$ NMR:$\delta_H[(CD_3)_2SO]$ 2.17(2H, quintet, J = 6.3Hz, $CH_2\underline{CH_2}CH_2$), 4.50-(4H,m,$CH_2ON,CH_2OC=O$), 6.68(2H,br.s, $D_2O$ exchangeable, $NH_2$), 7.53 (2H,m, aromatic), 7.67(1H,m, aromatic) 7.99(2H,m, aromatic), 8.35(1H,s,H-8), 8.60(1H,s,H-6).

| | | | | |
|---|---|---|---|---|
| Found: | C,56.51; | H,4.74; | N,21.86%, | $M^+$ 313.1176. |
| $C_{15}H_{15}N_5O_3 . 0.3H_2O$ requires: | C,56.52; | H,4.94; | N,21.98%; | $M^+$ 313.1175. |

Example 25

(S)-9-(1,4-Diacetoxybut-2-oxy)guanine

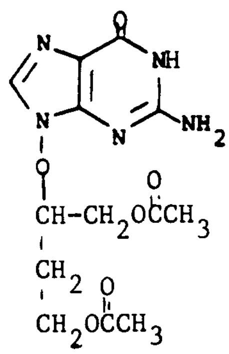

A solution of (S)-9-(1,4-dihydroxybut-2-oxy)guanine (50 mg, 0.2 mmol), in dry N,N-dimethylformamide (3ml) was treated with acetic anhydride (0.15ml, 1.6 mmol) and 4-dimethylaminopyridine (4mg). The solution was stirred at room temperature for 1 hour, the solvent removed and the residue dissolved in chloroform (30ml) and shaken with saturated sodium bicarbonate solution (20ml). The chloroform layer, containing some precipitated product, was evaporated to dryness and the residue chromatographed on silica, eluting with chloroform-methanol (30:1), to give (S)-9-(1,4-diacetoxybut-2-oxy)guanine (40mg, 60%). IR: (KBr) $\nu_{max}$ 3430, 3310, 3200, 3120, 3015, 2930, 2900, 2850, 2790 2740, 1740, 1720, 1700, 1630, 1600, 1535, 1475, 1430, 1380, 1375, 1320, 1240, 1225, 1160, 1115, 1065, 1055, 1010, 960, 945, 900, 890, 860, 825, 780 $cm^{-1}$. $^1H$ NMR:$\delta_H[(CD_3)_2SO]$ $\delta$2.02(8H,m,2 x $CH_3$ plus $CH_2$), 4.17(4H,m,2 x $CH_2$), 4.61(1H,m, CH), 6.47(2H,s, $D_2O$ exchangeable, $NH_2$), 7.90 (1H,s, CH), 10.69 (1H,s,$D_2O$ exchangeable NH).

| | | | |
|---|---|---|---|
| Found: | C,44.50; | H,5.04; | N,19.95%. |
| $C_{13}H_{17}N_5O_6$ . $0.5H_2O$ requires: | C,44.82; | H,5.21; | N,20.11%. |

Example 26

2-Amino-9-(2,3-dihydroxyprop-1-oxy)-6-methoxypurine.

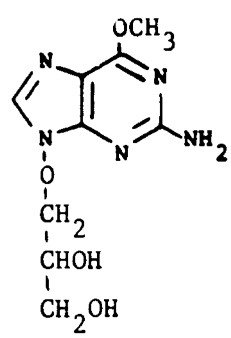

2-Amino-6-chloro-9-(2,3-dihydroxyprop-1-oxy)purine (60 mg, 0.23 mmol) was dissolved in a 1.3M sodium methoxide solution in methanol (0.55ml, 0.68 mmol) and stirred at 100°C for 1.5 hours. The reaction was evaporated to dryness under reduced pressure, the residue absorbed on silica and chromatographed using chloroform-methanol (20:1), affording the title compound (30mg, 51%) as a hygroscopic foam. IR: $\nu_{max}$ (KBr) 3400, 3220, 1620, 1590, 1500, 1480, 1400 $cm^{-1}$; $^1H$ NMR: $\delta_H[(CD_3)_2SO]$ 3.40(2H,m, $\underline{CH_2}OH$), 3.75(1H,m, $\underline{CH}OH$), 3.96 (3H,s, $CH_3$), 4.13(1H,q, J=7.7, 10.5Hz, $CH_2ON$), 4.36(1H,q, J=3.3, 10.5Hz, $CH_2ON$),4.71(1H, t, J=5.8 Hz, $D_2O$ exchangeable, OH), 5.18(1H,d, J=5.2Hz, $D_2O$ exchangeable, OH), 6.64-(2H,br.s, $NH_2$), 8.07(1H,s,H-8). $^m/z$ 255 ($M^+$, 31%).

| | | | | |
|---|---|---|---|---|
| Found: | C, 40.95; | H, 5.40; | N, 26.62%; | $M^+$ 255.0965. |
| $C_9H_{13}N_5O_4.0.5H_2O$. requires: | C, 40.91; | H, 5.34; | N, 26.51%; | $M^+$ 255.0967. |

Example 27

(R)-9-(2,3-Dihydroxyprop-1-oxy)guanine

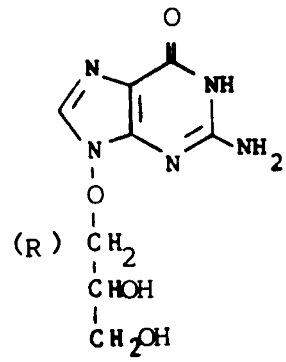

(R)-6-chloro-9-(2,2-dimethyl-1,3-dioxolan-4-ylmethoxy)-2-formamidopurine (150mg, 0.46 mmol) was dissolved in 80% formic acid (6ml) and stirred for 2.5 hours at 100°C. The solvent was removed under reduced pressure, the residue dissolved in methanol (1ml) containing 0.88 ammonia (1ml) and the solution stirred at 25°C for 1 hour. The reaction was evaporated to dryness, the residue dissolved in a minimum amount of water and the solution filtered hot through a glass fibre paper. Slow cooling of the filtrate afforded (R)-9-(2,3-dihydroxyprop-1-oxy)guanine (50mg, 45%), m.p. 255° d., $[\alpha]_D^{25}$-14.9° (C 0.1 in water). UV: $\lambda_{max}$-($H_2O$) 252 ($\epsilon$ 12,200)nm, IR; $\nu_{max}$ (KBr) 3340, 3190, 1690, 1640, 1600, 1540 $cm^{-1}$, $^1H$ NMR: $\delta_H[(CD_3)_2SO]$ 3.39 (2H,m, $C\underline{H}_2OH$), 3.73 (1H,m,CH), 4.10 (1H,q,J=7.7, 10.5 Hz, $CH_2ON$), 4.32 (1H, q, J=3.3, 10.5 Hz, $CH_2ON$), 4.70 (1H, t, J=5.6 Hz, $D_2O$ exchangeable, OH), 5.15. (1H, d, J=5.OHz, $D_2O$ exchangeable, OH), 6.62 (2H, br. s, $D_2O$ exchangeable, $NH_2$), 7.90 (1H, s, H-8), 10.55 (1H, br.s, $D_2O$ exchangeable, NH). $^m/z$ 241 ($M^+$, 2%).

| | | | | |
|---|---|---|---|---|
| Found: | C, 37.91; | H, 4.80; | N, 28.11%; | $M^+$241.0797. |
| $C_8H_{11}N_5O_4.0.5H_2O$. requires | C, 38.39; | H, 4.82; | N, 28.00%; | $M^+$241.0807 |

Example 28

9-(3,4-Dihydroxybut-1-oxy)guanine

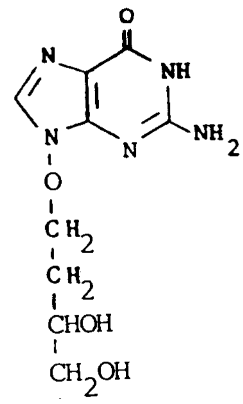

A solution of 2-amino-6-chloro-9-(3,4-dihydroxybut-1-oxy)purine (100mg, 0.37 mmol) in 80% formic acid (7ml) was stirred at 100 °C for 1.5 hours. The reaction was evaporated to dryness, the residue dissolved in methanol (5ml) and 0.88 ammonia (5ml) and stirred for 0.5 hour at 25 °C. The solvents were removed under reduced pressure, the residue dissolved in hot water and filtered. The filtrate was slowly cooled affording 9-(3,4-dihydroxybut-1-yl)guanine (65mg, 70%) as a pale brown solid, m.p. 254-8 °C. UV: $\lambda_{max}$ ($H_2O$) 252 ($\epsilon$ 12,900)nm. IR: $\nu_{max}$ (KBr) 3330, 3170, 1690, 1640, 1600, 1540, 1475 $cm^{-1}$; $^1H$ NMR: $\delta_H$ [$(CD_3)_2SO$] 1.64 (1H, m, $\underline{CH_2}CH_2ON$), 1.88 (1H, m, $\underline{CH_2}CH_2ON$), 3.31 (2H, m, $\underline{CH_2}OH$), 3.61 (1H, m, $\underline{CH}OH$), 4.34 (2H, m, $\underline{CH_2}ON$), 4.58 (1H, t, J=5.5 Hz, $D_2O$ exchangeable, OH), 4.64 (1H, d, J=4.9Hz, $D_2O$ exchangeable, OH). 6.59 (2H, br.s, $D_2O$ exchangeable, $NH_2$), 7.91 (1H, s, H-8), 10.38 (1H, br.s, $D_2O$ exchangeable, NH). $^m/z$ 255 ($M^+$, 10%).

| | | | | |
|---|---|---|---|---|
| Found: | C, 40.78; | H, 5.31; | N, 26.66%; | $M^+$255.0968. |
| $C_9H_{13}N_5O_4.0.5\ H_2O$ requires | C, 40.91; | H, 5.34; | N, 26.51%; | $M^+$255.0967. |

Example 29

2-Amino-9-(3,4-dihydroxybut-1-oxy)purine

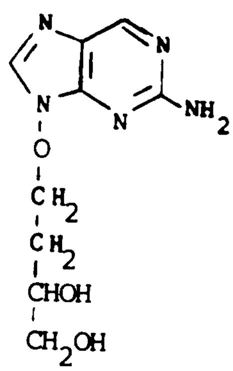

A mixture of 2-amino-6-chloro-9-(3,4-dihydroxybut-1-oxy)purine (150mg, 0.55 mmol), ammonium formate (140mg, 2.2 mmol), 10% palladium on charcoal (20mg) and methanol (5ml) was heated under reflux for 3 hours. The solution was filtered and the filtrate was evaporated to dryness under reduced pressure. The residue was recrystallised from hot ethanol affording 2-amino-9-(3,4-dihydroxybut-1-oxy)purine (100mg, 76%), m.p. 157-8 °C. UV: $\lambda_{max}$ ($H_2O$) 221 ($\epsilon$ 24,700), 305 ($\epsilon$ 7100)nm. IR: $\nu_{max}$ (KBr) 3420, 3310, 3200, 1640,

1620, 1580, 1520, 1480, 1450 cm$^{1}$; $^{1}$H NMR: $\delta_H$ [$(CD_3)_2SO$] 1.65 (1H, m, $CH_2CH_2ON$), 1.91 (1H, m, $CH_2CH_2ON$), 3.32 (2H, m, $CH_2OH$), 3.63 (1H, m, CHOH), 4.41 (2H, m, $CH_2ON$), 4.59 (1H, t, J = 5.7 Hz, $D_2O$ exchangeable, OH), 4.71 (1H, d, J = 5.2 Hz, $D_2O$ exchangeable, OH), 6.71 (2H, br.s, $D_2O$ exchangeable, $NH_2$), 8.30 (1H, s, H-8), 8.59 (1H, s, H-6).

| | | | | |
|---|---|---|---|---|
| Found: | C, 44.94; | H, 5.56; | N, 29.01%; | $M^+$239.1022. |
| $C_9H_{13}N_5O_3$ requires | C, 45.18; | H, 5.48; | N, 29.28%; | $M^+$239.1018. |

Example 30

(R)-2-Amino-9-(2,3-dihydroxyprop-1-oxy)purine

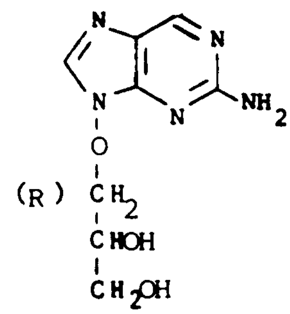

A mixture of (R)-2-amino-6-chloro-9-(2,3-dihydroxyprop-1-oxy)purine (54mg, 0.21 mmol), 10% palladium on charcoal (10mg), ammonium formate (53mg, 0.83 mmol) and methanol (2ml) was heated under reflux for 3 hours. The reaction was cooled, filtered and evaporated to dryness under reduced pressure. The residue was recrystallised from ethanol affording (R)-2-amino-9-(2,3-dihydroxyprop-1-oxy)purine (26mg, 55%) as a pale brown solid, m.p. 145-7 °C. IR: $\nu_{max}$ (KBr) 3380, 3320, 3200, 1650, 1620, 1580, 1520 cm$^{-1}$; $^{1}$H NMR: $\delta_H$ [$(CD_3)_2SO$] 3.40 (2H, m, $CH_2OH$), 3.77 (1H, m, CHOH), 4.18 (1H, q, J = 7.6, 10.5Hz, $CH_2ON$), 4.39 (1H, q, J = 3.3, 10.5Hz, $CH_2ON$), 4.73 (1H, m, $D_2O$ exchangeable, OH), 5.16 (1H, m, $D_2O$ exchangeable, OH), 6.72 (2H, br.s, $D_2O$ exchangeable, $NH_2$), 8.27 (1H, s, H-8), 8.59 (1H, s, H-6).

Example 31

(S)-9-(2,3-Dihydroxyprop-1-oxy)guanine.

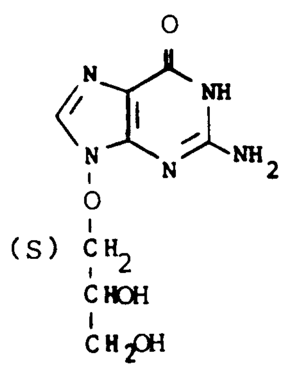

The title compound is prepared by analogous procedures to those described in Description 14 and Example 27.

Antiviral Activity

1. Plaque Reduction Test for Herpes Simplex Viruses 1 and 2.

Cells (Vero or MRC-5) were grown to confluence in 24 well multi-dishes (well diameter = 1.5cm). The drained cell monolayers were each infected with approximately 50 infectious particles of herpes simplex virus 1 (HSV-1; strain HFEM) or herpes simplex virus 2 (HSV-2; strain MS) in 100µl of phosphate-buffered saline. The virus was adsorbed for 1 hour at room temperature. After adsorption, residual inoculum was removed from each well and replaced with 0.5ml of Eagle's MEM containing 5% newborn calf serum and 0.9% agarose (A37). Once the agarose had set, dilutions of the test compound, which had been prepared in Eagle's MEM (containing 5% newborn calf serum), were added, each well receiving 0.5ml of liquid overlay. The test compound was diluted to give the following series of concentrations: 200, 60, 20, 6....0.06µg/ml; final concentrations in the assay ranged, therefore, between 100µg/ml and 0.03µg/ml. The infected cultures were incubated at 37°C in a humidified atmosphere of 5% $CO_2$ until plaques were clearly visible (2 or 3 days for Vero cells, usually 1 day for MRC-5 cells).

2. Plaque Reduction Test for Varicella Zoster-Virus

MRC-5 cells were grown to confluence in 24 well multi-dishes (well diameter = 1.5cm). The drained cell monolayers were each infected with approximately 50 infectious particles of varicella zoster virus (VZV; Ellen strain) in 100µl of phosphate-buffered saline. The virus was adsorbed for 1 hour at room temperature. After adsorption, residual inoculum was removed from each well and replaced with 0.5ml of Eagle's MEM containing 5% heat-inactivated foetal calf serum and 0.9% agarose (A37). Once the agarose had set, dilutions of the test compound, which had been prepared in Eagle's MEM (containing 5% heat-inactivated foetal calf serum), were added, each well receiving 0.5ml of liquid overlay. The test compound was diluted to give the following series of cencentrations: 200, 60, 20, 6....0.06µg/ml; final concentrations in the assay ranged, therefore, between 100µg/ml and 0.03µg/ml. The infected cultures were incubated at 37°C in a humidified atmosphere of 5% $CO_2$ until plaques were clearly visible (5 or 6 days).

Cultures from 1 and 2 were fixed in formal saline, the agarose overlays were carefully washed off, and then the cell monolayers were stained with carbol fuchsin. A stereo microscope was used to count plaques. The $IC_{50}$ (concentration of drug which inhibits plaque number by 50% relative to the number of plaques observed in virus control monolayers) of the test compound was calculated. In addition, the monolayers were examined for evidence of drug-induced cytotoxicity; the minimum concentration at which cytotoxicity occurs was recorded.

3. CPE Inhibition Test (Replicating Cells) for Herpes Simplex Virus 1

MRC-5 cells (in Eagles MEM containing 5% newborn calf serum) were infected in suspension with herpes simplex virus 1, strain SC16 (approximately one infectious particle per 10 cells). One hundred microlitres of the infected cell suspension (containing approximately 2 x $10^4$ cells) were dispensed into each well of a 96 well microtitre plate containing an equal volume of the test drug in medium (Eagles MEM containing 5% newborn calf serum) at concentrations ranging from 200 to 0.09µg/ml (3-fold dilution steps); final concentrations therefore ranged between 100 to 0.045µg/ml. The plates were then incubated at 37°C in a humidified atmosphere of 5% $CO_2$ for 3 days when the virus-induced cytopathic effect (CPE) in the control wells reached 100%. The plates were fixed in formal saline and stained with carbol fuchsin. The plates were then examined to find what concentration of test compound reduced the virus-induced CPE by 50% ($IC_{50}$). Plates of uninfected cells were set up in parallel to determined the minimum concentration of test compound which caused cytotoxicity.

4. CPE Inhibition Test (Established Monolayer) for Lentiviruses

3 x $10^4$ sheep choroid plexus (SCP) cells were plated into individual wells of a 96 well microtitre plate in 100µl of Eagle's MEM with Hanks salts containing 10% heat inactivated foetal calf serum (FCS). When monolayers had become established (after 1 or 2 days growth) they were washed with 200µl of maintenance medium (Eagle's MEM with Hanks salts containing 0.5% FCS) and infected with 100µl of visna virus (strain K184) in maintenance medium (30 $TCID_{50}$/ml). Test samples were diluted with maintenance medium in further 96 well microtitre plates over the range 200-0.09µg/ml by 3-fold dilution steps. 100µl of the diluted samples was then transferred directly onto virus-infected monolayers (final concentration range therefore 100-0.045μg/ml) and incubated at 37°C in a humidified, 5% $CO_2$ incubation until virus-induced CPE was maximal in the untreated virus-infected controls (usually 12-14 days). The plates were fixed with formal saline and stained with crystal violet. Virus-induced CPE was then scored microscopically and the minimum concentration of sample giving complete protection of the cell monolayers (MIC) determined.

Results

| Example No. | $IC_{50}$ (μg/ml)[a] | | | | | MIC (μg/ml) |
|---|---|---|---|---|---|---|
| | Herpes Simplex Type 1 virus | | Herpes Simplex Type 2 virus | | Varicella Zoster virus | Visna Virus |
| | HFEM strain in Vero cells | SC16 strain in MRC-5 cells | MS strain in Vero cells | MS strain in MRC-5 cells | Ellen strain in MRC-5 cells | K184 strain in SCP cells |
| 1 | 0.4,0.2 | 1.0,0.3,0.2 | 0.3,0.2 | 0.1 | 0.8,0.3 | 3.0 |
| 2 | 4.3 | 0.4,0.6 | | 1.1 | | |
| 3 | 2.8 | 1.2 | | 2.1 | | |
| 4 | | 100 | | 67 | 21 | |
| 5 | 1.5 | 1.8,1.8 | 1.5,1.5 | 1.5 | 2.9 | 0.1 |
| 6 | 8.6 | 1.9 | 1.6 | 0.7 | 2.3 | 10 |
| 7 | | 100 | | | 57 | |
| 8 | | 11 | | | | 30 |
| 11 | 0.7 | 0.1 | | | 0.1 | |
| 14 | | 100 | | | | |
| 15 | | 7.4 | | | | |

[a] Each figure is the value obtained in a single test.

Toxicity

At concentrations up to 30μg/ml, none of the compounds was cytotoxic for uninfected cells used in any of the tests.

**Claims**

**Claims for the following Contracting States : BE, CH, DE, FR, GB, IT, LI, LU, NL, SE**

**1.** A compound of formula (I), or a pharmaceutically acceptable salt thereof:

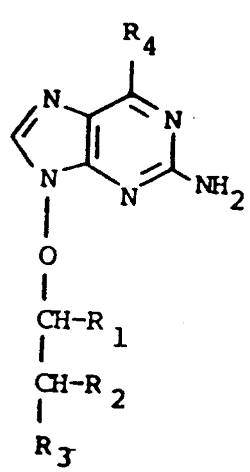

(I)

wherein:
$R_1$ is hydrogen or $CH_2OH$;
$R_2$ is hydrogen or, (when $R_1$ is hydrogen), hydroxy or $CH_2OH$;
$R_3$ is $CH_2OH$ or, (when $R_1$ and $R_2$ are both hydrogen), $CH(OH)CH_2OH$;
$R_4$ is hydrogen, hydroxy, amino or $OR_5$ wherein
$R_5$ is $C_{1-6}$ alkyl, phenyl or phenyl $C_{1-2}$ alkyl either of which phenyl moieties may be substituted by one or two halo, $C_{1-4}$ alkyl or $C_{1-4}$ alkoxy groups;
and in which any OH groups in $R_1$, $R_2$ and/or $R_3$ may be in the form of O-acyl, phosphate, cyclic acetal or cyclic carbonate derivatives thereof, wherein O-acyl derivatives are wherein one or two OH groups in $R_1$, $R_2$ and/or $R_3$ form carboxylic ester groups which are $C_{1-7}$ alkanoyl and benzoyl optionally substituted by one or two $C_{1-4}$ alkyl, $C_{1-4}$ alkoxy, halogen or $CF_3$ groups;
phosphate derivatives are wherein one of the acyclic OH groups is replaced by $(HO)_2$-$PO_2$ or wherein two OH groups on adjacent carbon atoms are replaced by a bridging -O-P(OH)$O_2$- group; a cyclic acetal derivative is wherein two OH groups in $R_1$, $R_2$ and $R_3$ are -O-C($C_{1-3}$ alkyl)$_2$-O-; and wherein cyclic carbonate is -O-CO-O-.

**2.** A compound of formula (I) according to claim 1, wherein $R_1$ and $R_2$ are both hydrogen and $R_3$ is $CH_2OH$, and derivatives thereof as defined in claim 1.

**3.** A compound of formula (I) according to claim 1, wherein $R_1$ is hydrogen and $R_2$ and $R_3$ are both $CH_2OH$, and derivatives thereof as defined in claim 1.

**4.** A compound of formula (I) according to claim 1, wherein $R_1$ is hydrogen, $R_2$ is hydroxy and $R_3$ is $CH_2OH$, and derivatives thereof as defined in claim 1.

**5.** A compound of formula (I) according to claim 1, wherein $R_1$ is $CH_2OH$, $R_2$ is hydrogen and $R_3$ is $CH_2OH$, and derivatives thereof as defined in claim 1.

**6.** A compound of formula (I) according to claim 1, wherein $R_1$ and $R_2$ are both hydrogen and $R_3$ is CH-(OH)$CH_2OH$, and derivatives thereof as defined in claim 1.

**7.** A compound according to any one of claims 1 to 6 wherein $R_4$ is hydroxy.

**8.** A compound according to any one of claims 1 to 6 wherein $R_4$ is hydrogen.

**9.** A compound according to any one of claims 1 to 8 wherein OH groups in $R_1$, $R_2$, and/or $R_3$ are in the form of an acyl derivative thereof wherein the acyl derivative(s) is/are an acetate, hexanoate or benzoate.

**10.** 9-(3-Hydroxy-2-hydroxymethylpropl-1-oxy)guanine,
9-(2,3-dihydroxyprop-1-oxy)guanine,
2-amino-9-(2,3-dihydroxyprop-1-oxy)purine,
9-(1,4-dihydroxybut-2-oxy)guanine,
2-amino-9-(1,4-dihydroxybut-2-oxy)purine,
2-amino-9-(1,4-diacetoxybut-2-oxy)purine,
2-amino-9-(1,4-dibutyryloxybut-2-oxy)purine,
(R)-9-(1,4-dihydroxybut-2-oxy)guanine,
(S)-9-(1,4-dihydroxybut-2-oxy)guanine,
2-amino-9-(3-hydroxy-2-hydroxymethylprop-1-oxy)purine,
9-(3-acetoxy-2-acetoxymethylprop-1-oxy)-2-aminopurine,
2-amino-9-(3-propionyloxy-2-propionyloxymethylprop-1-oxy)purine,
2-amino-9-(3-benzoyloxy-2-benzoyloxymethylprop-1-oxy)purine,
2-amino-9-(3-hydroxy-2-hydroxymethylprop-1-oxy)-6-methoxypurine,
2,6-diamino-9-(3-hydroxy-2-hydroxymethylprop-1-oxy)purine,
(S)-9-(1,4-diacetoxybut-2-oxy)guanine,
2-amino-9-(2,3-dihydroxyprop-1-oxy)-6-methoxypurine.
(R)-9-(2,3-dihydroxyprop-1-oxy)guanine,
9-(3,4-dihydroxybut-1-oxy)guanine,

2-amino-9-(3,4-dihydroxybut-1-oxy)purine,
(R)-2-amino-9-(2,3-dihydroxyprop-1-oxy)purine or
(S)-9-(2,3-dihydroxyprop-1-oxy)guanine.

**11.** 9-(3-Hydroxyprop-1-oxy)guanine,
9-(3-acetoxyprop-1-oxy)guanine,
9-(3-benzoyloxyprop-1-oxy)guanine,
2-amino-6-ethoxy-9-(3-hydroxyprop-1-oxy)purine,
2-amino-9-(3-hydroxyprop-1-oxy)purine,
9-(3-hexanoyloxyprop-1-oxy)guanine,
9-(3-acetoxyprop-1-oxy)-2-aminopurine,
2-amino-9-(3-hexanoyloxyprop-1-oxy)purine or
2-amino-9-(3-benzoyloxyprop-1-oxy)purine.

**12.** A process for the preparation of a compound of formula (I) according to claim 1 or a pharmaceutically acceptable salt thereof, which process comprises cyclising a compound of formula (II):

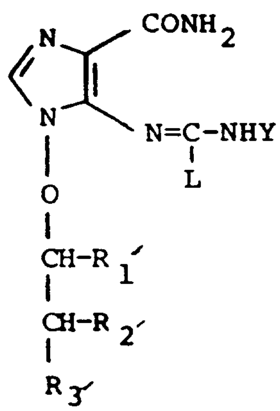

(II)

wherein L is a sulphur leaving group or $NH_2$, Y is a protecting group and $R_1'$, $R_2'$ and $R_3'$ are $R_1$, $R_2$ and $R_3$ respectively or $R_1$, $R_2$ and/or $R_3$ wherein OH groups are protected; and thereafter, when $R_4$ in the desired compound of formula (I) is other than hydroxy, converting an $R_4$ hydroxy group to chloro, followed by either (i) reduction to $R_4$ is hydrogen, (ii) replacement of chloro by $NH_2$ to give $R_4$ is amino; or (iii) reaction with $OR_5^-$ ion to give $R_4$ is $OR_5$; and deprotecting Y and converting $R_1'$ to $R_1$, $R_2'$ to $R_2$ and $R_3'$ to $R_3$ when desired or necessary, and optionally forming a pharmaceutically acceptable salt, O-acyl, phosphate, cyclic acetal or cyclic carbonate derivative thereof.

**13.** A process for the preparation of a compound of formula (I) according to claim 1 or a pharmaceutically acceptable salt thereof, which process comprises converting the chloro group in a compound of formula (VI):

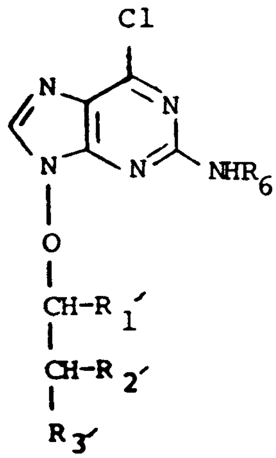

(VI)

wherein $R_6$ is hydrogen or an N-protecting group, and $R_1$', $R_2$' and $R_3$' are as defined in claim 12, by either (i) hydrolysis to give $R_4$ as hydroxy, (ii) reduction to give a compound of formula (I) wherein $R_4$ is hydrogen, (iii) replacement of chloro by $NH_2$ to give $R_4$ is amino; or (iv) reaction with $OR_5^-$ to give a compound of formula (I) wherein $R_4$ is $OR_5$, and thereafter converting $R_1$' to $R_1$, $R_2$' to $R_2$ and/or $R_3$' to $R_3$ when necessary, and/or optionally forming a pharmaceutically acceptable salt or derivative thereof as defined in claim 1.

**14.** A process for the preparation of a compound of formula (I) according to claim 1, which process comprises the deprotection of a compound of formula (I) as defined in claim 1, wherein any OH groups in $R_1$, $R_2$ and/or $R_3$ are in protected form; and thereafter optionally forming a pharmaceutically acceptable salt or derivative thereof as defined in claim 1.

**15.** A compound of formula (II) as defined in claim 12.

**16.** A compound of formula (V):

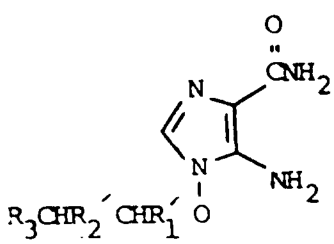

(V)

wherein $R_1$', $R_2$' and $R_3$' are as defined in claim 12.

**17.** A compound of formula (VI) as defined in claim 13.

**18.** A compound of formula (VIII):

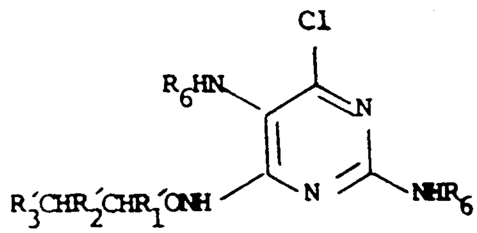

wherein $R_6$ is formyl, and $R_1'$, $R_2'$, and $R_3'$ are as defined in claim 12.

**19.** A compound of formula (IV):

$R_3'CHR_2'CHR_1'ONH_2$ (IV)

wherein $R_1'$, $R_2'$, and $R_3'$, are as defined in claim 12, other than compounds wherein $R_2'$ and $R_3'$ are protected OH groups wherein OH groups on adjacent carbon atoms are replaced by a bond.

**20.** A compound of formula (I) as defined in claim 1, except that $R_1$, $R_2$ and/or $R_3$ are in protected form as defined for $R_1'$, $R_2'$and/or $R_3'$ in claim 12.

**21.** The intermediate compound of formula (II), (V), (VI), (VIII) or (IV), as defined in claims 14 to 18, or the intermediate compound according to claim 19, for the preparation of any compound according to claim 10.

**22.** A pharmaceutical composition comprising a compound according to any one of claims 1 to 10, or a pharmaceutically acceptable salt thereof, and a pharmaceutically acceptable carrier.

**23.** A compound according to any one of claims 1 to 10, or a pharmaceutically acceptable salt thereof, for use as an active therapeutic substance.

**24.** A compound according to any one of claims 1 to 10, or a pharmaceutically acceptable salt thereof, for use in the treatment of viral infections.

**25.** Use of a compound according to any one of claims 1 to 10, or a pharmaceutically acceptable salt thereof, in the manufacture of a medicament for the treatment of viral infections.

**26.** Use of a compound according to claim 11, or a pharmaceutically acceptable salt thereof, in the manufacture of a medicament for the treatment of herpesvirus infections.

**Claims for the following Contracting States : AT, ES**

**1.** A process for the preparation of a compound of formula (I), or a pharmaceutically acceptable salt thereof:

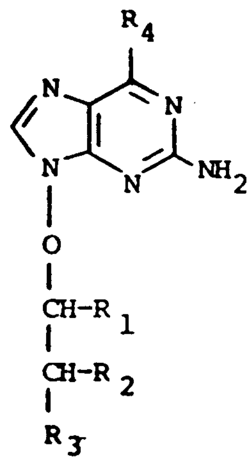

(I)

wherein:

$R_1$ is hydrogen or $CH_2OH$;

$R_2$ is hydrogen or, (when $R_1$ is hydrogen), hydroxy or $CH_2OH$;

$R_3$ is $CH_2OH$ or, (when $R_1$ and $R_2$ are both hydrogen), $CH(OH)CH_2OH$;

$R_4$ is hydrogen, hydroxy, amino or $OR_5$ wherein

$R_5$ is $C_{1-6}$ alkyl, phenyl or phenyl $C_{1-2}$ alkyl either of which phenyl moieties may be substituted by one or two halo, $C_{1-4}$ alkyl or $C_{1-4}$ alkoxy groups;

and in which any OH groups in $R_1$, $R_2$ and/or $R_3$ may be in the form of O-acyl, phosphate, cyclic acetal or cyclic phosphate derivatives thereof; wherein O-acyl derivatives are wherein one or two OH groups in $R_1$, $R_2$ and/or $R_3$ form carboxylic ester groups which are $C_{1-7}$ alkanoyl and benzoyl optionally substituted by one or two $C_{1-4}$ alkyl, $C_{1-4}$ alkoxy, halogen or $CF_3$ groups;

phosphate derivatives are wherein one of the acyclic OH groups is replaced by $(HO)_2$-$PO_2$ or wherein two OH groups on adjacent carbon atoms are replaced by a bridging -O-P(OH)$O_2$- group; a cyclic acetal derivative is wherein two OH groups in $R_1$, $R_2$ and $R_3$ are -O-C($C_{1-3}$ alkyl)$_2$-O-; and wherein cyclic carbonate is -O-CO-O-;

which process comprises cyclising a compound of formula (II):

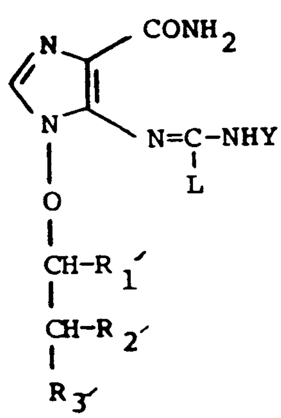

(II)

wherein L is a sulphur leaving group or $NH_2$, Y is a protecting group and $R_1'$, $R_2'$ and $R_3'$ are $R_1$, $R_2$ and $R_3$ respectively or $R_1$, $R_2$ and/or $R_3$ wherein OH groups are protected; and thereafter, when $R_4$ in the desired compound of formula (I) is other than hydroxy, converting an $R_4$ hydroxy group to chloro, followed by either (i) reduction to $R_4$ is hydrogen, (ii) replacement of chloro by $NH_2$ to give $R_4$ is amino; or (iii) reaction with $OR_5^-$ ion to give $R_4$ is $OR_5$; and deprotecting Y and converting $R_1'$ to $R_1$, $R_2'$ to $R_2$ and $R_3'$ to $R_3$ when desired or necessary, and optionally forming a pharmaceutically acceptable salt, O-acyl, phosphate, cyclic acetal or cyclic carbonate derivative thereof.

2. A process for the preparation of a compound of formula (I) as defined in claim 1, or a pharmaceutically acceptable salt thereof, which process comprises converting the chloro group in a compound of formula (VI):

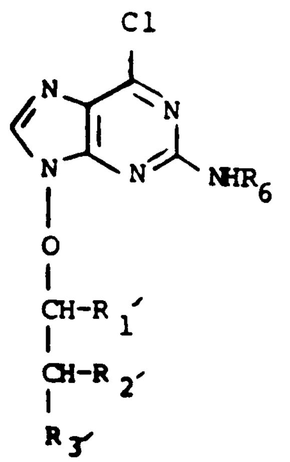

(VI)

wherein $R_6$ is hydrogen or an N-protecting group, and $R_1'$, $R_2'$ and $R_3'$ are as defined in claim 1, by either (i) hydrolysis to give $R_4$ as hydroxy, (ii) reduction to give a compound of formula (I) wherein $R_4$ is hydrogen, (iii) replacement of chloro by $NH_2$ to give $R_4$ is amino; or (iv) reaction with $OR_5^-$ to give a compound of formula (I) wherein $R_4$ is $OR_5$, and thereafter converting $R_1'$ to $R_1$, $R_2'$ to $R_2$ and/or $R_3'$ to $R_3$ when necessary, and/or optionally forming a pharmaceutically acceptable salt or derivative thereof as defined in claim 1.

3. A process for the preparation of a compound of formula (I) as defined in claim 1, which process comprises the deprotection of a compound of formula (I) as defined in claim 1, wherein any OH groups in $R_1$, $R_2$ and/or $R_3$ are in protected form; and thereafter optionally forming a pharmaceutically acceptable salt or derivative thereof as defined in claim 1.

4. A process according to any one of claims 1 to 3 for the preparation of a compound of formula (I) as defined in claim 1, wherein $R_1$ and $R_2$ are both hydrogen and $R_3$ is $CH_2OH$, and derivatives thereof as defined in claim 1.

5. A process according to any one of claims 1 to 3 for the preparation of a compound of formula (I) as defined in claim 1, wherein $R_1$ is hydrogen and $R_2$ and $R_3$ are both $CH_2OH$, and derivatives thereof as defined in claim 1.

6. A process according to any one of claims 1 to 3 for the preparation of a compound of formula (I) as defined in claim 1, wherein $R_1$ is hydrogen, $R_2$ is hydroxy and $R_3$ is $CH_2OH$, and derivatives thereof as defined in claim 1.

7. A process according to any one of claims 1 to 3 for the preparation of a compound of formula (I) as defined in claim 1, wherein $R_1$ is $CH_2OH$, $R_2$ is hydrogen and $R_3$ is $CH_2OH$, and derivatives thereof as defined in claim 1.

8. A process according to any one of claims 1 to 3 for the preparation of a compound of formula (I) as defined in claim 1, wherein $R_1$ and $R_2$ are both hydrogen and $R_3$ is $CH(OH)CH_2OH$, and derivatives thereof as defined in claim 1.

9. A process according to any one of claims 1 to 3 for the preparation of a compound of formula (I) as defined in any one of claims 1 and 4 to 8, wherein $R_4$ is hydroxy.

10. A process according to any one of claims 1 to 3 for the preparation of a compound of formula (I) as defined in any one of claims 1 and 4 to 8, wherein $R_4$ is hydrogen.

**11.** A process according to any one of claims 1 to 3 for the preparation of a compound or formula (I) as defined in any one of claims 4 to 10 wherein OH groups in $R_1$, $R_2$, and/or $R_3$ are in the form of an acyl derivative thereof wherein the acyl derivative(s) is/are an acetate, hexanoate or benzoate.

**12.** A process according to any one of claims 1 to 3 for the preparation of a compound of formula (I) which is:
9-(3-hydroxy-2-hydroxymethylprop-1-oxy)guanine,
9-(2,3-dihydroxyprop-1-oxy)guanine,
2-amino-9-(2,3-dihydroxyprop-1-oxy)purine,
9-(1,4-dihydroxybut-2-oxy)guanine,
2-amino-9-(1,4-dihydroxybut-2-oxy)purine,
2-amino-9-(1,4-diacetoxybut-2-oxy)purine,
2-amino-9-(1,4-dibutyryloxybut-2-oxy)purine,
(R)-9-(1,4-dihydroxybut-2-oxy)guanine,
(S)-9-(1,4-dihydroxybut-2-oxy)guanine,
2-amino-9-(3-hydroxy-2-hydroxymethylprop-1-oxy)-purine,
9-(3-acetoxy-2-acetoxymethylprop-1-oxy)-2-aminopurine,
2-amino-9-(3-propionyloxy-2-propionyloxymethylprop-1-oxy)purine,
2-amino-(3-benzoyloxy-2-benzoyloxymethylprop-1-oxy)purine,
2-amino-9-(3-hydroxy-2-hydroxymethylprop-1-oxy)-6-methoxypurine,
2,6-diamino-9-(3-hydroxy-2-hydroxymethylprop-1-oxy)purine,
(S)-9-(1,4-diacetoxybut-2-oxy)guanine,
2-amino-9-(2,3-dihydroxyprop-1-oxy)-6-methoxypurine,
(R)-9-(2,3-dihydroxyprop-1-oxy)guanine,
9-(3,4-dihydroxybut-1-oxy)guanine,
2-amino-9-(3,4-dihydroxybut-1-oxy)purine,
(R)-2-amino-9-(2,3-dihydroxyprop-1-oxy)purine or
(S)-9-(2,3-dihydroxyprop-1-oxy)guanine.

**13.** A process according to any one of claims 1 to 3, for the preparation of a compound of formula (I) which is:
9-(3-hydroxyprop-1-oxy)guanine,
9-(3-acetoxyprop-1-oxy)guanine,
9-(3-benzoyloxyprop-1-oxy)guanine,
2-amino-6-ethoxy-9-(3-hydroxyprop-1-oxy)purine,
2-amino-9-(3-hydroxyprop-1-oxy)purine,
9-(3-hexanoyloxyprop-1-oxy)guanine,
9-(3-acetoxyprop-1-oxy)-2-aminopurine,
2-amino-9-(3-hexanoyloxyprop-1-oxy)purine or
2-amino-9-(3-benzoyloxyprop-1-oxy)purine.

**14.** Use of a compound of formula (I) as defined in any one of claims 1 and 4 to 12, or a pharmaceutically acceptable salt thereof, in the manufacture of a medicament for the treatment of viral infections.

**15.** Use of a compound of formula (I), as defined in claim 13, or a pharmaceutically acceptable salt thereof, in the manufacture of a medicament for the treatment of herpesvirus infections.

**Claims for the following Contracting State : GR**

**1.** A compound of formula (I), or a pharmaceutically acceptable salt thereof:

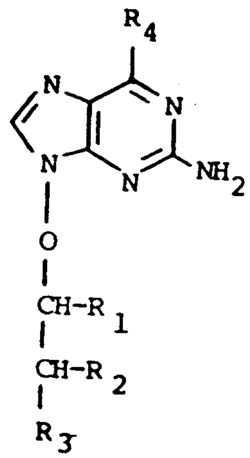

(I)

wherein:
$R_1$ is hydrogen or $CH_2OH$;
$R_2$ is hydrogen or, (when $R_1$ is hydrogen), hydroxy or $CH_2OH$;
$R_3$ is $CH_2OH$ or, (when $R_1$ and $R_2$ are both hydrogen), $CH(OH)CH_2OH$;
$R_4$ is hydrogen, hydroxy, amino or $OR_5$ wherein
$R_5$ is $C_{1-6}$ alkyl, phenyl or phenyl $C_{1-2}$ alkyl either of which phenyl moieties may be substituted by one or two halo, $C_{1-4}$ alkyl or $C_{1-4}$ alkoxy groups;
and in which any OH groups in $R_1$, $R_2$ and/or $R_3$ may be in the form of O-acyl, phosphate, cyclic acetal or cyclic carbonate derivatives thereof, wherein O-acyl derivatives are wherein one or two OH groups in $R_1$, $R_2$ and/or $R_3$ form carboxylic ester groups which are $C_{1-7}$ alkanoyl and benzoyl optionally substituted by one or two $C_{1-4}$ alkyl, $C_{1-4}$ alkoxy, halogen or $CF_3$ groups;
phosphate derivatives are wherein one of the acyclic OH groups is replaced by $(HO)_2$-$PO_2$ or wherein two OH groups on adjacent carbon atoms are replaced by a bridging -O-P(OH)$O_2$- group; a cyclic acetal derivative is wherein two OH groups in $R_1$, $R_2$ and $R_3$ are -O-C($C_{1-3}$ alkyl)$_2$-O-; and wherein cyclic carbonate is -O-CO-O-.

**2.** A compound of formula (I) according to claim 1, wherein $R_1$ and $R_2$ are both hydrogen and $R_3$ is $CH_2OH$, and derivatives thereof as defined in claim 1.

**3.** A compound of formula (I) according to claim 1, wherein $R_1$ is hydrogen and $R_2$ and $R_3$ are both $CH_2OH$, and derivatives thereof as defined in claim 1.

**4.** A compound of formula (I) according to claim 1, wherein $R_1$ is hydrogen, $R_2$ is hydroxy and $R_3$ is $CH_2OH$, and derivatives thereof as defined in claim 1.

**5.** A compound of formula (I) according to claim 1, wherein $R_1$ is $CH_2OH$, $R_2$ is hydrogen and $R_3$ is $CH_2OH$, and derivatives thereof as defined in claim 1.

**6.** A compound of formula (I) according to claim 1, wherein $R_1$ and $R_2$ are both hydrogen and $R_3$ is CH-(OH)$CH_2OH$, and derivatives thereof as defined in claim 1.

**7.** A compound according to any one of claims 1 to 6 wherein $R_4$ is hydroxy.

**8.** A compound according to any one of claims 1 to 6 wherein $R_4$ is hydrogen.

**9.** A compound according to any one of claims 1 to 8 wherein OH groups in $R_1$, $R_2$, and/or $R_3$ are in the form of an acyl derivative thereof wherein the acyl derivative(s) is/are an acetate, hexanoate or

benzoate.

**10.** 9-(3-Hydroxy-2-hydroxymethylprop-1-oxy)guanine,
9-(2,3-dihydroxyprop-1-oxy)guanine,
2-amino-9-(2,3-dihydroxyprop-1-oxy)purine,
9-(1,4-dihydroxybut-2-oxy)guanine,
2-amino-9-(1,4-dihydroxybut-2-oxy)purine,
2-amino-9-(1,4-diacetoxybut-2-oxy)purine,
2-amino-9-(1,4-dibutyryloxybut-2-oxy)purine,
(R)-9-(1,4-dihydroxybut-2-oxy)guanine,
(S)-9-(1,4-dihydroxybut-2-oxy)guanine,
2-amino-9-(3-hydroxy-2-hydroxymethylprop-1-oxy)purine,
9-(3-acetoxy-2-acetoxymethylprop-1-oxy)-2-aminopurine,
2-amino-9-(3-propionyloxy-2-propionyloxymethylprop-1-oxy)purine,
2-amino-9-(3-benzoyloxy-2-benzoyloxymethylprop-1-oxy)purine,
2-amino-9-(3-hydroxy-2-hydroxymethylprop-1-oxy)-6-methoxypurine,
2,6-diamino-9-(3-hydroxy-2-hydroxymethylprop-1-oxy)purine,
(S)-9-(1,4-diacetoxybut-2-oxy)guanine,
2-amino-9-(2,3-dihydroxyprop-1-oxy)-6-methoxypurine.
(R)-9-(2,3-dihydroxyprop-1-oxy)guanine,
9-(3,4-dihydroxybut-1-oxy)guanine,
2-amino-9-(3,4-dihydroxybut-1-oxy)purine,
(R)-2-amino-9-(2,3-dihydroxyprop-1-oxy)purine or
(S)-9-(2,3-dihydroxyprop-1-oxy)guanine.

**11.** 9-(3-Hydroxyprop-1-oxy)guanine,
9-(3-acetoxyprop-1-oxy)guanine,
9-(3-benzoyloxyprop-1-oxy)guanine,
2-amino-6-ethoxy-9-(3-hydroxyprop-1-oxy)purine,
2-amino-9-(3-hydroxyprop-1-oxy)purine,
9-(3-hexanoyloxyprop-1-oxy)guanine,
9-(3-acetoxyprop-1-oxy)-2-aminopurine,
2-amino-9-(3-hexanoyloxyprop-1-oxy)purine or
2-amino-9-(3-benzoyloxyprop-1-oxy)purine.

**12.** A process for the preparation of a compound of formula (I) according to claim 1 or a pharmaceutically acceptable salt thereof, which process comprises cyclising a compound of formula (II):

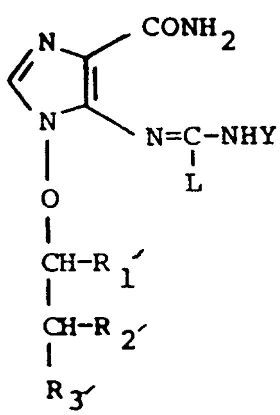

(II)

wherein L is a sulphur leaving group or $NH_2$, Y is a protecting group and $R_1'$, $R_2'$ and $R_3'$ are $R_1$, $R_2$ and $R_3$ respectively or $R_1$, $R_2$ and/or $R_3$ wherein OH groups are protected; and thereafter, when $R_4$ in the desired compound of formula (I) is other than hydroxy, converting an $R_4$ hydroxy group to chloro,

followed by either (i) reduction to $R_4$ is hydrogen, (ii) replacement of chloro by $NH_2$ to give $R_4$ is amino; or (iii) reaction with $OR_5^-$ ion to give $R_4$ is $OR_5$; and deprotecting Y and converting $R_1'$ to $R_1$, $R_2'$ to $R_2$ and $R_3'$ to $R_3$ when desired or necessary, and optionally forming a pharmaceutically acceptable salt, O-acyl, phosphate, cyclic acetal or cyclic carbonate derivative thereof.

**13.** A process for the preparation of a compound of formula (I) according to claim 1 or a pharmaceutically acceptable salt thereof, which process comprises converting the chloro group in a compound of formula (VI):

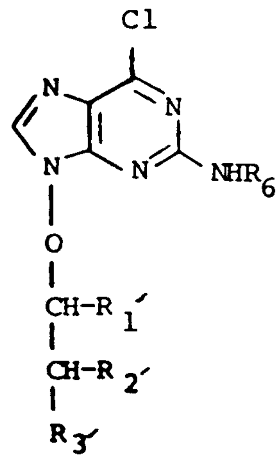

(VI)

wherein $R_6$ is hydrogen or an N-protecting group, and $R_1'$, $R_2'$ and $R_3'$ are as defined in claim 12, by either (i) hydrolysis to give $R_4$ as hydroxy, (ii) reduction to give a compound of formula (I) wherein $R_4$ is hydrogen, (iii) replacement of chloro by $NH_2$ to give $R_4$ is amino; or (iv) reaction with $OR_5^-$ to give a compound of formula (I) wherein $R_4$ is $OR_5$, and thereafter converting $R_1'$ to $R_1$, $R_2'$ to $R_2$ and/or $R_3'$ to $R_3$ when necessary, and/or optionally forming a pharmaceutically acceptable salt or derivative thereof as defined in claim 1.

**14.** A process for the preparation of a compound of formula (I) according to claim 1, which process comprises the deprotection of a compound of formula (I) as defined in claim 1, wherein any OH groups in $R_1$, $R_2$ and/or $R_3$ are in protected form; and thereafter optionally forming a pharmaceutically acceptable salt or derivative thereof as defined in claim 1.

**15.** A compound of formula (II) as defined in claim 12.

**16.** A compound of formula (V):

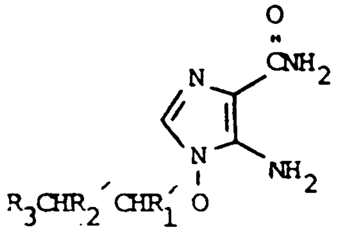

(V)

wherein $R_1'$, $R_2'$ and $R_3'$ are as defined in claim 12.

**17.** A compound of formula (VI) as defined in claim 13.

**18.** A compound of formula (VIII):

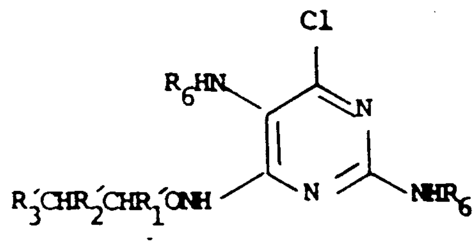

wherein $R_6$ is formyl, and $R_1$', $R_2$', and $R_3$' are as defined in claim 12.

**19.** A compound of formula (IV):

$R_3$'$CHR_2$'$CHR_1$'$ONH_2$ (IV)

wherein $R_1$', $R_2$', and $R_3$', are as defined in claim 12, other than compounds wherein $R_2$' and $R_3$' are protected OH groups wherein OH groups on adjacent carbon atoms are replaced by a bond.

**20.** A compound of formula (I) as defined in claim 1, except that $R_1$, $R_2$ and/or $R_3$ are in protected form as defined for $R_1$', $R_2$'and/or $R_3$' in claim 12.

**21.** The intermediate compound of formula (II), (V), (VI), (VIII) or (IV), as defined in claims 14 to 18, or the intermediate compound according to claim 19, for the preparation of any compound according to claim 10.

**22.** Use of a compound according to any one of claims 1 to 10, or a pharmaceutically acceptable salt thereof, in the manufacture of a medicament for the treatment of viral infections.

**23.** Use of a compound according to claim 11, or a pharmaceutically acceptable salt thereof, in the manufacture of a medicament for the treatment of herpesvirus infections.

**Patentansprüche**
**Patentansprüche für folgende Vertragsstaaten : BE, CH, DE, FR, GB, IT, LI, LU, NL, SE**

**1.** Verbindung der Formel (I) oder ein pharmazeutisch verträgliches Salz davon:

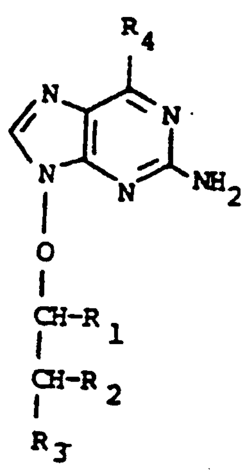

(I)

worin:
$R_1$ ein Wasserstoffatom oder die $CH_2OH$-Gruppe ist;

$R_2$ ein Wasserstoffatom oder (wenn $R_1$ ein Wasserstoffatom ist) eine Hydroxylgruppe oder die $CH_2OH$-Gruppe ist;
$R_3$ die $CH_2OH$-Gruppe oder (wenn $R_1$ und $R_2$ beide ein Wasserstoffatom sind) die $CH(OH)CH_2OH$-Gruppe ist;
$R_4$ ein Wasserstoffatom, eine Hydroxyl-, Aminogruppe oder ein $OR_5$-Rest ist, wobei
$R_5$ ein $C_{1-6}$-Alkylrest, eine Phenylgruppe oder ein Phenyl-$C_{1-2}$-alkyl-Rest ist, wobei jede der Phenyleinheiten durch ein oder zwei Halogenatomen, $C_{1-4}$-Alkyl- oder $C_{1-4}$-Alkoxyresten substituiert sein kann;
und wobei jede der OH-Gruppen in $R_1$, $R_2$ und/oder $R_3$ in Form des O-Acyl-, Phosphat-, cyclischen Acetal- oder cyclischen Carbonat-Derivats davon vorliegen kann, wobei O-Acyl-Derivate jene sind, bei denen ein oder zwei OH-Gruppen in $R_1$, $R_2$ und/oder $R_3$ Carboxylesterreste bilden, die $C_{1-7}$-Alkanoylreste und Benzoylgruppen sind, die gegebenenfalls mit ein oder zwei $C_{1-4}$-Alkyl-, $C_{1-4}$-Alkoxyresten, Halogenatomen oder $CF_3$-Gruppen substituiert sind;
die Phosphat-Derivate jene sind, bei denen eine der acyclischen OH-Gruppen durch $(HO)_2$-$PO_2$ ersetzt ist, oder wobei zwei OH-Gruppen auf benachbarten Kohlenstoffatomen durch eine überbrückende -O-P-(OH)$O_2$-Gruppe ersetzt sind;
das cyclische Acetal-Derivat ein Derivat ist, bei dem zwei OH-Gruppen in $R_1$, $R_2$ und $R_3$ ein -O-C-($C_{1-3}$-Alkyl)$_2$-O-Rest ist; und wobei das cyclische Carbonat-Derivat -O-CO-O-ist.

**2.** Verbindung der Formel (I) nach Anspruch 1, wobei $R_1$ und $R_2$ beide ein Wasserstoffatom sind und $R_3$ die $CH_2OH$-Gruppe ist, und Derivate davon nach Anspruch 1.

**3.** Verbindung der Formel (I) nach Anspruch 1, wobei $R_1$ ein Wasserstoffatom ist und $R_2$ und $R_3$ beide die $CH_2OH$-Gruppe sind, und Derivate davon nach Anspruch 1.

**4.** Verbindung der Formel (I) nach Anspruch 1, wobei $R_1$ ein Wasserstoffatom ist, $R_2$ eine Hydroxylgruppe ist und $R_3$ die $CH_2OH$-Gruppe ist, und Derivate davon nach Anspruch 1.

**5.** Verbindung der Formel (I) nach Anspruch 1, wobei $R_1$ die $CH_2OH$-Gruppe ist, $R_2$ ein Wasserstoffatom ist und $R_3$ die $CH_2OH$-Gruppe ist, und Derivate davon nach Anspruch 1.

**6.** Verbindung der Formel (I) nach Anspruch 1, wobei $R_1$ und $R_2$ beide ein Wasserstoffatom sind und $R_3$ die $CH(OH)CH_2OH$-Gruppe ist, und Derivate davon nach Anspruch 1.

**7.** Verbindung nach einem der Ansprüche 1 bis 6, wobei $R_4$ eine Hydroxylgruppe ist.

**8.** Verbindung nach einem der Ansprüche 1 bis 6, wobei $R_4$ ein Wasserstoffatom ist.

**9.** Verbindung nach einem der Ansprüche 1 bis 8, wobei die OH-Gruppen in $R_1$, $R_2$ und/oder $R_3$ in Form eines Acyl-Derivats davon vorliegen, wobei das (die) Acyl-Derivat(e) Acetat, Hexanoat oder Benzoat ist (sind).

**10.** 9-(3-Hydroxy-2-hydroxymethylprop-1-oxy)guanin,
9-(2,3-Dihydroxyprop-1-oxy)guanin,
2-Amino-9-(2,3-dihydroxyprop-1-oxy)purin,
9-(1,4-Dihydroxybut-2-oxy)guanin,
2-Amino-9-(1,4-dihydroxybut-2-oxy)purin,
2-Amino-9-(1,4-diacetoxybut-2-oxy)purin,
2-Amino-9-(1,4-dibutyryloxybut-2-oxy)purin,
(R)-9-(1,4-Dihydroxybut-2-oxy)guanin,
(S)-9-(1,4-Dihydroxybut-2-oxy)guanin,
2-Amino-9-(3-hydroxy-2-hydroxymethylprop-1-oxy)purin,
9-(3-Acetoxy-2-acetoxymethylprop-1-oxy)-2-aminopurin,
2-Amino-9-(3-propionyloxy-2-propionyloxymethylprop-1-oxy)purin,
2-Amino-9-(3-benzoyloxy-2-benzoyloxymethylprop-1-oxy)purin,
2-Amino-9-(3-hydroxy-2-hydroxymethylprop-1-oxy)-6-methoxypurin,
2,6-Diamino-9-(3-hydroxy-2-hydroxymethylprop-1-oxy)purin,
(S)-9-(1,4-Diacetoxybut-2-oxy)guanin,

2-Amino-9-(2,3-dihydroxyprop-1-oxy)-6-methoxypurin,
(R)-9-(2,3-Dihydroxyprop-1-oxy)guanin,
9-(3,4-Dihydroxybut-1-oxy)guanin,
2-Amino-9-(3,4-dihydroxybut-1-oxy)purin,
(R)-2-Amino-9-(2,3-dihydroxyprop-1-oxy)purin oder
(S)-9-(2,3-Dihydroxyprop-1-oxy)guanin.

**11.** 9- (3-Hydroxyprop-1-oxy)guanin,
9-(3-Acetoxyprop-1-oxy)guanin,
9-(3-Benzoyloxyprop-1-oxy)guanin,
2-Amino-6-ethoxy-9-(3-hydroxyprop-1-oxy)purin,
2-Amino-9-(3-hydroxyprop-1-oxy)purin,
9-(3-Hexanoyloxyprop-1-oxy)guanin,
9-(3-Acetoxyprop-1-oxy)-2-aminopurin,
2-Amino-9-(3-hexanoyloxyprop-1-oxy)purin oder
2-Amino-9-(3-benzoyloxyprop-1-oxy)purin.

**12.** Verfahren zur Herstellung einer Verbindung der Formel (I) nach Anspruch 1 oder eines pharmazeutisch verträglichen Salzes davon, wobei das Verfahren umfaßt: Ringschluß einer Verbindung der Formel (II):

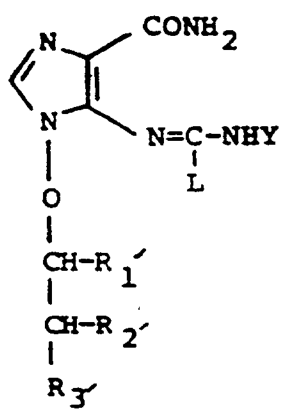

(II)

worin L eine Schwefel-Abgangsgruppe oder die $NH_2$-Gruppe ist, Y eine Schutzgruppe ist und $R_1'$, $R_2'$ und $R_3'$ jeweils $R_1$, $R_2$ und $R_3$ sind, oder $R_1$, $R_2$ und/oder $R_3$ sind, worin die OH-Gruppen geschützt sind; und anschließend, wenn $R_4$ in der gewünschten Verbindung der Formel (I) von einer Hydroxylgruppe verschieden ist, Überführen der Hydroxylgruppe $R_4$ in ein Chloratom, anschließend entweder (i) Reduktion zu $R_4$ als Wasserstoffatom, (ii) Austausch des Chloratoms durch die $NH_2$-Gruppe, wodurch $R_4$ als Aminogruppe erhalten wird, oder (iii) Umsetzen mit einem $OR_5^-$-Ion, wodurch $R_4$ als ein $OR_5$-Rest erhalten wird; und Entfernen der Schutzgruppe Y und Überführen von $R_1'$ in $R_1$ $R_2'$ in $R_2$ und $R_3'$ in $R_3$, wenn erwünscht oder erforderlich, und gegebenenfalls Herstellen des pharmazeutisch verträglichen Salzes oder des O-Acyl-, Phosphat-, cyclischen Acetal- oder cyclischen Carbonat-Derivats davon.

**13.** Verfahren zur Herstellung einer Verbindung der Formel (I) nach Anspruch 1 oder eines pharmazeutisch verträglichen Salzes davon, umfassend das Überführen des Chloratoms in der Verbindung der Formel (VI):

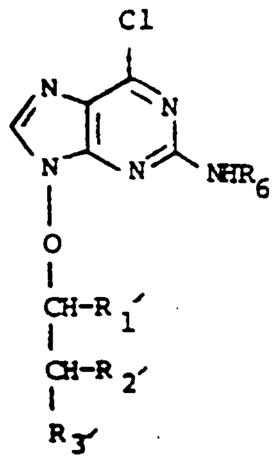

(VI)

worin $R_6$ ein Wasserstoffatom oder eine N-Schutzgruppe ist, und $R_1'$, $R_2'$ und $R_3'$ wie in Anspruch 12 definiert sind, entweder durch (i) Hydrolyse, wodurch $R_4$ als Hydroxylgruppe erhalten wird, (ii) Reduktion, wodurch eine Verbindung der Formel (I) erhalten wird, worin $R_4$ ein Wasserstoffatom ist, (iii) Austausch des Chloratoms durch die $NH_2$-Gruppe, wodurch $R_4$ als Aminogruppe erhalten wird, oder (iv) Umsetzen mit $OR_5^-$ , wodurch eine Verbindung der Formel (I) erhalten wird, worin $R_4$ ein $OR_5$-Rest ist, und anschließendes Überführen von $R_1'$ in $R_1$, $R_2'$ in $R_2$ und/oder $R_3'$ in $R_3$, falls erforderlich und/oder gegebenenfalls Herstellen eines pharmazeutisch verträglichen Salzes oder Derivats davon nach Anspruch 1.

**14.** Verfahren zur Herstellung einer Verbindung der Formel (I) nach Anspruch 1, umfassend die Entfernung der Schutzgruppe einer Verbindung der Formel (I) nach Anspruch 1, wobei jede OH-Gruppe in $R_1$, $R_2$ und/oder $R_3$ in geschützter Form vorliegt; und anschließend gegebenenfalls die Herstellung eines pharmazeutisch verträglichen Salzes oder Derivats davon wie in Anspruch 1 definiert.

**15.** Verbindung der Formel (II) nach Anspruch 12.

**16.** Verbindung der Formel (V):

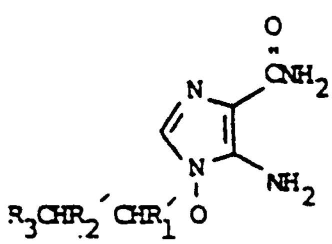

(V)

worin R1', R2' und R3' wie in Anspruch 12 definiert sind.

**17.** Verbindung der Formel (VI) nach Anspruch 13.

**18.** Verbindung der Formel (VIII):

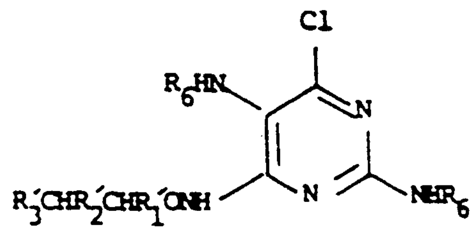

worin $R_6$ eine Formylgruppe ist und R1', R2', und R3' wie in Anspruch 12 definiert sind.

**19.** Verbindung der Formel (IV):

$R_3$'$CHR_2$'$CHR_1$'$ONH_2$ (IV)

wobei $R_1$', $R_2$' und $R_3$' wie in Anspruch 12 definiert und von Verbindungen verschieden sind, worin $R_2$' und $R_3$' geschützte OH-Gruppen sind, wobei die OH-Gruppen an benachbarten Kohlenstoffatomen durch eine Bindung ersetzt sind.

**20.** Verbindung der Formel (I) nach Anspruch 1, außer daß $R_1$, $R_2$ und/oder $R_3$ in geschützter Form vorliegen, wie für $R_1$', $R_2$' und/oder $R_3$' in Anspruch 12 definiert.

**21.** Zwischenverbindung der Formel (II), (V), (VI), (VIII) oder (IV) nach Anspruch 14 bis 18 oder Zwischenverbindung nach Anspruch 19 zur Herstellung einer Verbindung nach Anspruch 10.

**22.** Arzneimittel, umfassend eine Verbindung nach einem der Ansprüche 1 bis 10 oder ein pharmazeutisch verträgliches Salz davon und einen pharmazeutisch verträglichen Träger.

**23.** Verbindung nach einem der Ansprüche 1 bis 10 oder ein pharmazeutisch verträgliches Salzes davon zur Verwendung als therapeutisch wirksame Substanz.

**24.** Verbindung nach einem der Ansprüche 1 bis 10 oder pharmazeutisch verträgliches Salz davon zur Verwendung bei der Behandlung von Virusinfektionen.

**25.** Verwendung einer Verbindung nach einem der Ansprüche 1 bis 10 oder eines pharmazeutisch verträglichen Salzes davon zur Herstellung eines Medikaments zur Behandlung von Virusinfektionen.

**26.** Verwendung einer Verbindung nach Anspruch 11 oder eines pharmazeutisch verträglichen Salzes davon zur Herstellung eines Medikamentes zur Behandlung von Herpesvirusinfektionen.

**Patentansprüche für folgende Vertragsstaaten : AT, ES**

**1.** Verfahren zur Herstellung einer Verbindung der Formel (I) oder eines pharmazeutisch verträglichen Salzes davon:

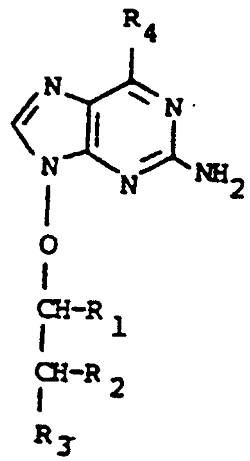

(I)

worin:
$R_1$ ein Wasserstoffatom oder die $CH_2OH$-Gruppe ist;
$R_2$ ein Wasserstoffatom oder (wenn $R_1$ ein Wasserstoffatom ist) eine Hydroxylgruppe oder die $CH_2OH$-Gruppe ist;
$R_3$ die $CH_2OH$-Gruppe oder (wenn $R_1$ und $R_2$ beide ein Wasserstoffatom sind) die $CH(OH)CH_2OH$-Gruppe ist;
$R_4$ ein Wasserstoffatom, eine Hydroxyl-, Aminogruppe oder ein $OR_5$-Rest ist, wobei
$R_5$ ein $C_{1-6}$-Alkylrest, eine Phenylgruppe oder ein Phenyl-$C_{1-2}$-alkyl-Rest ist, wobei jede der Phenyleinheiten durch ein oder zwei Halogenatomen, $C_{1-4}$-Alkyl- oder $C_{1-4}$-Alkoxyresten substituiert sein kann;
und wobei jede der OH-Gruppen in $R_1$, $R_2$ und/oder $R_3$ in Form des O-Acyl-, Phosphat-, cyclischen Acetal- oder cyclischen Phosphat-Derivats davon vorliegen kann, wobei O-Acyl-Derivate jene sind, bei denen ein oder zwei OH-Gruppen in $R_1$, $R_2$ und/oder $R_3$ Carboxylesterreste bilden, die $C_{1-7}$-Alkanoylreste und Benzoylgruppen sind, die gegebenenfalls mit ein oder zwei $C_{1-4}$-Alkyl-, $C_{1-4}$-Alkoxyresten, Halogenatomen oder $CF_3$-Gruppen substituiert sind;
die Phosphat-Derivate jene sind, bei denen eine der acyclischen OH-Gruppen durch $(HO)_2$-$PO_2$ ersetzt ist, oder wobei zwei OH-Gruppen auf benachbarten Kohlenstoffatomen durch eine überbrückende -O-P-(OH)$O_2$-Gruppe ersetzt sind;
das cyclische Acetal-Derivat ein Derivat ist, bei dem zwei OH-Gruppen in $R_1$, $R_2$ und $R_3$ ein -O-C-($C_{1-3}$-Alkyl)$_2$-O-Rest ist; und wobei das cyclische Carbonat-Derivat -O-CO-O-ist;
wobei das Verfahren umfaßt:
Ringschluß einer Verbindung der Formel (II):

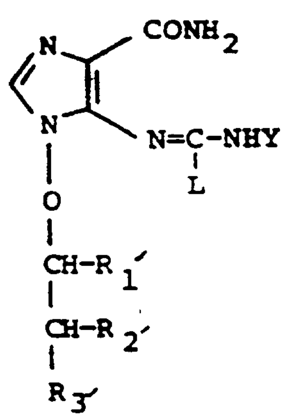

(II)

worin L eine Schwefel-Abgangsgruppe oder die $NH_2$-Gruppe ist, Y eine Schutzgruppe ist und $R_1'$, $R_2'$ und $R_3'$ jeweils $R_1$, $R_2$ und $R_3$ sind, oder $R_1$, $R_2$ und/oder $R_3$ sind, worin die OH-Gruppen geschützt sind; und anschließend, wenn $R_4$ in der gewünschten Verbindung der Formel (I) von einer Hydroxylgruppe verschieden ist, Überführen der Hydroxylgruppe $R_4$ in ein Chloratom, anschließend entweder (i) Reduktion zu $R_4$ als Wasserstoffatom, (ii) Austausch des Chloratoms durch die $NH_2$-Gruppe, wodurch $R_4$ als Aminogruppe erhalten wird, oder (iii) Umsetzen mit einem $OR_5^-$-Ion, wodurch $R_4$ als ein $OR_5$-Rest erhalten wird; und Entfernen der Schutzgruppe Y und Überführen von $R_1'$ in $R_1$, $R_2'$ in $R_2$ und $R_3'$ in $R_3$, wenn erwünscht oder erforderlich, und gegebenenfalls Herstellen des pharmazeutisch verträglichen Salzes oder des O-Acyl-, Phosphat-, cyclischen Acetal- oder cyclischen Carbonat-Derivats davon.

**2.** Verfahren zur Herstellung einer Verbindung der Formel (I) nach Anspruch 1 oder ein pharmazeutisch verträglichen Salzes davon, umfassend das Überführen des Chloratoms in einer Verbindung der Formel (VI):

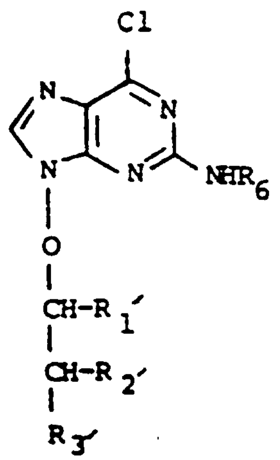

(VI)

worin $R_6$ ein Wasserstoffatom oder eine N-Schutzgruppe ist, und $R_1'$ $R_2'$ und $R_3'$ wie in Anspruch 1 definiert sind, entweder durch (i) Hydrolyse, wodurch $R_4$ als Hydroxylgruppe erhalten wird, (ii) Reduktion, wodurch eine Verbindung der Formel (I) erhalten wird, worin $R_4$ ein Wasserstoffatom ist, (iii) Austausch des Chloratoms durch die $NH_2$-Gruppe, wodurch $R_4$ als Aminogruppe erhalten wird, oder (iv) Umsetzen mit $OR_5^-$, wodurch eine Verbindung der Formel (I) erhalten wird, worin $R_4$ ein $OR_5$-Rest ist, und anschließendes Überführen von $R_1'$ in $R_1$, $R_2'$ in $R_2$ und/oder $R_3'$ in $R_3$, falls erfordelich und/oder gegebenenfalls Herstellen eines pharmazeutisch verträglichen Salzes oder Derivats davon nach Anspruch 1.

**3.** Verfahren zur Herstellung einer Verbindung der Formel (I) nach Anspruch 1, umfassend die Entfernung der Schutzgruppe einer Verbindung der Formel (I) nach Anspruch 1, wobei jede OH-Gruppe in $R_1$, $R_2$ und/oder $R_3$ in geschützter Form vorliegt; und anschließend gegebenenfalls die Herstellung eines pharmazeutisch verträglichen Salzes oder Derivats davon wie in Anspruch 1 definiert.

**4.** Verfahren nach einem der Ansprüche 1 bis 3 zur Herstellung einer Verbindung der Formel (I) nach Anspruch 1, worin $R_1$ und $R_2$ beide ein Wasserstoffatom sind und $R_3$ die $CH_2OH$-Gruppe ist, und eines Derivats davon nach Anspruch 1.

**5.** Verfahren nach einem der Ansprüche 1 bis 3 zur Herstellung einer Verbindung der Formel (I) nach Anspruch 1, wobei $R_1$ ein Wasserstoffatom ist und $R_2$ und $R_3$ beide die $CH_2OH$-Gruppe sind, und eines Derivats davon nach Anspruch 1.

**6.** Verfahren nach einem der Ansprüche 1 bis 3 zur Herstellung einer Verbindung der Formel (I) nach Anspruch 1, wobei $R_1$ ein Wasserstoffatom ist, $R_2$ eine Hydroxylgruppe ist und $R_3$ die $CH_2OH$-Gruppe ist, und eines Derivats davon nach Anspruch 1.

**7.** Verfahren nach einem der Ansprüche 1 bis 3 zur Herstellung einer Verbindung der Formel (I) nach Anspruch 1, wobei $R_1$ die $CH_2OH$-Gruppe ist, $R_2$ ein Wasserstoffatom ist und $R_3$ die $CH_2OH$-Gruppe ist, und eines Derivats davon nach Anspruch 1.

8. Verfahren nach einem der Ansprüche 1 bis 3 zur Herstellung einer Verbindung der Formel (I) nach Anspruch 1, wobei $R_1$ und $R_2$ beide ein Wasserstoffatom sind und $R_3$ die $CH(OH)CH_2OH$-Gruppe ist, und eines Derivats davon nach Anspruch 1.

9. Verfahren nach einem der Ansprüche 1 bis 3 zur Herstellung einer Verbindung der Formel (I) nach einem der Ansprüche 1 und 4 bis 8, wobei $R_4$ eine Hydroxylgruppe ist.

10. Verfahren nach einem der Ansprüche 1 bis 3 zur Herstellung einer Verbindung der Formel (I) nach einem der Ansprüche 1 und 4 bis 8, wobei $R_4$ ein Wasserstoffatom ist.

11. Verfahren nach einem der Ansprüche 1 bis 3 zur Herstellung einer Verbindung der Formel (I) nach einem der Ansprüche 4 bis 10, wobei die OH-Gruppen in $R_1$, $R_2$ und/oder $R_3$ in Form eines Acyl-Derivats davon vorliegen, wobei das (die) Acyl-Derivat(e) ein Acetat-, Hexanoat- oder Benzoat-Derivat ist (sind).

12. Verfahren nach einem der Ansprüche 1 bis 3 zur Herstellung einer Verbindung der Formel (I), welche ist:
9-(3-Hydroxy-2-hydroxymethylprop-1-oxy)guanin,
9-(2,3-Dihydroxyprop-1-oxy)guanin,
2-Amino-9-(2,3-dihydroxyprop-1-oxy)purin,
9-(1,4-Dihydroxybut-2-oxy)guanin,
2-Amino-9-(1,4-dihydroxybut-2-oxy)purin,
2-Amino-9-(1,4-diacetoxybut-2-oxy)purin,
2-Amino-9-(1,4-dibutyryloxybut-2-oxy)purin,
(R)-9-(1,4-Dihydroxybut-2-oxy)guanin,
(S)-9-(1,4-Dihydroxybut-2-oxy)guanin,
2-Amino-9-(3-hydroxy-2-hydroxymethylprop-1-oxy)purin,
9-(3-Acetoxy-2-acetoxymethylprop-1-oxy)-2-aminopurin,
2-Amino-9-(3-propionyloxy-2-propionyloxymethylprop-1-oxy)purin,
2-Amino- (3-benzoyloxy-2-benzoyloxymethylprop-1-oxy)purin,
2-Amino-9-(3-hydroxy-2-hydroxymethylprop-1-oxy)-6-methoxypurin,
2,6-Diamino-9-(3-hydroxy-2-hydroxymethylprop-1-oxy)purin,
(S)-9-(1,4-Diacetoxybut-2-oxy)guanin,
2-Amino-9-(2,3-dihydroxyprop-1-oxy)-6-methoxypurin,
(R)-9-(2,3-Dihydroxyprop-1-oxy)guanin,
9-(3,4-Dihydroxybut-1-oxy)guanin,
2-Amino-9-(3,4-dihydroxybut-1-oxy)purin,
(R)-2-Amino-9-(2,3-dihydroxyprop-1-oxy)purin oder
(S)-9-(2,3-Dihydroxyprop-1-oxy)guanin.

13. Verfahren nach einem der Ansprüche 1 bis 3 zur Herstellung einer Verbindung der Formel (I), welche ist:
9- (3-Hydroxyprop-1-oxy)guanin,
9-(3-Acetoxyprop-1-oxy)guanin,
9-(3-Benzoyloxyprop-1-oxy)guanin,
2-Amino-6-ethoxy-9-(3-hydroxyprop-1-oxy)purin,
2-Amino-9-(3-hydroxyprop-1-oxy)purin,
9-(3-Hexanoyloxyprop-1-oxy)guanin,
9-(3-Acetoxyprop-1-oxy)-2-aminopurin,
2-Amino-9-(3-hexanoyloxyprop-1-oxy)purin oder
2-Amino-9-(3-benzoyloxyprop-1-oxy)purin.

14. Verwendung einer Verbindung der Formel (I) nach einem der Ansprüche 1 und 4 bis 12 oder eines pharmazeutisch verträglichen Salzes davon zur Herstellung eines Medikaments zur Behandlung von Virusinfektionen.

15. Verwendung einer Verbindung der Formel (I) nach Anspruch 13 oder eines pharmazeutisch verträglichen Salzes davon zur Herstellung eines Medikamentes zur Behandlung von Herpesvirusinfektionen.

**Patentansprüche für folgenden Vertragsstaat : GR**

1. Verbindung der Formel (I) oder ein pharmazeutisch verträgliches Salz davon:

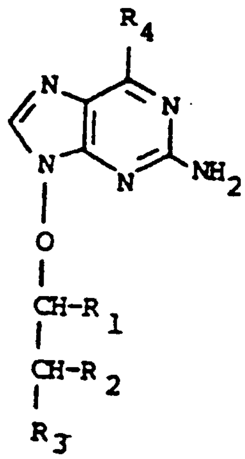

(I)

worin:
$R_1$ ein Wasserstoffatom oder die $CH_2OH$-Gruppe ist;
$R_2$ ein Wasserstoffatom oder (wenn $R_1$ ein Wasserstoffatom ist) eine Hydroxylgruppe oder die $CH_2OH$-Gruppe ist;
$R_3$ die $CH_2OH$-Gruppe oder (wenn $R_1$ und $R_2$ beide ein Wasserstoffatom sind) die $CH(OH)CH_2OH$-Gruppe ist;
$R_4$ ein Wasserstoffatom, eine Hydroxyl-, Aminogruppe oder ein $OR_5$-Rest ist, wobei
$R_5$ ein $C_{1-6}$-Alkylrest, eine Phenylgruppe oder ein Phenyl-$C_{1-2}$-alkyl-Rest ist, wobei jede der Phenyleinheiten durch ein oder zwei Halogenatomen, $C_{1-4}$-Alkyl- oder $C_{1-4}$ Alkoxyresten substituiert sein kann;
und wobei jede der OH-Gruppen in $R_1$, $R_2$ und/oder $R_3$ in Form des O-Acyl-, Phosphat-, cyclischen Acetal- oder cyclischen Carbonat-Derivats davon vorliegen kann, wobei O-Acyl-Derivate jene sind, bei denen ein oder zwei OH-Gruppen in $R_1$, $R_2$ und/oder $R_3$ Carboxylesterreste bilden, die $C_{1-7}$-Alkanoylreste und Benzoylgruppen sind, die gegebenenfalls mit ein oder zwei $C_{1-4}$-Alkyl-, $C_{1-4}$-Alkoxyresten, Halogenatomen oder $CF_3$-Gruppen substituiert sind;
die Phosphat-Derivate jene sind, bei denen eine der acyclischen OH-Gruppen durch $(HO)_2$-$PO_2$ ersetzt ist, oder wobei zwei OH-Gruppen auf benachbarten Kohlenstoffatomen durch eine überbrückende -O-P-$(OH)O_2$-Gruppe ersetzt sind;
das cyclische Acetal-Derivat ein Derivat ist, bei dem zwei OH-Gruppen in $R_1$, $R_2$ und $R_3$ ein -O-C-$(C_{1-3}$-Alkyl$)_2$-O-Rest ist; und wobei das cyclische Carbonat-Derivat -O-CO-O-ist.

2. Verbindung der Formel (I) nach Anspruch 1, wobei $R_1$ und $R_2$ beide ein Wasserstoffatom sind und $R_3$ die $CH_2OH$-Gruppe ist, und Derivate davon nach Anspruch 1.

3. Verbindung der Formel (I) nach Anspruch 1, wobei $R_1$ ein Wasserstoffatom ist und $R_2$ und $R_3$ beide die $CH_2OH$-Gruppe sind, und Derivate davon nach Anspruch 1.

4. Verbindung der Formel (I) nach Anspruch 1, wobei $R_1$ ein Wasserstoffatom ist, $R_2$ eine Hydroxylgruppe ist und $R_3$ die $CH_2OH$-Gruppe ist, und Derivate davon nach Anspruch 1.

5. Verbindung der Formel (I) nach Anspruch 1, wobei $R_1$ die $CH_2OH$-Gruppe ist, $R_2$ ein Wasserstoffatom ist und $R_3$ die $CH_2OH$-Gruppe ist, und Derivate davon nach Anspruch 1.

6. Verbindung der Formel (I) nach Anspruch 1, wobei $R_1$ und $R_2$ beide ein Wasserstoffatom sind und $R_3$ die $CH(OH)CH_2OH$-Gruppe ist, und Derivate davon nach Anspruch 1.

7. Verbindung nach einem der Ansprüche 1 bis 6, wobei $R_4$ eine Hydroxylgruppe ist.

8. Verbindung nach einem der Ansprüche 1 bis 6, wobei $R_4$ ein Wasserstoffatom ist.

9. Verbindung nach einem der Ansprüche 1 bis 8, wobei die OH-Gruppen in $R_1$, $R_2$ und/oder $R_3$ in Form eines Acyl-Derivats davon vorliegen, wobei das (die) Acyl-Derivat(e) Acetat, Hexanoat oder Benzoat ist (sind).

10. 9-(3-Hydroxy-2-hydroxymethylprop-1-oxy)guanin,
9-(2,3-Dihydroxyprop-1-oxy)guanin,
2-Amino-9-(2,3-dihydroxyprop-1-oxy)purin,
9-(1,4-Dihydroxybut-2-oxy)guanin,
2-Amino-9-(1,4-dihydroxybut-2-oxy)purin,
2-Amino-9-(1,4-diacetoxybut-2-oxy)purin,
2-Amino-9-(1,4-dibutyryloxybut-2-oxy)purin,
(R)-9-(1,4-Dihydroxybut-2-oxy)guanin,
(S)-9-(1,4-Dihydroxybut-2-oxy)guanin,
2-Amino-9-(3-hydroxy-2-hydroxymethylprop-1-oxy)purin,
9-(3-Acetoxy-2-acetoxymethylprop-1-oxy)-2-aminopurin,
2-Amino-9-(3-propionyloxy-2-propionyloxymethylprop-1-oxy)purin,
2-Amino-9-(3-benzoyloxy-2-benzoyloxymethylprop-1-oxy)purin,
2-Amino-9-(3-hydroxy-2-hydroxymethylprop-1-oxy)-6-methoxypurin,
2,6-Diamino-9-(3-hydroxy-2-hydroxymethylprop-1-oxy)purin,
(S)-9-(1,4-Diacetoxybut-2-oxy)guanin,
2-Amino-9-(2,3-dihydroxyprop-1-oxy)-6-methoxypurin,
(R)-9-(2,3-Dihydroxyprop-1-oxy)guanin,
9-(3,4-Dihydroxybut-1-oxy)guanin,
2-Amino-9-(3,4-dihydroxybut-1-oxy)purin,
(R)-2-Amino-9-(2,3-dihydroxyprop-1-oxy)purin oder
(S)-9-(2,3-Dihydroxyprop-1-oxy)guanin.

11. 9- (3-Hydroxyprop-1-oxy)guanin,
9-(3-Acetoxyprop-1-oxy)guanin,
9-(3-Benzoyloxyprop-1-oxy)guanin,
2-Amino-6-ethoxy-9-(3-hydroxyprop-1-oxy)purin,
2-Amino-9-(3-hydroxyprop-1-oxy)purin,
9-(3-Hexanoyloxyprop-1-oxy)guanin,
9-(3-Acetoxyprop-1-oxy)-2-aminopurin,
2-Amino-9-(3-hexanoyloxyprop-1-oxy)purin oder
2-Amino-9-(3-benzoyloxyprop-1-oxy)purin.

12. Verfahren zur Herstellung einer Verbindung der Formel (I) nach Anspruch 1 oder eines pharmazeutisch verträglichen Salzes davon, wobei das Verfahren umfaßt:
Ringschluß einer Verbindung der Formel (II):

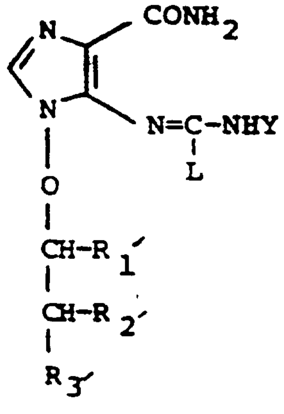

(II)

worin L eine Schwefel-Abgangsgruppe oder die $NH_2$-Gruppe ist, Y eine Schutzgruppe ist und $R_1'$, $R_2'$ und $R_3'$ jeweils $R_1$, $R_2$ und $R_3$ sind, oder $R_1$, $R_2$ und/oder $R_3$ sind, worin die OH-Gruppen geschützt sind; und anschließend, wenn $R_4$ in der gewünschten Verbindung der Formel (I) von einer Hydroxylgruppe verschieden ist, Überführen der Hydroxylgruppe $R_4$ in ein Chloratom, anschließend entweder (i) Reduktion zu $R_4$ als Wasserstoffatom, (ii) Austausch des Chloratoms durch die $NH_2$-Gruppe, wodurch $R_4$ als Aminogruppe erhalten wird, oder (iii) Umsetzen mit einem $OR_5^-$-Ion, wodurch $R_4$ als ein $OR_5$-Rest erhalten wird; und Entfernen der Schutzgruppe Y und Überführen von $R_1'$ in $R_1$, $R_2'$ in $R_2$ und $R_3'$ in $R_3$, wenn erwünscht oder erforderlich, und gegebenenfalls Herstellen des pharmazeutisch verträglichen Salzes oder des O-Acyl-, Phosphat-, cyclischen Acetal- oder cyclischen Carbonat-Derivats davon.

**13.** Verfahren zur Herstellung einer Verbindung der Formel (I) nach Anspruch 1 oder eines pharmazeutisch verträglichen Salzes davon, umfassend das Überführen des Chloratoms in der Verbindung der Formel (VI):

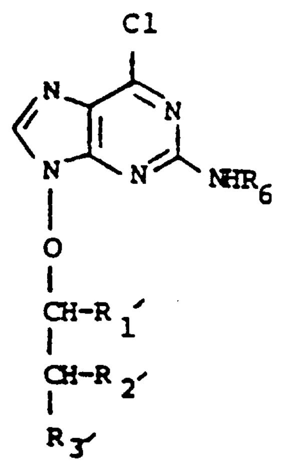

(VI)

worin $R_6$ ein Wasserstoffatom oder eine N-Schutzgruppe ist, und $R_1'$, $R_2'$ und $R_3'$ wie in Anspruch 12 definiert sind, entweder durch (i) Hydrolyse, wodurch $R_4$ als Hydroxylgruppe erhalten wird, (ii) Reduktion, wodurch eine Verbindung der Formel (I) erhalten wird, worin $R_4$ ein Wasserstoffatom ist, (iii) Austausch des Chloratoms durch die $NH_2$-Gruppe, wodurch $R_4$ als Aminogruppe erhalten wird, oder (iv) Umsetzen mit $OR_5^-$, wodurch eine Verbindung der Formel (I) erhalten wird, worin $R_4$ ein $OR_5$-Rest ist, und anschließendes Überführen von $R_1'$ in $R_1$, $R_2'$ in $R_2$ und/oder $R_3'$ in $R_3$, falls erforderlich und/oder gegebenenfalls Herstellen eines pharmazeutisch verträglichen Salzes oder Derivats davon nach Anspruch 1.

**14.** Verfahren zur Herstellung einer Verbindung der Formel (I) nach Anspruch 1, umfassend die Entfernung der Schutzgruppe einer Verbindung der Formel (I) nach Anspruch 1, wobei jede OH-Gruppe in $R_1$, $R_2$ und/oder $R_3$ in geschützter Form vorliegt; und anschließend gegebenenfalls die Herstellung eines pharmazeutisch verträglichen Salzes oder Derivats davon wie in Anspruch 1 definiert.

**15.** Verbindung der Formel (II) nach Anspruch 12.

**16.** Verbindung der Formel (V):

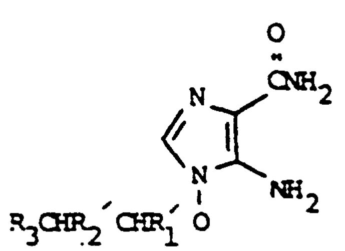

(V)

worin R1', R2' und R3' wie in Anspruch 12 definiert sind.

**17.** Verbindung der Formel (VI) nach Anspruch 13.

**18.** Verbindung der Formel (VIII):

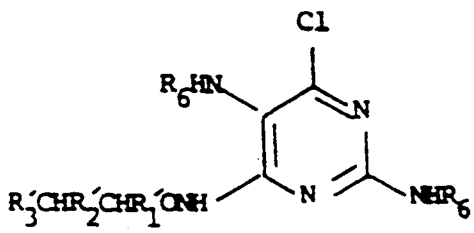

worin $R_6$ eine Formylgruppe ist und R1', R2', und R3' wie in Anspruch 12 definiert sind.

**19.** Verbindung der Formel (IV):

$R_3$'$CHR_2$'$CHR_1$'$ONH_2$ (IV)

wobei $R_1$', $R_2$' und $R_3$' wie in Anspruch 12 definiert und von Verbindungen verschieden sind, worin $R_2$' und $R_3$' geschützte OH-Gruppen sind, wobei die OH-Gruppen an benachbarten Kohlenstoffatomen durch eine Bindung ersetzt sind.

**20.** Verbindung der Formel (I) nach Anspruch 1, außer daß $R_1$, $R_2$ und/oder $R_3$ in geschützter Form vorliegen, wie für $R_1$', $R_2$' und/oder $R_3$' in Anspruch 12 definiert.

**21.** Zwischenverbindung der Formel (II), (V), (VI), (VIII) oder (IV) nach Anspruch 14 bis 18 oder Zwischenverbindung nach Anspruch 19 zur Herstellung einer Verbindung nach Anspruch 10.

**22.** Verwendung einer Verbindung nach einem der Ansprüche 1 bis 10 oder eines pharmazeutisch verträglichen Salzes davon zur Herstellung eines Medikaments zur Behandlung von Virusinfektionen.

**23.** Verwendung einer Verbindung nach Anspruch 11 oder eines pharmazeutisch verträglichen Salzes davon zur Herstellung eines Medikamentes zur Behandlung von Herpesvirusinfektionen.

**Revendications**

**Revendications pour les Etats contractants suivants : BE, CH, DE, FR, GB, IT, LI, LU, NL, SE**

**1.** Composé de formule (I), ou un de ses sels pharmaceutiquement acceptables :

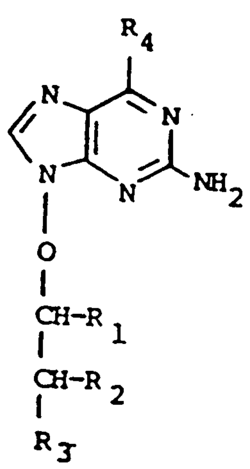

(I)

formule dans laquelle :

$R_1$ représente l'hydrogène ou un groupe $CH_2OH$ ;

$R_2$ représente l'hydrogène ou, (lorsque $R_1$ représente l'hydrogène), un groupe hydroxy ou $CH_2OH$ ;

$R_3$ représente un groupe $CH_2OH$ ou, (lorsque $R_1$ et $R_2$ représentent l'un et l'autre l'hydrogène), un groupe $CH(OH)CH_2OH$ ;

$R_4$ représente l'hydrogène, un groupe hydroxy, amino ou $OR_5$ dans lequel $R_5$ représente un groupe alkyle en $C_1$ à $C_6$, phényle ou phényl-(alkyle en $C_1$ ou $C_2$), chacun des groupements phényle pouvant être substitué avec un ou deux groupes halogéno, alkyle en $C_1$ à $C_4$ ou alkoxy en $C_1$ à $C_4$ ;

et dans laquelle n'importe quels groupes OH dans $R_1$, $R_2$ et/ou $R_3$ peuvent être sous forme de leurs derivés O-acylés, phosphate, acétal cyclique ou carbonate cyclique, les dérives O-acylés étant ceux dans lesquels un ou deux groupes OH dans $R_1$, $R_2$ et/ou $R_3$ forment des groupes ester carboxylique qui sont des groupes alcanoyle en $C_1$ à $C_7$ et benzoyle portant facultativement comme substituants un ou deux groupes alkyle en $C_1$ à $C_4$, alkoxy en $C_1$ à $C_4$, halogéno ou $CF_3$ ; les dérivés du type phosphate étant ceux dans lesquels un des groupes OH acycliques est remplacé par un groupe$(HO)_2$-$PO_2$ ou dans lesquels deux groupes OH sur des atomes de carbone adjacents sont remplacés parun groupe -O-P(OH)$O_2$- de pontage ; un dérivé du type acétal cyclique étant un dérivé dans lequel deux groupes OH dans $R_1$, $R_2$ et $R_3$ sont remplacés par un groupe -O-C(alkyle en $C_1$ à $C_3$)$_2$-O ; et un carbonate cyclique étant un groupe -O-CO-O-.

**2.** Composé de formule (I) suivant la revendication 1, dans lequel $R_1$ et $R_2$ représentent l'un et l'autre l'hydrogène et $R_3$ représente un groupe $CH_2OH$, et ses dérivés répondant à la définition suivant la revendication 1.

**3.** Composé de formule (I) suivant la revendication 1, dans lequel $R_1$ représente l'hydrogène et $R_2$ et $R_3$ représentent l'un et l'autre un groupe $CH_2OH$, et ses dérivés répondant à la définition suivant la revendication 1.

**4.** Composé de formule (I) suivant la revendication 1, dans lequel $R_1$ représente l'hydrogène, $R_2$ représente un groupe hydroxy et $R_3$ représente un groupe $CH_2OH$, et ses dérivés répondant à la définition suivant la revendication 1.

**5.** Composé de formule (I) suivant la revendication 1, dans lequel $R_1$ représente un groupe $CH_2OH$, $R_2$ représente l'hydrogène et $R_3$ représente un groupe $CH_2OH$, et ses dérivés répondant à la définition suivant la revendication 1.

**6.** Composé de formule (I) suivant la revendication 1, dans lequel $R_1$ et $R_2$ représentent l'un et l'autre l'hydrogène et $R_3$ représente un groupe $CH(OH)CH_2OH$, et ses dérivés répondant à la définition suivant la revendication 1.

**7.** Composé suivant l'une quelconque des revendications 1 à 6, dans lequel $R_4$ représente un groupe hydroxy.

**8.** Composé suivant l'une quelconque des revendications 1 à 6, dans lequel $R_4$ représente l'hydrogène.

**9.** Composé suivant l'une quelconque des revendications 1 à 8, dans lequel les groupes OH dans $R_1$, $R_2$ et/ou $R_3$ sont sous forme d'un de leurs dérivés acylés, ledit ou lesdits dérivés acylés consistant en acétates, hexanoates ou benzoates.

**10.** 9-(3-hydroxy-2-hydroxyméthylprop-1-oxy)guanine,
9-(2,3-dihydroxyprop-1-oxy)guanine,
2-amino-9-(2,3-dihydroxyprop-1-oxy)purine,
9-(1,4-dihydroxybut-2-oxy)guanine,
2-amino-9-(1,4-dihydroxybut-2-oxy)purine,
2-amino-9-(1,4-diacétoxybut-2-oxy)purine,
2-amino-9-(1,4-dibutyryloxybut-2-oxy)purine,
(R)-9-(1,4-dihydroxybut-2-oxy)guanine,
(S)-9-(1,4-dihydroxybut-2-oxy)guanine,

2-amino-9-(3-hydroxy-2-hydroxyméthylprop-1-oxy)purine,
9-(3-acétoxy-2-acétoxyméthylprop-1-oxy)-2-aminopurine,
2-amino-9-(3-propionyloxy-2-propionyloxyméthylprop-1-oxy)purine,
2-amino-9-(3-benzoyloxy-2-benzoyloxyméthylprop-1-oxy)purine,
2-amino-9-(3-hydroxy-2-hydroxyméthylprop-1-oxy)-6-méthoxypurine,
2,6-diamino-9-(3-hydroxy-2-hydroxyméthylprop-1-oxy)purine,
(S)-9-(1,4-diacétoxybut-2-oxy)guanine,
2-amino-9-(2,3-dihydroxyprop-1-oxy)-6-méthoxypurine,
(R)-9-(2,3-dihydroxyprop-1-oxy)guanine,
9-(3,4-dihydroxybut-1-oxy)guanine,
2-amino-9-(3,4-dihydroxybut-1-oxy)purine,
(R)-2-amino-9-(2,3-dihydroxyprop-1-oxy)purine ou
(S)-9-(2,3-dihydroxyprop-1-oxy)guanine.

**11.** 9-(3-hydroxyprop-1-oxy)guanine,
9-(3-acétoxyprop-1-oxy)guanine,
9-(3-benzoyloxyprop-1-oxy)guanine,
2-amino-6-éthoxy-9-(3-hydroxyprop-1-oxy)purine,
2-amino-9-(3-hydroxyprop-1-oxy)purine,
9-(3-hexanoyloxyprop-1-oxy)guanine,
9-(3-acétoxyprop-1-oxy)-2-aminopurine,
2-amino-9-(3-hexanoyloxyprop-1-oxy)purine ou
2-amino-9-(3-benzoyloxyprop-1-oxy)purine.

**12.** Procédé de préparation d'un composé de formule (I) suivant la revendication 1 ou d'un de ses sels pharmaceutiquement acceptables, procédé qui comprend la cyclisation d'un composé de formule (II) :

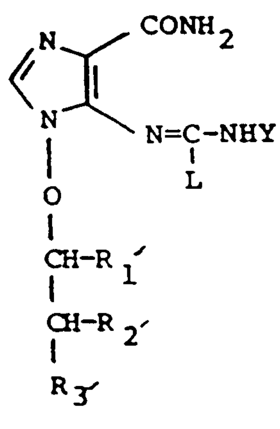

(II)

dans laquelle L représente un groupe partant renfermant un atome de soufre ou un groupe $NH_2$, Y représente un groupe protecteur et $R_1'$, $R_2'$ et $R_3'$ représentent respectivement des groupes $R_1$, $R_2$ et $R_3$ ou des groupes $R_1$, $R_2$ et/ou $R_3$ dans lesquels les groupes OH sont protégés ; et ensuite, lorsque $R_4$ dans le composé désiré de formule (I) est un groupe autre qu'un groupe hydroxy, la transformation d'un groupe hydroxy $R_4$ en un groupe chloro, suivie par (i) la réduction en un groupe $R_4$ consistant en l'hydrogène, (ii) le remplacement du groupe chloro par un groupe $NH_2$, donnant un groupe $R_4$ consistant en un groupe amino ; ou (iii) la réaction avec l'ion $OR_5^-$, donnant un groupe $R_4$ consistant en un groupe $OR_5$ ; et l'élimination du groupe protecteur Y et la transformation de $R_1'$ en $R_1$, $R_2'$ en $R_2$ et $R_3'$ en $R_3$ lorsque cela est désiré ou nécessaire, et, facultativement, la formation d'un de ses sels dérivés O-acylés, phosphate, acétal cyclique ou carbonate cyclique, pharmaceutiquement acceptables.

**13.** Procédé de préparation d'un composé de formule (I) suivant la revendication 1 ou d'un de ses sels pharmaceutiquement acceptables, procédé qui comprend la transformation du groupe chloro dans un composé de formule (VI) :

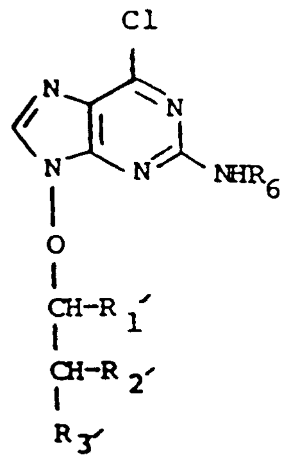

(VI)

dans laquelle $R_6$ représente l'hydrogène ou un groupe protecteur de l'atome d'azote, et $R_1$', $R_2$' et $R_3$' répondent aux définitions mentionnées dans la revendication 12, par (i) une hydrolyse donnant un groupe $R_4$ consistant en un groupe hydroxy, (ii) une réduction donnant un composé de formule (I) dans laquelle $R_4$ représente l'hydrogène, (iii) un remplacement du groupe chloro par un groupe $NH_2$, donnant un groupe $R_4$ consistant en un groupe amino ; ou (iv) une réaction avec l'ion $OR_5^-$, donnant un composé de formule (I) dans laquelle $R_4$ représente un groupe $OR_5$, et ensuite la transformation de $R_1$' en $R_1$, $R_2$' en $R_2$ et/ou $R_3$' en $R_3$ lorsque cela est nécessaire, et/ou facultativement la formation d'un de ses sels pharmaceutiquement acceptables ou d'un de ses dérivés répondant à la définition suivant la revendication 1.

**14.** Procédé de préparation d'un composé de formule (I) suivant la revendication 1, procédé qui comprend l'élimination de la protection d'un composé de formule (I) répondant à la définition suivant la revendication 1, dans laquelle n'importe quels groupes OH dans $R_1$, $R_2$ et/ou $R_3$ sont sous forme protégée ; et ensuite, facultativement, la formation d'un de ses sels pharmaceutiquement acceptables ou d'un de ses dérivés répondant à la définition suivant la revendication 1.

**15.** Composé de formule (II) répondant à la définition suivant la revendication 12.

**16.** Composé de formule (V) :

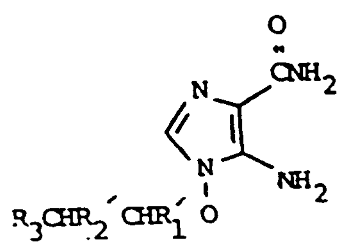

(V)

dans laquelle $R_1$', $R_2$' et $R_3$' répondent aux définitions suivant la revendication 12.

**17.** Composé de formule (VI) répondant à la définition suivant la revendication 13.

**18.** Composé de formule (VIII) :

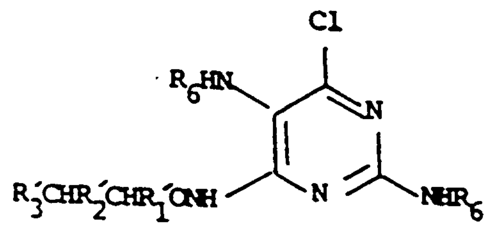

dans laquelle $R_6$ représente un groupe formyle et $R_1'$, $R_2'$ et $R_3'$ répondent aux définitions suivant la revendication 12.

**19.** Composé de formule (IV) :

$R_3'CHR_2'CHR_1'ONH_2$ (IV)

dans laquelle $R_1'$, $R_2'$ et $R_3'$ répondent aux définitions suivant la revendication 12, autre que des composés dans lesquels $R_2'$ et $R_3'$ sont des groupes OH protégés, dont les groupes OH sur des atomes de carbone adjacents sont remplacés par une liaison.

**20.** Composé de formule (I) répondant à la définition suivant la revendication 1, sauf que $R_1'$, $R_2$ et/ou $R_3$ sont sous une forme protégée répondant à la définition pour $R_1'$, $R_2'$ et/ou $R_3'$ dans la revendication 12.

**21.** Composé intermédiaire de formules (II), (V), (VI), (VIII) ou (IV), répondant à la définition suivant les revendications 14 à 18, ou composé intermédiaire suivant la revendication 19, pour la préparation de n'importe quel composé suivant la revendication 10.

**22.** Composition pharmaceutique comprenant un composé suivant l'une quelconque des revendications 1 à 10, ou un de ses sels pharmaceutiquement acceptables, et un support pharmaceutiquement acceptable.

**23.** Composé suivant l'une quelconque des revendications 1 à 10, ou un de ses sels pharmaceutiquement acceptables, destiné à être utilisé comme substance thérapeutique active.

**24.** Composé suivant l'une quelconque des revendications 1 à 10, ou un de ses sels pharmaceutiquement acceptables, destiné à être utilisé dans le traitement d'infections virales.

**25.** Utilisation d'un composé suivant l'une quelconque des revendications 1 à 10, ou d'un de ses sels pharmaceutiquement acceptables, dans la production d'un médicament destiné au traitement d'infections virales.

**26.** Utilisation d'un composé suivant la revendication 11, ou d'un de ses sels pharmaceutiquement acceptables, dans la production d'un médicament destiné au traitement d'infections par virus de l'herpès.

**Revendications pour les Etats contractants suivants : AT, ES**

**1.** Procédé de préparation d'un composé de formule (I), ou d'un de ses sels pharmaceutiquement acceptables :

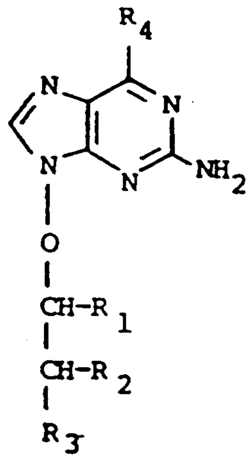

(I)

dans laquelle :

$R_1$ représente l'hydrogène ou un groupe $CH_2OH$ ;

$R_2$ représente l'hydrogène ou, (lorsque $R_1$ représente l'hydrogène), un groupe hydroxy ou $CH_2OH$ ;

$R_3$ représente un groupe $CH_2OH$ ou, (lorsque $R_1$ et $R_2$ représentent l'un et l'autre l'hydrogène), un groupe $CH(OH)CH_2OH$ ;

$R_4$ représente l'hydrogène, un groupe hydroxy, amino ou $OR_5$ dans lequel $R_5$ représente un groupe alkyle en $C_1$ à $C_6$, phényle ou phényl-(alkyle en $C_1$ ou $C_2$), chacun des groupements phényle pouvant être substitué avec un ou deux groupes halogéno, alkyle en $C_1$ à $C_4$ ou alkoxy en $C_1$ à $C_4$ ;

et dans laquelle n'importe quels groupes OH dans $R_1$, $R_2$ et/ou $R_3$ peuvent être sous forme d'un de leurs dérivés O-acylés, phosphate, acétal cyclique ou phosphate cyclique, les dérivés O-acylés étant ceux dans lesquels un ou deux groupes OH dans $R_1$, $R_2$ et/ou $R_3$ forment des groupes ester carboxylique qui sont des groupes alcanoyle en $C_1$ à $C_7$ et benzoyle facultativement substitués avec un ou deux groupes alkyle en $C_1$ à $C_4$, alkoxy en $C_1$ à $C_4$, halogéno ou $CF_3$ ; les dérivés du type phosphate étant ceux dans lesquels un des groupes OH acycliques est remplacé par un groupe $(HO)_2$-$PO_2$ ou dans lesquels deux groupes OH sur des atomes de carbone adjacents sont remplacés par un groupe -O-P(OH)$O_2$- de pontage ; un dérivé du type acétal cyclique étant un dérivé dans lequel deux groupes OH dans $R_1$, $R_2$ et $R_3$ sont remplacés par un groupe -O-C(alkyle en $C_1$ à $C_3$)$_2$-O ; et un carbonate cyclique étant un groupe -O-CO-O- ;

procédé qui comprend la cyclisation d'un composé de formule (II) :

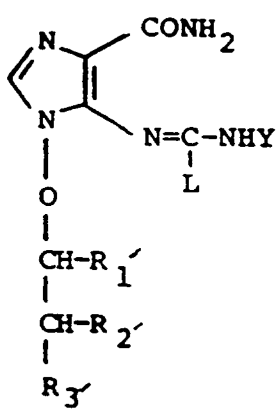

(II)

dans laquelle L représente un groupe partant renfermant un atome de soufre ou un groupe $NH_2$, Y représente un groupe protecteur et $R_1'$, $R_2'$ et $R_3'$ représentent respectivement des groupes $R_1$, $R_2$ et $R_3$ ou des groupes $R_1$, $R_2$ et/ou $R_3$ dans lesquels les groupes OH sont protégés ; et ensuite, lorsque $R_4$ dans le composé désiré de formule (I) est un groupe autre qu'un groupe hydroxy, la transformation

d'un groupe hydroxy $R_4$ en un groupe chloro, suivie par (i) une réduction en un groupe $R_4$ consistant en l'hydrogène, (ii) un remplacement du groupe chloro par un groupe $NH_2$, donnant un groupe $R_4$ consistant en un groupe amino ; ou (iii) une réaction avec l'ion $OR_5^-$, donnant un groupe $R_4$ consistant en un groupe $OR_5$ ; et l'élimination du groupe protecteur Y et la transformation de $R_1'$ en $R_1$, $R_2'$ en $R_2$ et $R_3'$ en $R_3$ lorsque cela est désiré ou nécessaire et, facultativement, la formation d'un de ses sels, dérivés O-acylés, phosphate, acétal cyclique ou carbonate cyclique, pharmaceutiquement acceptables.

2. Procédé de préparation d'un composé de formule (I) répondant à la définition suivant la revendication 1 ou d'un de ses sels pharmaceutiquement acceptables, procédé qui comprend la transformation du groupe chloro dans un composé de formule (VI) :

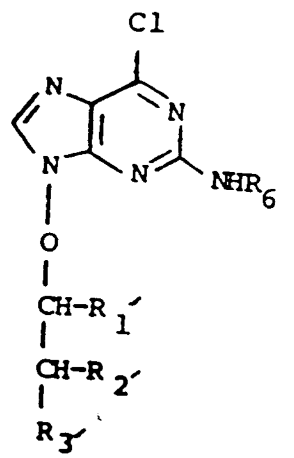

(VI)

dans laquelle $R_6$ représente l'hydrogène ou un groupe protecteur de l'atome d'azote, et $R_1'$, $R_2'$ et $R_3'$ répondent aux définitions suivant la revendication 1, par (i) une hydrolyse donnant un groupe $R_4$ consistant en un groupe hydroxy, (ii) une réduction donnant un composé de formule (I) dans laquelle $R_4$ représente l'hydrogène, (iii) un remplacement du groupe chloro par un groupe $NH_2$, donnant un groupe $R_4$ consistant en un groupe amino ; ou (iv) une réaction avec l'ion $OR_5^-$, donnant un composé de formule (I) dans laquelle $R_4$ représente un groupe $OR_5$ et, ensuite, la transformation de $R_1'$ en $R_1$, $R_2'$ en $R_2$ et/ou $R_3'$ en $R_3$ lorsque cela est nécessaire, et/ou, facultativement, la formation d'un de ses sels pharmaceutiquement acceptables ou d'un de ses dérivés répondant à la définition suivant la revendication 1.

3. Procédé de préparation d'un composé de formule (I) répondant à la définition suivant la revendication 1, procédé qui comprend l'élimination de la protection d'un composé de formule (I) répondant à la définition suivant la revendication 1, dans laquelle n'importe quels groupes OH dans $R_1$, $R_2$ et/ou $R_3$ sont sous forme protégée ; et ensuite, facultativement, la formation d'un de ses sels ou dérivés pharmaceutiquement acceptables répondant à la définition suivant la revendication 1.

4. Procédé suivant l'une quelconque des revendications 1 à 3 pour la préparation d'un composé de formule (I) répondant à la définition suivant la revendication 1, dans laquelle $R_1$ et $R_2$ représentent l'un et l'autre l'hydrogène et $R_3$ représente un groupe $CH_2OH$, et de ses dérivés répondant à la définition suivant la revendication 1.

5. Procédé suivant l'une quelconque des revendications 1 à 3 pour la préparation d'un composé de formule (I) répondant à la définition suivant la revendication 1, dans laquelle $R_1$ représente l'hydrogène et $R_2$ et $R_3$ représentent l'un et l'autre un groupe $CH_2OH$, et de ses dérivés répondant à la définition suivant la revendication 1.

6. Procédé suivant l'une quelconque des revendications 1 à 3 pour la préparation d'un composé de formule (I) répondant à la définition suivant la revendication 1, dans laquelle $R_1$ représente l'hydrogène, $R_2$ représente un groupe hydroxy et $R_3$ représente un groupe $CH_2OH$, et de ses dérivés répondant à la définition suivant la revendication 1.

7. Procédé suivant l'une quelconque des revendications 1 à 3 pour la préparation d'un composé de formule (I) répondant à la définition suivant la revendication 1, dans laquelle $R_1$ représente un groupe $CH_2OH$, $R_2$ représente l'hydrogène et $R_3$ représente un groupe $CH_2OH$, et de ses dérivés répondant à la définition suivant la revendication 1.

8. Procédé suivant l'une quelconque des revendications 1 à 3 pour la préparation d'un composé de formule (I) répondant à la définition suivant la revendication 1, dans laquelle $R_1$ et $R_2$ représentent l'un et l'autre l'hydrogène et $R_3$ représente un groupe $CH(OH)CH_2OH$, et de ses dérivés répondant à la définition suivant la revendication 1.

9. Procédé suivant l'une quelconque des revendications 1 à 3 pour la préparation d'un composé de formule (I) répondant à la définition suivant l'une quelconque des revendications 1 et 4 à 8, dans laquelle $R_4$ représente un groupe hydroxy.

10. Procédé suivant l'une quelconque des revendications 1 à 3 pour la préparation d'un composé de formule (I) répondant à la définition suivant l'une quelconque des revendications 1 et 4 à 8, dans laquelle $R_4$ représente l'hydrogène.

11. Procédé suivant l'une quelconque des revendications 1 à 3 pour la préparation d'un composé de formule (I) répondant à la définition suivant l'une quelconque des revendications 4 à 10, dans laquelle les groupes OH dans $R_1$, $R_2$ et/ou $R_3$ sont sous forme d'un de leurs dérivés acyle, le ou les dérivés acyle étant des groupes acétate, hexanoate ou benzoate.

12. Procédé suivant l'une quelconque des revendications 1 à 3 pour la préparation d'un composé de formule (I) qui est :
    la 9-(3-hydroxy-2-hydroxyméthylprop-1-oxy)guanine,
    la 9-(2,3-dihydroxyprop-1-oxy)guanine,
    la 2-amino-9-(2,3-dihydroxyprop-1-oxy)purine,
    la 9-(1,4-dihydroxybut-2-oxy)guanine,
    la 2-amino-9-(1,4-dihydroxybut-2-oxy)purine,
    la 2-amino-9-(1,4-diacétoxybut-2-oxy)purine,
    la 2-amino-9-(1,4-dibutyryloxybut-2-oxy)purine,
    la (R)-9-(1,4-dihydroxybut-2-oxy)guanine,
    la (S)-9-(1,4-dihydroxybut-2-oxy)guanine,
    la 2-amino-9-(3-hydroxy-2-hydroxyméthylprop-1-oxy)-purine,
    la 9-(3-acétoxy-2-acétoxyméthylprop-1-oxy)-2-amino-purine,
    la 2-amino-9-(3-propionloxy-2-propionloxyméthylprop-1-oxy)purine,
    la 2-amino-9-(3-benzoyloxy-2-benzoyloxyméthylprop-1-oxy)purine,
    la 2-amino-9-(3-hydroxy-2-hydroxyméthylprop-1-oxy)-6-méthoxypurine,
    la 2,6-diamino-9-(3-hydroxy-2-hydroxyméthylprop-1-oxy)purine,
    la (S)-9-(1,4-diacétoxybut-2-oxy)guanine,
    la 2-amino-9-(2,3-dihydroxyprop-1-oxy)-6-méthoxypurine,
    la (R)-9-(2,3-dihydroxyprop-1-oxy)guanine,
    la 9-(3,4-dihydroxybut-1-oxy)guanine,
    la 2-amino-9-(3,4-dihydroxybut-1-oxy)purine,
    la(R)-2-amino-9-(2,3-dihydroxyprop-1-oxy)purine ou
    la (S)-9-(2,3-dihydroxyprop-1-oxy)guanine.

13. Procédé suivant l'une quelconque des revendications 1 à 3, pour la préparation d'un composé de formule (I) qui est :
    la 9-(3-hydroxyprop-1-oxy)guanine,
    la 9-(3-acétoxyprop-1-oxy)guanine,
    la 9-(3-benzoyloxyprop-1-oxy)guanine,
    la 2-amino-6-éthoxy-9-(3-hydroxyprop-1-oxy)purine,
    la 2-amino-9-(3-hydroxyprop-1-oxy)purine,
    la 9-(3-hexanoyloxyprop-1-oxy)guanine,
    la 9-(3-acétoxyprop-1-oxy)-2-aminopurine,
    la 2-amino-9-(3-hexanoyloxyprop-1-oxy)purine ou

la 2-amino-9-(3-benzoyloxyprop-1-oxy)purine.

**14.** Utilisation d'un composé de formule (I) répondant à la définition suivant l'une quelconque des revendications 1 et 4 à 12, ou d'un de ses sels pharmaceutiquement acceptables, dans la production d'un médicament destiné au traitement d'infections virales.

**15.** Utilisation d'un composé de formule (I), répondant à la définition suivant la revendication 13, ou d'un de ses sels pharmaceutiquement acceptables, dans la production d'un médicament destiné au traitement d'infections par virus de l'herpès.

**Revendications pour l'Etat contractant suivant : GR**

**1.** Composé de formule (I), ou un de ses sels pharmaceutiquement acceptables :

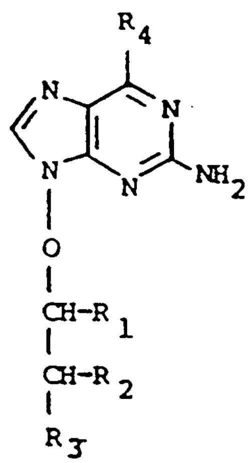

(I)

formule dans laquelle :

$R_1$ représente l'hydrogène ou un groupe $CH_2OH$ ;

$R_2$ représente l'hydrogène ou, (lorsque $R_1$ représente l'hydrogène), un groupe hydroxy ou $CH_2OH$ ;

$R_3$ représente un groupe $CH_2OH$ ou, (lorsque $R_1$ et $R_2$ représentent l'un et l'autre l'hydrogène), un groupe $CH(OH)CH_2OH$ ;

$R_4$ représente l'hydrogène, un groupe hydroxy, amino ou $OR_5$ dans lequel $R_5$ représente un groupe alkyle en $C_1$ à $C_6$ ; phényle ou phényl-(alkyle en $C_1$ ou $C_2$), chacun des groupements phényle pouvant être substitué avec un ou deux groupes halogéno, alkyle en $C_1$ à $C_4$ ou alkoxy en $C_1$ à $C_4$ ;

et dans laquelle n'importe quels groupes OH dans $R_1$, $R_2$ et/ou $R_3$ peuvent être sous forme de leurs dérivés O-acylés, phosphate, acétal cyclique ou carbonate cyclique, les dérivés O-acylés étant des dérivés dans lesquels un ou deux groupes OH dans $R_1$, $R_2$ et/ou $R_3$ forment des groupes ester carboxylique qui sont des groupes alcanoyle en $C_1$ à $C_7$ et benzoyle facultativement substitués avec un ou deux groupes alkyle en $C_1$ à $C_4$, alkoxy en $C_1$ à $C_4$, halogéno ou $CF_3$ ; les dérivés du type phosphate étant ceux dans lesquels un des groupes OH acycliques est remplacé par un groupe $(HO)_2$-$PO_2$ ou dans lesquels deux groupes OH sur des atomes de carbone adjacents sont remplacés par un groupe $-O-P(OH)O_2-$ de pontage ; un dérivé du type acétal cyclique étant un dérivé dans lequel deux groupes OH dans $R_1$, $R_2$ et $R_3$ forment un groupe -O-C(alkyle en $C_1$ à $C_3)_2$-O ; et un carbonate cyclique étant un groupe -O-CO-O-.

**2.** Composé de formule (I) suivant la revendication 1, dans lequel $R_1$ et $R_2$ représentent l'un et l'autre l'hydrogène et $R_3$ représente un groupe $CH_2OH$, et ses dérivés répondant à la définition suivant la revendication 1.

**3.** Composé de formule (I) suivant la revendication 1, dans lequel $R_1$ représente l'hydrogène et $R_2$ et $R_3$ représentent l'un et l'autre un groupe $CH_2OH$, et ses dérivés répondant à la définition suivant la revendication 1.

4. Composé de formule (I) suivant la revendication 1, dans lequel $R_1$ représente l'hydrogène, $R_2$ représente un groupe hydroxy et $R_3$ représente un groupe $CH_2OH$, et ses dérivés répondant à la définition suivant la revendication 1.

5. Composé de formule (I) suivant la revendication 1, dans lequel $R_1$ représente un groupe $CH_2OH$, $R_2$ représente l'hydrogène et $R_3$ représente un groupe $CH_2OH$, et ses dérivés répondant à la définition suivant la revendication 1.

6. Composé de formule (I) suivant la revendication 1, dans lequel $R_1$ et $R_2$ représentent l'un et l'autre l'hydrogène et $R_3$ représente un groupe $CH(OH)CH_2OH$, et ses dérivés répondant à la définition suivant la revendication 1.

7. Composé suivant l'une quelconque des revendications 1 à 6, dans lequel $R_4$ représente un groupe hydroxy.

8. Composé suivant l'une quelconque des revendications 1 à 6, dans lequel $R_4$ représente l'hydrogène.

9. Composé suivant l'une quelconque des revendications 1 à 8, dans lequel les groupes OH dans $R_1$, $R_2$ et/ou $R_3$ sont sous forme d'un de leurs dérivés acylés, ledit ou lesdits dérivés acylés consistant en acétates, hexanoates ou benzoates.

10. 9-(3-hydroxy-2-hydroxyméthylprop-1-oxy)guanine,
9-(2,3-dihydroxyprop-1-oxy)guanine,
2-amino-9-(2,3-dihydroxyprop-1-oxy)purine,
9-(1,4-dihydroxybut-2-oxy)guanine,
2-amino-9-(1,4-dihydroxybut-2-oxy)purine,
2-amino-9-(1,4-diacétoxybut-2-oxy)purine,
2-amino-9-(1,4-dibutyryloxybut-2-oxy)purine,
(R)-9-(1,4-dihydroxybut-2-oxy)guanine,
(S)-9-(1,4-dihydroxybut-2-oxy)guanine,
2-amino-9-(3-hydroxy-2-hydroxyméthylprop-1-oxy)purine,
9-(3-acétoxy-2-acétoxyméthylprop-1-oxy)-2-aminopurine,
2-amino-9-(3-propionyloxy-2-propionyloxyméthylprop-1-oxy)purine,
2-amino-9-(3-benzoyloxy-2-benzoyloxyméthylprop-1-oxy)purine,
2-amino-9-(3-hydroxy-2-hydroxyméthylprop-1-oxy)-6-méthoxypurine,
2,6-diamino-9-(3-hydroxy-2-hydroxyméthylprop-1-oxy)purine,
(S)-9-(1,4-diacétoxybut-2-oxy)guanine,
2-amino-9-(2,3-dihydroxyprop-1-oxy)-6-méthoxypurine,
(R)-9-(2,3-dihydroxyprop-1-oxy)guanine,
9-(3,4-dihydroxybut-1-oxy)guanine,
2-amino-9-(3,4-dihydroxybut-1-oxy)purine,
(R)-2-amino-9-(2,3-dihydroxyprop-1-oxy)purine ou
(S)-9-(2,3-dihydroxyprop-1-oxy)guanine.

11. 9-(3-hydroxyprop-1-oxy)guanine,
9-(3-acétoxyprop-1-oxy)guanine,
9-(3-benzoyloxyprop-1-oxy)guanine,
2-amino-6-éthoxy-9-(3-hydroxyprop-1-oxy)purine,
2-amino-9-(3-hydroxyprop-1-oxy)purine,
9-(3-hexanoyloxyprop-1-oxy)guanine,
9-(3-acétoxyprop-1-oxy)-2-aminopurine,
2-amino-9-(3-hexanoyloxyprop-1-oxy)purine ou
2-amino-9-(3-benzoyloxyprop-1-oxy)purine.

12. Procédé de préparation d'un composé de formule (I) suivant la revendication 1 ou d'un de ses sels pharmaceutiquement acceptables, procédé qui comprend la cyclisation d'un composé de formule (II) :

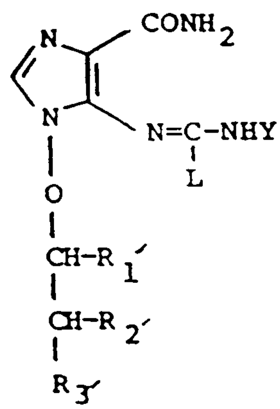

(II)

dans laquelle L représente un groupe partant renfermant un atome de soufre ou un groupe $NH_2$, Y représente un groupe protecteur et $R_1'$, $R_2'$ et $R_3'$ représentent respectivement des groupes $R_1$, $R_2$ et $R_3$ ou des groupes $R_1$, $R_2$ et/ou $R_3$ dans lesquels les groupes OH sont protégés ; et ensuite, lorsque $R_4$ dans le composé désiré de formule (I) est un groupe autre qu'un groupe hydroxy, la transformation d'un groupe hydroxy $R_4$ en un groupe chloro, suivie par (i) la réduction en un groupe $R_4$ consistant en l'hydrogène, (ii) le remplacement du groupe chloro par un groupe $NH_2$, donnant un groupe $R_4$ consistant en un groupe amino ; ou (iii) la réaction avec l'ion $OR_5^-$, donnant un groupe $R_4$ consistant en un groupe $OR_5$ ; et l'élimination du groupe protecteur Y et la transformation de $R_1'$ en $R_1$, $R_2'$ en $R_2$ et $R_3'$ en $R_3$ lorsque cela est désiré ou nécessaire, et, facultativement, la formation d'un de ses sels dérivés O-acylés, phosphate, acétal cyclique ou carbonate cyclique, pharmaceutiquement acceptables.

**13.** Procédé de préparation d'un composé de formule (I) suivant la revendication 1 ou d'un de ses sels pharmaceutiquement acceptables, procédé qui comprend la transformation du groupe chloro dans un composé de formule (VI) :

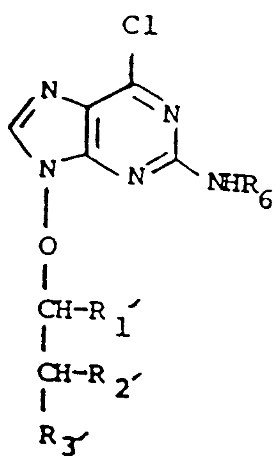

(VI)

dans laquelle $R_6$ représente l'hydrogène ou un groupe protecteur de l'atome d'azote, et $R_1'$, $R_2'$ et $R_3'$ répondent aux définitions mentionnées dans la revendication 12, par (i) une hydrolyse donnant un groupe $R_4$ consistant en un groupe hydroxy, (ii) une réduction donnant un composé de formule (I) dans laquelle $R_4$ représente l'hydrogène, (iii) un remplacement du groupe chloro par un groupe $NH_2$, donnant un groupe $R_4$ consistant en un groupe amino ; ou (iv) une réaction avec l'ion $OR_5^-$, donnant un composé de formule (I) dans laquelle $R_4$ représente un groupe $OR_5$, et ensuite la transformation de $R_1'$ en $R_1$, $R_2'$ en $R_2$ et/ou $R_3'$ en $R_3$ lorsque cela est nécessaire et/ou facultativement, la formation d'un de ses sels ou dérivés pharmaceutiquement acceptables répondant à la définition suivant la revendication 1.

**14.** Procédé de préparation d'un composé de formule (I) suivant la revendication 1, procédé qui comprend l'élimination de la protection d'un composé de formule (I) répondant à la définition suivant la revendication 1, dans laquelle n'importe quel groupe OH dans $R_1$, $R_2$ et/ou $R_3$ sont sous forme protégée ; et ensuite, facultativement, la formation d'un de ses sels ou dérivés pharmaceutiquement acceptables répondant à la définition suivant la revendication 1.

**15.** Composé de formule (II) répondant à la définition suivant la revendication 12.

**16.** Composé de formule (V) :

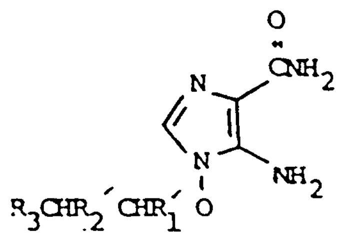

(V)

dans laquelle $R_1'$, $R_2'$ et $R_3'$ répondent aux définitions suivant la revendication 12.

**17.** Composé de formule (VI) répondant à la définition suivant la revendication 13.

**18.** Composé de formule (VIII) :

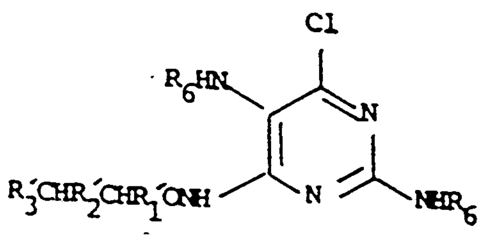

dans laquelle $R_6$ représente un groupe formyle et $R_1'$, $R_2'$ et $R_3'$ répondent aux définitions suivant la revendication 12.

**19.** Composé de formule (IV) :

$R_3'CHR_2'CHR_1'ONH_2$ (IV)

dans laquelle $R_1'$, $R_2'$ et $R_3'$ répondent aux définitions suivant la revendication 12, autre que des composés dans lesquels $R_2'$ et $R_3'$ sont des groupes OH protégés, dont les groupes OH sur des atomes de carbone adjacents sont remplacés par une liaison.

**20.** Composé de formule (I) répondant à la définition suivant la revendication 1, sauf que $R_1$, $R_2$ et/ou $R_3$ sont sous une forme protégée répondant à la définition pour $R_1'$, $R_2'$ et/ou $R_3'$ dans la revendication 12.

**21.** Composé intermédiaire de formules (II), (V), (VI), (VIII) ou (IV), répondant à la définition suivant les revendications 14 à 18, ou composé intermédiaire suivant la revendication 19, pour la préparation de n'importe quel composé suivant la revendication 10.

**22.** Utilisation d'un composé suivant l'une quelconque des revendications 1 à 10, ou d'un de ses sels pharmaceutiquement acceptables, dans la production d'un médicament destiné au traitement d'infections virales.

**23.** Utilisation d'un composé suivant la revendication 11, ou d'un de ses sels pharmaceutiquement acceptables, dans la production d'un médicament destiné au traitement d'infections par virus de l'herpès.